Europäisches Patentamt

European Patent Office

Office européen des brevets

(11) Publication number: **0 325 262 B1**

## EUROPEAN PATENT SPECIFICATION

(45) Date of publication of patent specification: **16.03.94**

(51) Int. Cl.5: **C12N 15/62**, C12N 5/10, C12P 21/02, A61K 37/02, A61K 39/395, G01N 33/569, //A61K39/21

(21) Application number: **89100913.6**

(22) Date of filing: **20.01.89**

(54) **Cloned genes encoding IG-CD4 fusion proteins and the use thereof.**

(30) Priority: **22.01.88 US 147351**

(43) Date of publication of application:
**26.07.89 Bulletin 89/30**

(45) Publication of the grant of the patent:
**16.03.94 Bulletin 94/11**

(84) Designated Contracting States:
**AT BE CH DE ES FR GB GR IT LI LU NL SE**

(56) References cited:
EP-A- 0 314 317
WO-A-89/01940
WO-A-89/02922
US-A- 104 329

NATURE, vol. 331, no. 6151, 7th January 1988, pages 84-86, London, GB; A. TRAUNECKER et al.: "Soluble CD4 molecules neutralize human immunodeficiency virus type 1"

DNA AND CELL BIOLOGY vol.9, no 5 (1990) p. 347-353

(73) Proprietor: **THE GENERAL HOSPITAL CORPO-RATION**
**55 Fruit Street**
**Boston, MA 02114(US)**

(72) Inventor: **Brian, Seed, Dr.**
**47A Joy Street**
**Boston MA 02114(US)**

(74) Representative: **Becker, Heinrich Karl Engelbert, Dr. et al**
**HOECHST AKTIENGESELLSCHAFT**
**Zentrale Patentabteilung**
**Gebäude F 821**
**D-65926 Frankfurt am Main (DE)**

**Description**

CROSS-REFERENCE TO RELATED APPLICATION

This application is a continuation-in-part of U.S. Application Serial No. 07/147,351 filed January 22, 1988.

FIELD OF THE INVENTION

The invention is in the field of recombinant genetics.

BACKGROUND OF THE INVENTION

The human and simian immunodeficiency viruses HIV and SIV are the causative agents of Acquired Immune Deficiency Syndrome (AIDS) and Simian Immunodeficiency Syndrome (SIDS), respectively. See Curren, J. et al., Science 329:1359-1357 (1985); Weiss, R. et al., Nature 324: 572-575 (1986). The HIV virus contains an envelope glycoprotein, gp120 which binds to the CD4 protein present on the surface of helper T lymphocytes, macrophages and other cells. Dalgleish et al. Nature, 312:763 (1984). After the gp120 binds to CD4, virus entry is facilitated by an envelope-mediated fusion of the viral target cell membranes.

During the course of infection, the host organism develops antibodies against viral proteins, including the major envelope glycoproteins gp120 and gp41. Despite this humoral immunity, the disease progresses, resulting in a lethal immunosuppression characterized by multiple opportunistic infections, parasitemia, dementia and death. The failure of host anti-viral antibodies to arrest the progression of the disease represents one of the most vexing and alarming aspects of the infection, and augurs poorly for vaccination efforts based upon conventional approaches.

Two factors may play a role in the inefficacy of the humoral response to immunodeficiency viruses. First, like other RNA viruses (and like retroviruses in particular), the immunodeficiency viruses show a high mutation rate which allows antigenic variation to progress at a high rate in response to host immune surveillance. Second, the envelope glycoproteins themselves are heavily glycosylated molecules presenting few epitopes suitable for high affinity antibody binding. The poorly antigenic, "moving" target which the viral envelope presents, allows the host little opportunity for restricting viral infection by specific antibody production.

Cells infected by the HIV virus express the gp120 glycoprotein on their surface. Gp120 mediates fusion events among $CD4^+$ cells via a reaction similar to that by which the virus enters the uninfected cell, leading to the formation of short-lived multinucleated giant cells. Syncytium formation is dependent on a direct interaction of the gp120 envelope glycoprotein with the CD4 protein. Dalgleish et al., supra, Klatzmann, D. et al., Nature 312:763 (1984); McDougal, J.S. et al. Science, 231:382 (1986); Sodroski, J. et al., Nature, 322:470 (1986); Lifson, J.D. et al., Nature, 323:725 (1986); Sodroski, J. et al., Nature, 321:412 (1986).

The CD4 protein consists of a 370 amino acid extracellular region containing four immunoglobulin-like domains, a membrane spanning domain, and a charged intracellular region of 40 amino acid residues. Maddon, P. et al., Cell 42:93 (1985); Clark, S. et al., Proc. Natl. Acad. Sci. (USA) 84:1649 (1987).

Evidence that CD4-gp120 binding is responsible for viral infection of cells bearing the CD4 antigen includes the finding that a specific complex is formed between gp120 and CD4. McDougal et al., supra. Other workers have shown that cell lines, which were non-infective for HIV, were converted to infectable cell lines following transfection and expression of the human CD4 cDNA gene. Maddon et al., Cell 47:333-348 (1986).

In contrast to the majority of antibody-envelope interactions, the receptor-envelope interaction is characterized by a high affinity ($K_a \approx 10^8$ l/mole) immutable association. Moreover, the affinity of the virus for CD4 is at least 3 orders of magnitude higher than the affinity of CD4 for its putative endogenous ligand, the MHC class II antigens. Indeed, to date, a specific physical association between monomeric CD4 and class II antigens has not been demonstrated.

In response to bacterial or other particle infection, the host organism usually produces serum antibodies that bind to specific proteins or carbohydrates on the bacterial or particle surface, coating the bacteria. This antibody coat on the bacterium or other particle stimulates cytolysis by Fc-receptor-bearing lymphoid cells by antibody-dependent cellular toxicity (ADCC). Other serum proteins, collectively called complement (C), bind to antibody-coated targets, and also can coat foreign particles nonspecifically. They cause cell death by lysis, or stimulate ingestion by binding to specific receptors on the macrophage called complement receptors. See Darnell J. et al., in Molecular Cell Biology, Scientific American Books, pp. 641 and 1087 (1986).

The most effective complement activating classes of human Ig are IgM and IgG1. The complement system consists of 14 proteins that, acting in order, cause lysis of cells. Nearly all of the C proteins exist in normal serum as inactive precursors. When activated, some become highly specific proteolytic enzymes whose substrate is the next protein in a sequential chain reaction.

The entire C sequence can be triggered by either of two initiation pathways. In one (the classic pathway), Ab-Ag complexes bind and activate C1, C4 and C2 to form a C3-splitting enzyme. In the second pathway, polysaccharides commonly on the surface of many bacteria and fungi bind with trace amounts of a C3 fragment and then with two other proteins (factor B and properdin) to form another C3-splitting enzyme. Once C3 is split by either pathway, the way is open for the remaining sequence of steps which lead to cell lysis. See Davis, B.D., et al., In Microbiology, 3rd ed., Harper and Row, Philadelphia, PA, pp. 452-466 (1980).

A number of workers have disclosed methods for preparing hybrid proteins. For example, Murphy, United States Patent 4,675,382 (1987), discloses the use of recombinant DNA techniques to make hybrid protein molecules by forming the desired fused gene coding for a hybrid protein of diptheria toxin and a polypeptide ligand such as a hormone, followed by expression of the fused gene.

Many workers have prepared monoclonal antibodies (Mabs) by recombinant DNA techniques. Monoclonal antibodies are highly specific well-characterized molecules in both primary and tertiary structure. They have been widely used for in vitro immunochemical characterization and quantitation of antigens. Genes for heavy and light chains have been introduced into appropriate hosts and expressed, followed by reaggregation of the individual chains into functional antibody molecules (see, for example, Munro, Nature 312:597 (1984); Morrison, S.L., Science 229:1202 (1985); Oi et al., Biotechniques 4:214 (1986); Wood et al., Nature 314:446-449 (1985)). Light- and heavy-chain variable regions have been cloned and expressed in foreign hosts wherein they maintained their binding ability (Moore et al., European Patent Application 0088994 (published September 21, 1983)).

Chimeric or hybrid antibodies have also been prepared by recombinant DNA techniques. Oi and Morrison, Biotechniques 4:214 (1986) describe a strategy for producing such chimeric antibodies which include a chimeric human IgG anti-leu3 antibody.

Gascoigne, N.R.J., et al., Proc. Natl. Acad. Sci. (USA) 84:2936-2940 (1987) disclose the preparation of a chimeric gene construct containing a T-cell receptor a-chain variable (V) domain and the constant (C) region coding sequence of an immunoglobulin $_\gamma$2a molecule. Cells transfected with the chimeric gene synthesize a protein product that expresses immunoglobulin and T-cell receptor antigenic determinants as well as protein A binding sites. This protein associates with a normal $\lambda$ chain to form an apparently normal tetrameric ($H_2 L_2$, where H = heavy and L = light) immunoglobulin molecule that is secreted.

Sharon, J., et al., Nature 309:54 (1984), disclose construction of a chimeric gene encoding the variable (V) region of a mouse heavy chain specific for the hapten azophenylarsonate and the constant (C) region of a mouse kappa light chain ($V_H C_K$). This gene was introduced into a mouse myeloma cell line. The chimeric gene was expressed to give a protein which associated with light chains secreted from the myeloma cell line to give an antibody molecule specific for azophenylarsonate.

Morrison, Science 229:1202 (1985), discloses that variable light-or variable heavy-chain regions can be attached to a non-Ig sequence to create fusion proteins. This article states that the potential uses for the fusion proteins are three: (1) to attach antibody specifically to enzymes for use in assays; (2) to isolate non-Ig proteins by antigen columns; and (3) to specifically deliver toxic agents.

Recent techniques for the stable introduction of immunoglobulin genes into myeloma cells (Banerji, J., et al., Cell 33:729-740 (1983); Potter, H., et al., Proc. Natl. Acad. Sci. (USA) 81:7161-7165 (1984)), coupled with detailed structural information, have permitted the use of in vitro DNA methods such as mutagenesis, to generate recombinant antibodies possessing novel properties.

PCT Application WO87/02671 discloses methods for producing genetically engineered antibodies of desired variable region specificity and constant region properties through gene cloning and expression of light and heavy chains. The mRNA from cloned hybridoma B cell lines which produce monoclonal antibodies of desired specificity is isolated for cDNA cloning. The generation of light and heavy chain coding sequences is accomplished by excising the cloned variable regions and ligating them to light or heavy chain module vectors. This gives cDNA sequences which code for immunoglobulin chains. The lack of introns allows these cDNA sequences to be expressed in prokaryotic hosts, such as bacteria, or in lower eukaryotic hosts, such as yeast.

The generation of chimeric antibodies in which the antigen-binding portion of the immunoglobulin is fused to other moieties has been demonstrated. Examples of non-immunoglobulin genes fused to antibodies include Staphylococcus aureus nuclease, the mouse oncogene c-myc, and the Klenow fragment of E. coli DNA polymerase I (Neuberger, M.S., et al., Nature 312:604-612 (1984); Neuberger, M.S., Trends in Biochemical Science, 347-349 (1985)). European Patent Application 120,694 discloses the genetic engineering of the variable and constant regions of an immunoglobulin molecule that is expressed in E. coli host cells. It is further disclosed that the immunoglobulin molecule may be synthesized by a host cell with another peptide moiety attached to one of the constant domains. Such peptide moieties are described as either cytotoxic or enzymatic. The application and the examples describe the use of a lambda-like chain derived from a monoclonal antibody which binds to 4-hydroxy-3-nitrophenyl (NP) haptens.

European Patent Application 125,023 relates to the use of recombinant DNA techniques to produce immunoglobulin molecules that are chimeric or otherwise modified. One of the uses described for these immunoglobulin molecules is for whole-body diagnosis and treatment by injection of the antibodies directed to specific target tissues. The presence of the disease can be determined by attaching a suitable label to the antibodies, or the diseased tissue can be attacked by carrying a suitable drug with the antibodies. The application describes antibodies engineered to aid the specific delivery of an agent as "altered antibodies."

PCT Application WO83/101533 describes chimeric antibodies wherein the variable region of an immunoglobulin molecule is linked to a portion of a second protein which may comprise the active portion of an enzyme.

Boulianne et al., Nature 312:643 (1984) constructed an immunoglobulin gene in which the DNA segments that encode mouse variable regions specific for the hapten trinitrophenol (TNP) are joined to segments that encode human mu and kappa regions. These chimeric genes were expressed to give functional TNP-binding chimeric IgM.

Morrison et al., P.N.A.S. (USA) 81:6851 (1984), disclose a chimeric molecule utilizing the heavy-chain variable region exons of an anti-phosphoryl choline myeloma protein G, which were joined to the exons of either human kappa light-chain gene. The genes were transfected into mouse myeloma cell lines, generating transformed cells that produced chimeric mouse-human IgG with antigen-binding function.

Despite the progress that has been achieved on determining the mechanism of HIV infection, a need continues to exist for methods of treating HIV viral infections.

## SUMMARY OF THE INVENTION

The invention relates to a gene comprising a DNA sequence which encodes a fusion protein comprising 1) CD4, or a fragment thereof which binds to HIV gp120, and 2) an immunoglobulin light or heavy chain; wherein said CD4 or HIV gp120-binding fragment thereof replaces the variable region of the light or heavy immunoglobulin chain.

The invention also relates to vectors containing the gene of the invention and hosts transformed with the vectors.

The invention also relates to a method of producing a fusion protein comprising CD4, or fragment thereof which binds to HIV gp120, and an immunoglobulin light or heavy chain, wherein the variable region of the immunoglobulin light or heavy chain has been substituted with CD4, or HIV gp120-binding fragment thereof, which comprises:

cultivating in a nutrient medium under protein producing conditions, a host strain transformed with the vector containing the gene of the invention, said vector further comprising expression signals which are recognized by said host strain and direct expression of said fusion protein, and

recovering the fusion protein so produced.

The invention also relates to a fusion protein comprising CD4, or fragment thereof which is capable of binding to HIV gp120, fused at the C-terminus to a second protein which comprises an immunoglobulin light or heavy chain, wherein the variable region of said light or heavy chain is substituted with CD4 or a HIV gp120 binding fragment thereof.

The invention also relates to an immunoglobulin-like molecule comprising the fusion protein of the invention together with an immunoglobulin light or heavy-chain, wherein said immunoglobulin like molecule binds HIV gp120.

The IgG1 fusion proteins and immunoglobulin-like molecules may be useful for both complement-mediated and cell-mediated (ADCC) immunity, while the IgM fusion proteins are useful principally through complement-mediated immunity.

The invention also relates to a complex between the fusion proteins and immunoglobulin-like molecule of the invention and HIV gp120.

The invention also relates to a method for treating HIV or SIV infections comprising administering the fusion protein or immunoglobulin-like molecule of the invention to an animal.

The invention further relates to a method for detecting HIV gp120 in a sample comprising contacting a sample suspected of containing HIV or gp120 with the fusion protein or immunoglobulin-like molecule of the invention, and detecting whether a complex has formed.

DESCRIPTION OF THE PREFERRED EMBODIMENTS

The invention is directed to a protein gene which comprises
1) a DNA sequence which codes for CD4, or fragment thereof which binds to HIV gp120, fused to
2) a DNA sequence which encodes an immunoglobulin heavy chain.
Preferably, the antibody has effector function.
The invention is also directed to a protein gene which comprises
1) a DNA sequence which codes for CD4, or fragment thereof which binds to HIV gp120, fused to
2) a DNA sequence which encodes an immunoglobulin light chain; wherein said sequence which codes for CD4, or HIV gp120-binding fragment thereof, replaces the variable region of the light immunoglobulin chain.

The invention is also directed to the expression of these novel fusion proteins in transformed hosts and the use thereof to treat and diagnose HIV infections. In particular, the invention relates to expressing said genes in mammalian hosts which express complementary light or heavy chain immunoglobulins to give immunoglobulin-like molecules which have antibody effector function and also bind to HIV or SIV gp120.

The term "antibody effector function" as used herein denotes the ability to fix complement or to activate ADCC.

The fusion proteins and immunoglobulin-like molecules may be administered to an animal for the purpose of treating HIV or SIV infections. By the terms "HIV infections" is intended the condition of having AIDS, AIDS related complex (ARC) or where an animal harbors the AIDS virus, but does not exhibit the clinical symptoms of AIDS or ARC. By the terms "SIV infections" is intended the condition of being infected with simian immunodeficiency virus.

By the term "animal" is intended all animals which may derive benefit from the administration of the fusion proteins and immunoglobulin-like molecules of the invention. Foremost among such animals are humans, however, the invention is not intended to be so limited.

By the term "fusion protein" is intended a fused protein comprising CD4, or fragment thereof which is capable of binding to gp120, linked at its C-terminus to an immunoglobulin chain wherein a portion of the N-terminus of the immunoglobulin is replaced with CD4. In general, that portion of immunoglobulin which is deleted is the variable region. The fusion proteins of the invention may also comprise immunoglobulins where more than just the variable region has been deleted and replaced with CD4 or HIV gp120 binding fragment thereof. For example, the $V_H$ and CH1 regions of an immunoglobulin chain may be deleted. Preferably, any amount of the N-terminus of the immunoglobulin heavy chain can be deleted as long as the remaining fragment has antibody effector function. The minimum sequence required for binding complement encompasses domains CH2 and CH3. Joining of Fc portions by the hinge region is advantageous for increasing the efficiency of complement binding.

The CD4 portion of the fusion protein may comprise the complete CD4 sequence, the 370 amino acid extracellular region and the membrane spanning domain, or the extracellular region. The fusion protein may comprise fragments of the extracellular region obtained by cutting the DNA sequence which encodes CD4 at the BspM1 site at position 514 or the PvuII site at position 629 (see Table 1) to give nucleotide sequences which encode CD4 fragments which retain binding to gp120. In general, any fragment of CD4 may be used as long as it retains binding to gp120.

Where the fusion protein comprises an immunoglobulin light chain, it is necessary that no more of the Ig chain be deleted than is necessary to form a stable complex with a heavy chain Ig. In particular, the cysteine residues necessary for disulfide bond formation must be preserved on both the heavy and light chain moieties.

When expressed in a host, e.g., a mammalian cell, the fusion protein may associate with other light or heavy Ig chains secreted by the cell to give a functioning immunoglobulin-like molecule which is capable of binding to gp120. The gp120 may be in solution, expressed on the surface of infected cells, or may be present on the surface of the HIV virus itself. Alternatively, the fusion protein may be expressed in a mammalian cell which does not secrete other light or heavy Ig chains. When expressed under these conditions, the fusion protein may form a homodimer.

Genomic or CDNA sequences may be used in the practice of the invention. Genomic sequences are expressed efficiently in myeloma cells, since they contain native promoter structures.

5

The constant regions of the antibody cloned and used in the chimeric immunoglobulin-like molecule may be derived from any mammalian source. The constant regions may be complement binding or ADCC active. However, preliminary work (see Examples) indicates that the fusion proteins of the invention may mediate HIV or SIV infected cell death by an ADCC or complement-independent mechanism. The constant regions may be derived from any appropriate isotype, including IgG1, IgG3, or IgM.

The joining of various DNA fragments, is performed in accordance with conventional techniques, employing blunt-ended or staggered-ended termini for ligation, restriction enzyme digestion to provide appropriate termini, filling in of cohesive ends as appropriate, alkali and phosphatase treatment to avoid undesirable joining, and ligation with appropriate ligases. The genetic construct may optionally encode a leader sequence to allow efficient expression of the fusion protein. For example, the leader sequence utilized by Maddon et al., Cell 42: 93-104 (1985) for the expression of CD4 may be used.

For cDNA, the cDNA may be cloned and the resulting clone screened, for example, by use of a complementary probe or by assay for expressed CD4 using an antibody as disclosed by Dalgleish et al., Nature 312:763-766 (1984); Klatzmann et al., Immunol. Today 7:291-297 (1986); McDougal et al., J. Immunol. 135:3151-3162 (1985); and McDougal, J. et al., J. Immunol. 137:2937-2944 (1986).

To express the fusion hybrid protein, transcriptional and translational signals recognized by an appropriate host element are necessary. Eukaryotic hosts which may be used include mammalian cells capable of culture in vitro, particularly leukocytes, more particularly myeloma cells or other transformed or oncogenic lymphocytes, e.g., EBV-transformed cells. Alternatively, non-mammalian cells may be employed, such as bacteria, fungi, e.g., yeast, filamentous fungi, or the like.

Preferred hosts for fusion protein production are mammalian cells, grown in vitro in tissue culture or in vivo in animals. Mammalian cells provide post translational modification to immunoglobulin protein molecules which provide for correct folding and glycosylation of appropriate sites. Mammalian cells which may be useful as hosts include cells of fibroblast origins such as VERO or CHO-K1 or cells of lymphoid origin, such as the hybridoma SP2/0-AG14 or the myeloma P3x63Sgh, and their derivatives. For the purpose of preparing an immunoglobulin-like molecule, a plasmid containing a gene which encodes a heavy chain immunoglobulin, wherein the variable region has been replaced with CD4 or fragment thereof which binds to gp120, may be introduced, for example, into J558L myeloma cells, a mouse plasmacytoma expressing the lambda-1 light chain but which does not express a heavy chain (see Oi et al., P.N.A.S. (USA) 80:825-829 (1983)). Other preferred hosts include COS cells, BHK cells and hepatoma cells.

The constructs may be joined together to form a single DNA segment or may be maintained as separate segments, by themselves or in conjunction with vectors.

Where the fusion protein is not glycosylated, any host may be used to express the protein which is compatible with replicon and control sequences in the expression plasmid. In general, vectors containing replicon and control sequences are derived from species compatible with a host cell are used in connection with the host. The vector ordinarily carries a replicon site, as well as specific genes which are capable of providing phenotypic selection in transformed cells. The expression of the fusion protein can also be placed under control with other regulatory sequences which may be homologous to the organism in its untransformed state. For example, lactose-dependent E. coli chromosomal DNA comprises a lactose or lac operon which mediates lactose utilization by elaborating the enzyme beta-galactosidase. The lac control elements may be obtained from bacterial phage lambda plac5, which is infective for E. coli. The lac promoter-operator system can be induced by IPTG.

Other promoters/operator systems or portions thereof can be employed as well. For example, colicin E1, galactose, alkaline phosphatase, tryptophan, xylose, tax, and the like can be used.

For mammalian hosts, several possible vector systems are available for expression. One class of vectors utilize DNA elements which are derived from animal viruses such as bovine papilloma virus, polyoma virus, adenovirus, vaccinia virus, baculovirus, retroviruses RSV, MMTV or MOMLV), or SV40 virus. Cells which have stably integrated the DNA into their chromosomes may be selected by introducing one or more markers which allow selection of transfected host cells. The marker may provide for prototropy to an auxotrophic host, biocide resistance, e.g., antibiotics, or heavy metals such as copper or the like. The selectable marker gene can be either directly linked to the DNA sequences to be expressed, or introduced into the same cell by cotransformation. Additional elements may also be needed for optimal synthesis of mRNA. These elements may include splice signals, as well as transcriptional promoters, enhancers, and termination signals. The cDNA expression vectors incorporating such elements includes those described by Okayama, H., Mol. Cel. Biol., 3:280 (1983) and others.

Once the vector or DNA sequence containing the constructs has been prepared for expression, the DNA constructs may be introduced to an appropriate host. Various techniques may be employed, such as protoplast fusion, calcium phosphate precipitation, electroporation or other conventional techniques. After the fusion, the cells are grown in media and screened for the appropriate activity. Expression of the gene(s) results in production of the fusion protein. This expressed fusion protein may then be subject to further assembly to form the immunoglobulin-like molecule.

The host cells for immunoglobulin production may be immortalized cells, primarily myeloma or lymphoma cells. These cells may be grown in appropriate nutrient medium in culture flasks or injected into a synergistic host, e.g., mouse or a rat, or immunodeficient host or host site, e.g., nude mouse or hamster pouch. In particular, the cells may be introduced into the abdominal cavity of an animal to allow production of ascites fluid which contains the immunoglobulin-like molecule. Alternatively, the cells may be injected subcutaneously and the chimeric antibody is harvested from the blood of the host. The cells may be used in the same manner as hybridoma cells. See Diamond et al., N. Eng. J. Med. 304:1344 (1981), and Kennatt, McKearn and Bechtol (Eds.), Monoclonal Antibodies: Hybridomas: -- A New Dimension in Biologic Analysis, Plenum, 1980.

The fusion proteins and immunoglobulin-like molecules of the invention may be isolated and purified in accordance with conventional conditions, such as extraction, precipitation, chromatography, affinity chromatography, electrophoresis or the like. For example, the IgG1 fusion proteins may be purified by passing a solution through a column which contains immobilized protein A or protein G which selectively binds the Fc portion of the fusion protein. See, for example, Reis, K.J., et al., J. Immunol. 132:3098-3102 (1984); PCT Application, Publication No. WO87/00329. The chimeric antibody may the be eluted by treatment with a chaotropic salt or by elution with aqueous acetic acid (1 M).

Alternatively the fusion proteins may be purified on anti-CD4 antibody columns, or on anti-immunoglobulin antibody columns.

In one embodiment of the invention, cDNA sequences which encode CD4, or a fragment thereof which binds gp120, may be ligated into an expression plasmid which codes for an antibody wherein the variable region of the gene has been deleted. Methods for the preparation of genes which encode the heavy or light chain constant regions of immunoglobulins are taught, for example, by Robinson, R. et al., PCT Application, Publication No. WO87-02671.

Preferred immunoglobulin-like molecules which contain CD4, or fragments thereof, contain the constant region of an IgM, IgG1 or IgG3 antibody which binds complement at the Fc region.

The fusion protein and immunoglobulin-like molecules of the invention may be used for the treatment of HIV viral infections. The fusion protein complexes to gp120 which is expressed on infected cells. Although the inventor is not bound by a particular theory, it appears that the Fc portion of the hybrid fusion protein may bind with complement, which mediates destruction of the cell. In this manner, infected cells are destroyed so that additional viral particle production is stopped.

For the purpose of treating HIV infections, the fusion protein or immunoglobulin-like molecule of the invention may additionally contain a radiolabel or therapeutic agent which enhances destruction of the HIV particle or HIV-infected cell.

Examples of radioisotopes which can be bound to the fusion protein or immunoglobulin-like molecule of the invention for use in HIV-therapy are $^{125}$I, $^{131}$I, $^{90}$Y, $^{67}$Cu, $^{217}$Bi, $^{211}$At, $^{212}$Pb, $^{47}$Sc, and $^{109}$Pd. Optionally, a label such as boron can be used which emits $\alpha$ and $\beta$ particles upon bombardment with neutron radiation.

For in vivo diagnosis radionucleotides may be bound to the fusion protein or immunoglobulin-like molecule of the invention either directly or by using an intermediary functional group. An intermediary group which is often used to bind radioisotopes, which exist as metallic cations, to antibodies is diethylenetriaminepentaacetic acid (DTPA). Typical examples of metallic cations which are bound in this manner are $^{99m}$Tc $^{123}$I, $^{111}$In, $^{131}$I, $^{97}$Ru, $^{67}$Cu, $^{67}$Ga, and $^{68}$Ga.

Moreover, the fusion protein and immunoglobulin-like molecule of the invention may be tagged with an NMR imaging agent which include paramagnetic atoms. The use of an NMR imaging agent allows the in vivo diagnosis of the presence of and the extent of HIV infection within a patient using NMR techniques. Elements which are particularly useful in this manner are $^{157}$Gd, $^{55}$Mn, $^{162}$Dy, $^{52}$Cr, and $^{56}$Fe.

Therapeutic agents may include, for example, bacterial toxins such as diphtheria toxin, or ricin. Methods for producing fusion proteins comprising fragment A of diphtheria toxin are taught in U.S. Patent 4,675,382 (1987). Diphtheria toxin contains two polypeptide chains. The B chain binds the toxin to a receptor on a cell surface. The A chain actually enters the cytoplasm and inhibits protein synthesis by inactivating elongation factor 2, the factor that translocates ribosomes along mRNA concomitant with hydrolysis of ETP. See Darnell, J., et al., in Molecular Cell Biology, Scientific American Books, Inc., page 662 (1986). Alternatively, a fusion protein comprising ricin, a toxic lectin, may be prepared.

Introduction of the chimeric molecules by gene therapy may also be contemplated, for example, using retroviruses or other means to introduce the genetic material encoding the fusion proteins into suitable target tissues. In this embodiment, the target tissues having the cloned genes of the invention may then

produce the fusion protein in vivo.

The dose ranges for the administration of the fusion protein or immunoglobulin-like molecule of the invention are those which are large enough to produce the desired effect whereby the symptoms of HIV or SIV infection are ameliorated. The dosage should not be so large as to cause adverse side effects, such as unwanted cross-reactions, anaphylactic reactions, and the like. Generally, the dosage will vary with the age, condition, sex and extent of disease in the patient, counterindications, if any, immune tolerance and other such variables, to be adjusted by the individual physician. Dosage can vary from .01 mg/kg to 50 mg/kg, preferably 0.1 mg/kg to 1.0 mg/kg, of the immunoglobulin-like molecule in one or more administrations daily, for one or several days. The immunoglobulin-like molecule can be administered parenterally by injection or by gradual perfusion over time. They can be administered intravenously, intraperitoneally, intramuscularly, or subcutaneously.

Preparations for parenteral administration include sterile or aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers, such as those based on Ringer's dextrose, and the like. Preservatives and other additives may also be present, such as, for example, antimicrobials, antioxidants, chelating agents, inert gases and the like. See, generally, Remington's Pharmaceutical Science, 16th Ed., Mack Eds., 1980.

The invention also relates to a method for preparing a medicament or pharmaceutical composition comprising the components of the invention, the medicament being used for therapy of HIV or SIV infection in animals.

The detection and quantitation of antigenic substances and biological samples frequently utilized immunoassay techniques. These techniques are based upon the formation of the complex between the antigenic substance, e.g., gp120, being assayed and an antibody or antibodies in which one or the other member of the complex may be detectably labeled. In the present invention, the immunoglobulin-like molecule or fusion protein may be labeled with any conventional label.

Thus, the hybrid fusion protein or immunoglobulin-like molecule of the invention can also be used in assay for HIV or SIV viral infection in a biological sample by contacting a sample, derived from an animal suspected of having an HIV or SIV infection, with the fusion protein or immunoglobulin-like molecule of the invention, and detecting whether a complex with gp120, either alone or on the surface of an HIV-infected cell, has formed.

For example, a biological sample may be treated with nitrocellulose, or other solid support which is capable of immobilizing cells, cell particles or soluble protein. The support may then be washed with suitable buffers followed by treatment with the fusion protein which may be detectably labeled. The solid phase support may then be washed with the buffer a second time to remove unbound fusion protein and the label on the fusion protein detected.

In carrying out the assay of the present invention on a sample containing gp120, the process comprises:

a) contacting a sample suspected containing gp120 with a solid support to effect immobilization of gp120, or cell which expresses gp120 on its surface;

b) contacting said solid support with the detectably labeled immunoglobulin-like molecule or fusion protein of the invention;

c) incubating said detectably labeled immunoglobulin-like molecule with said support for a sufficient amount of time to allow the immunoglobulin-like molecule or fusion protein to bind to the immobilized gp120 or cell which expresses gp120 on its surface;

d) separating the solid phase support from the incubation mixture obtained in step c); and

e) detecting the bound immunoglobulin-like molecule or fusion protein and thereby detecting and quantifying gp120.

Alternatively, labeled immunoglobulin-like molecule (or fusion protein) -gp120 complex in a sample may be separated from a reaction mixture by contacting the complex with an immobilized antibody or protein which is specific for an immunoglobulin or, e.g., protein A, protein G, anti-IgM or anti-IgG antibodies. Such anti-immunoglobulin antibodies may be monoclonal or polyclonal. The solid support may then be washed with suitable buffers to give an immobilized gp120-labeled immunoglobulin-like molecule antibody complex. The label on the fusion protein may then be detected to give a measure of endogenous gp120 and, thereby, the presence of HIV.

This aspect of the invention relates to a method for detecting HIV or SIV viral infection in a sample comprising

(a) contacting a sample suspected of containing gp120 with a fusion protein or immunoglobulin-like molecule comprising CD4, or fragment thereof which binds to gp120, and the Fc portion of an immunoglobulin chain,

(b) detecting whether a complex is formed.

The invention also relates to a method of detecting gp120 in a sample, further comprising

(c) contacting the mixture obtained in step (a) with an Fc binding molecule, such as an antibody, protein A, or protein G, which is immobilized on a solid phase support and is specific for the hybrid fusion protein, to give a gp120 fusion protein-immobilized antibody complex

(d) washing the solid phase support obtained in step (c) to remove unbound fusion protein,

(e) and detecting the label on the hybrid fusion protein.

Of course, the specific concentrations of detectably labeled immunoglobulin-like molecule (or fusion protein) and gp120, the temperature and time of incubation, as well as other assay conditions may be varied, depending on various factors including the concentration of gp120 in the sample, the nature of the sample, and the like. Those skilled in the art wild be able to determine operative and optimal assay conditions for each determination by employing routine experimentation.

Other such steps as washing, stirring, shaking, filtering and the like may be added to the assays as is customary or necessary for the particular situation.

One of the ways in which the immunoglobulin-like molecule or fusion protein of the present invention can be detectably labeled is by linking the same to an enzyme. This enzyme, in turn, when later exposed to its substrate, will react with the substrate in such a manner as to produce a chemical moiety which can be detected as, for example, by spectrophotometric, fluorometric or by visual means. Enzymes which can be used to detectably label the immunoglobulin-like molecule or fusion protein of the present invention include, but are not limited to, malate dehydrogenase, staphylococcal nuclease, delta-V-steroid isomerase, yeast alcohol dehydrogenase, alpha-glycerophosphate dehydrogenase, triose phosphate isomerase, horseradish peroxidase, alkaline phosphatase, asparaginase, glucose oxidase, beta-galactosidase, ribonuclease, urease, catalase, glucose-VI-phosphate dehydrogenase, glucoamylase and acetylcholine esterase.

The immunoglobulin-like molecule or fusion protein of the present invention may also be labeled with a radioactive isotope which can be determined by such means as the use of a gamma counter or a scintillation counter or by autoradiography. Isotopes which are particularly useful for the purpose of the present invention are: $^3$H, $^{125}$I, $^{131}$I, $^{32}$P, $^{35}$S, $^{14}$C, $^{51}$Cr, $^{36}$Cl, $^{57}$Co, $^{58}$Co, $^{59}$Fe and $^{75}$Se.

It is also possible to label the immunoglobulin-like molecule or fusion protein with a fluorescent compound. When the fluorescently labeled immunoglobulin-like molecule is exposed to light of the proper wave length, its presence can then be detected due to the fluorescence of the dye. Among the most commonly used fluorescent labelling compounds are fluorescein isothiocyanate, rhodamine, phycoerytherin, phycocyanin, allophycocyanin, o-phthaldehyde and fluorescamine.

The immunoglobulin-like molecule or fusion protein of the invention can also be detectably labeled using fluorescence emitting metals such as $^{152}$Eu, or others of the lanthanide series. These metals can be attached to the immunoglobulin-like molecule or fusion protein using such metal chelating groups as diethylenetriaminepentaacetic acid (DTPA) or ethylenediaminetetraacetic acid (EDTA).

The immunoglobulin-like molecule or fusion protein of the present invention also can be detectably labeled by coupling it to a chemiluminescent compound. The presence of the chemiluminescent-tagged immunoglobulin-like molecule or fusion protein is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

Likewise, a bioluminescent compound may be used to label the immunoglobulin-like molecule or fusion protein of the present invention. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

Detection of the immunoglobulin-like molecule or fusion protein may be accomplished by a scintillation counter, for example, if the detectable label is a radioactive gamma emitter, or by a fluorometer, for example, if the label is a fluorescent material. In the case of an enzyme label, the detection can be accomplished by colorimetric methods which employ a substrate for the enzyme. Detection may also be accomplished by visual comparison of the extent of enzymatic reaction of a substrate in comparison with similarly prepared standards.

The assay of the present invention is ideally suited for the preparation of a kit. Such a kit may comprise a carrier means being compartmentalized to receive in close confinement therewith one or more container means such as vials, tubes and the like, each of said container means comprising the separate elements of the immunoassay. For example, there may be a container means containing a solid phase support, and further container means containing the detectably labeled immunoglobulin-like molecule or fusion protein in solution. Further container means may contain standard solutions comprising serial dilutions of analytes such as gp120 or fragments thereof to be detected. The standard solutions of these analytes may be used to prepare a standard curve with the concentration of gp120 plotted on the abscissa and the detection signal on the ordinate. The results obtained from a sample containing gp120 may be interpolated from such a plot to give the concentration of gp120.

The immunoglobulin-like molecule or fusion protein of the present invention can also be used as a stain for tissue sections. For example, a labeled immunoglobulin-like molecule comprising CD4 or fragment thereof which binds to gp120 may be contacted with a tissue section, e.g., a brain biopsy specimen. This section may then be washed and the label detected.

The following examples are illustrative, but not limiting the method and composition of the present invention. Other suitable modifications and adaptations which are obvious to this skill in the art are within the spirit and scope of this invention.

EXAMPLES

Example 1: Preparation of CD4-Ig cDNA Constructs

The extracellular portion of the CD4 molecule (See Madden, P.J., et al., Cell 42:93-104 (1985)) was fused at three locations in a human IgG1 heavy chain constant region gene by means of a synthetic splice donor linker molecule. To exploit the splice donor linker, a BamHI linker having the sequence CGCGGATCCGCG was first inserted at amino acid residue 395 of the CD4 precursor sequence (nucleotide residue 1295). A synthetic splice donor sequence

GATCCCGAGGGTGAGTACTA

GGCTCCCACTCATGATTCGA

bounded by BamHI and HindIII complementary ends was created and fused to the HindIII site in the intron preceding the CH1 domain, to the EspI site in the intron preceding the hinge domain, and to the BanI site preceding the CH2 domain of the IgG1 genomic sequence. Assembly of the chimeric genes by ligation at the BamHI site afforded molecules in which either the variable (V) region, the V + CH1 regions, or the V, CH1 and hinge regions were replaced by CD4. In the last case, the chimeric molecule is expected to form a monomer structure, while in the former, a dimeric molecule is expected.

On such genetic construct which contains the DNA sequence which encodes CD4 linked to human IgG1 at the Hind3 site upstream of the CH1 region (fusion protein CD4H$\gamma$1) is depicted in Table 1. The plasmid containing this genetic construct (pCD4H$\gamma$1) has been deposited in E. coli (MC1061/P3) at the American Type Culture Collection (ATCC) under the terms of the Budapest Treaty and given accession number 67611.

A second genetic construct which contains the DNA sequence which encodes CD4 linked to human IgG1 at the Esp site upstream of the hinge region (fusion protein CD4E$\gamma$1) is depicted in Table 2. The plasmid containing this genetic construct (pCD4E$\gamma$1) has been deposited in E. coli (MC1061/P3) at the ATCC under the terms of the Budapest Treaty and given accession number 67610.

A third genetic construct which contains the DNA sequence which encodes CD4 linked to human IgM at the Mst2 site upstream of the CH1 region (fusion protein CD4M$\mu$) is depicted in Table 3. The plasmid containing this genetic construct (PCD4M$\mu$) has been deposited in E. coli (MC1061/P3) at the ATCC under the terms of the Budapest Treaty and given accession number 67608.

A fourth genetic construct which contains the DNA sequence which encodes CD4 linked to human IgM at the Pst site upstream of the CH2 region (fusion protein CD4P$\mu$) is depicted in Table 4. The plasmid containing this genetic construct (PCD4P$\mu$) has been deposited in E. coli (MC1061/P3) at the ATCC under the terms of the Budapest Treaty and given accession number 67609.

A fifth genetic construct which contains the DNA sequence which encodes CD4 linked to human IgG1 at the Ban1 site downstream from the hinge region (fusion protein CD4B$\gamma$1) is depicted in Table 5.

Two similar constructs were prepared from the human IgM heavy chain constant region by fusion with the introns upstream of the μ CH1 and CH2 domains at an MstII site and a PstI site respectively. The fusions were made by joining the PstI site of the CD4/IgG1 construct fused at the Esp site in IgG1 gene to the MstII and Pst sites in the IgM gene. In the first instance, this was performed by treatment of the Pst end with T4 DNA Polymerase and the MstII end with E. coli DNA Polymerase, followed by ligation; and in the second instance, by ligation alone.

Immunoprecipitation of the fusion proteins with a panel of monoclonal antibodies directed against CD4 epitopes showed that all of the epitopes were preserved. A specific high affinity association is demonstrated between the chimeric molecules and HIV envelope proteins expressed on the surface of cells transfected with an attenuated (reverse transcriptase deleted) proviral construct.

Table 1

```
      F N                                           S                 B
      N S                 B        M        H       DHA               S
      U P                 B        N        G       RAU               T
      4 B                 V        L        A       AE9               X
      H 2                 1        1        1       236               1
                                                     /
     GCCTGTTTGAGAAGCAGCGGGCAAGAAAGACGCAAGCCCAGAGGCCCTGCCATTTCTGTG
   1 --------+---------+---------+---------+---------+---------+ 60
     CGGACAAACTCTTCGTCGCCCGTTCTTTCTGCGTTCGGGTCTCCGGGACGGTAAAGACAC

      B     PS                    S                      M    HM        S
     DBS  ADNPA        D        DHNA                     N    AN       HNC
     DAP  VRLUU        D        RALU                     N    AN       PCR
     EN1  AAAM9        E        AEA9                     L    EL       AIF
     122  22416        1        2346                     1    31       211
      /    / / //               /                             /         /
     GGCTCAGGTCCCTACTGGCTCAGGCCCCTGCCTCCCTCGGCAAGGCCACAATGAACCGGG
  61 --------+---------+---------+---------+---------+---------+ 120
     CCGAGTCCAGGGATGACCGAGTCCGGGGACGGAGGGAGCCGTTCCGGTGTTACTTGGCCC

                                                     M   N   R   G  -
      H                          F                   F
      I                 B        N        HH         N  M   D
      N                 B        U        HA         U  N   D
      F                 V        4        AE         4  L   E
      1                 1        H        12         H  1   1
     GAGTCCCTTTTAGGCACTTGCTTCTGGTGCTGCAACTGGCGCTCCTCCCAGCAGCCACTC
 121 --------+---------+---------+---------+---------+---------+ 180
     CTCAGGGAAAATCCGTGAACGAAGACCACGACGTTGACCGCGAGGAGGGTCGTCGGTGAG

         V   P   F   R   H   L   L   L   V   L   Q   L   A   L   L   P   A   A   T   Q  -
         B         E   E                                           R   A
         B         C   C                                           S   L
         V         0   0                                           A   U
         1         K   K                                           1   1
     AGGGAAAGAAAGTGGTGCTGGGCAAAAAAGGGGATACAGTGGAACTGACCTGTACAGCTT
 181 --------+---------+---------+---------+---------+---------+ 240
     TCCCTTTCTTTCACCACGACCCGTTTTTTCCCCTATGTCACCTTGACTGGACATGTCGAA

         G   K   K   V   V   L   G   K   K   G   D   T   V   E   L   T   C   T   A   S  -
```

```
                          M M                                        H
                          B B                                        I
                          0 0                                        N
                          2 2                                        F
                                                                     1
      CCCAGAAGAAGAGCATACAATTCCACTGGAAAAACTCCAACCAGATAAAGATTCTGGGAA
241   ---------+---------+---------+---------+---------+---------+   300
      GGGTCTTCTTCTCGTATGTTAAGGTGACCTTTTTGAGGTTGGTCTATTTCTAAGACCCTT

        Q   K   K   S   I   Q   F   H   W   K   N   S   N   Q   I   K   I   L   G   N  -

                    B                   S                   S   F   H
                  NBS         F       AA          A         A   N H I
                  LAP         0       VU          L         U   U H N
                  AN1         K       A9          U         3   D A F
                  422         1       26          1         A   2 1 1
                   /                   /
      ATCAGGGCTCCTTCTTAACTAAAGGTCCATCCAAGCTGAATGATCGCGCTGACTCAAGAA
301   ---------+---------+---------+---------+---------+---------+   360
      TAGTCCCGAGGAAGAATTGATTTCCAGGTAGGTTCGACTTACTAGCGCGACTGAGTTCTT

        Q   G   S   F   L   T   K   G   P   S   K   L   N   D   R   A   D   S   R   R  -

                    S                       S           H               H
                  MANAS                   BA            I   A           I D
                  BVLUT                   CU            N   F           N D
                  0AA9Y                   L3            F   L           F E
                  22461                   1A            1   2           1 1
                   /                       /
      GAAGCCTTTGGGACCAAGGAAACTTCCCCCTGATCATCAAGAATCTTAAGATAGAAGACT
361   ---------+---------+---------+---------+---------+---------+   420
      CTTCGGAAACCCTGGTTCCTTTGAAGGGGGACTAGTAGTTCTTAGAATTCTATCTTCTGA

        S   L   W   D   Q   G   N   F   P   L   I   I   K   N   L   K   I   E   D   S  -

                                          S
                    M           M       AMAM                   M
                    B           N       VNUN                   A
                    0           L       AL9L                   E
                    2           1       2161                   1
                                         //
      CAGATACTTACATCTGTGAAGTGGAGGACCAGAAGGAGGAGGTGCAATTGCTAGTGTTCG
421   ---------+---------+---------+---------+---------+---------+   480
      GTCTATGAATGTAGACACTTCACCTCCTGGTCTTCCTCCTCCACGTTAACGATCACAAGC

        D   T   Y   I   C   E   V   E   D   Q   K   E   E   V   Q   L   L   V   F   G  -
```

```
                                        B                             S
                                        S                             T
                                        P                             Y
                                        M                             1
                                        1
     GATTGACTGCCAACTCTGACACCCACCTGCTTCAGGGGCAGAGCCTGACCCTGACCTTGG
481  ------------+---------+---------+---------+---------+---------+ 540
     CTAACTGACGGTTGAGACTGTGGGTGGACGAAGTCCCCGTCTCGGACTGGGACTGGAACC

      L  T  A  N  S  D  T  H  L  L  Q  G  Q  S  L  T  L  T  L  E -


         B   BS                                  H
         BS  SC              D              M     I     S
         AP  TR              D              N     N     T
         N1  NF              E              L     F     Y
         22  11              1              1     1     1
          /   /
     AGAGCCCCCCTGGTAGTAGCCCCTCAGTGCAATGTAGGAGTCCAAGGGGTAAAAACATAC
541  ------------+---------+---------+---------+---------+---------+ 600
     TCTCGGGGGGACCATCATCGGGGAGTCACGTTACATCCTCAGGTTCCCCATTTTTGTATG

      S  P  P  G  S  S  P  S  V  Q  C  R  S  P  R  G  K  N  I  Q -


                             N         BBH S  B           BS
                  M   MD     ASP     A  BSSGSC S    B  N   SC
                  B   ND     LPV     L  APTIAR T    A  L   TR
                  0   LE     UBU     U  N1NACF X    N  A   NF
                  2   11     122     1  221111 1    1  4   11
                             //         / ///               /
     AGGGGGGGGAAGACCCTCTCCGTGTCTCAGCTGGAGCTCCAGGATAGTGGCACCTGGACAT
601  ------------+---------+---------+---------+---------+---------+ 660
     TCCCCCCCCTTCTGGGAGAGGCACAGAGTCGACCTCGAGGTCCTATCACCGTGGACCTGTA

      G  G  K  T  L  S  V  S  Q  L  E  L  Q  D  S  G  T  W  T  C -

      N
      NS                     M                     NM  A
      LP                     B                     HA  L
      AH                     0                     EE  U
      31                     2                     11  1
       /
     GCACTGTCTTGCAGAACCAGAAGAAGGTGGAGTTCAAAATAGACATCGTGGTGCTAGCTT
661  ------------+---------+---------+---------+---------+---------+ 720
     CGTGACAGAACGTCTTGGTCTTCTTCCACCTCAAGTTTTATCTGTAGCACCACGATCGAA

      T  V  L  Q  N  Q  K  K  V  E  F  K  I  D  I  V  V  L  A  F -
```

14

```
       HS        M  M
       AT        N  N
       EU        L  L
       31        1  1
        /
TCCAGAAGGCCTCCAGCATAGTCTATAAGAAAGAGGGGGAACAGGTGGAGTTCTCCTTCC
721 ---------+---------+---------+---------+---------+---------+ 780
AGGTCTTCCGGAGGTCGTATCAGATATTCTTTCTCCCCCTTGTCCACCTCAAGAGGAAGG

     Q  K  A  S  S  I  V  Y  K  K  E  G  E  Q  V  E  F  S  F  P -

                      A              A     M
                      L              L     N
                      U              U     L
                      1              1     1
CACTCGCCTTTACAGTTGAAAAGCTGACGGGCAGTGGCGAGCTGTGGTGGCAGGCGGAGA
781 ---------+---------+---------+---------+---------+---------+ 840
GTGAGCGGAAATGTCAACTTTTCGACTGCCCGTCACCGCTCGACACCACCGTCCGCCTCT

     L  A  F  T  V  E  K  L  T  G  S  G  E  L  W  W  Q  A  E  R -

                   P  S
       H     M FM  A                            M
       P     N LN  U                            B
       H     L ML  3                            0
       1     1 11  A                            2
GGGCTTCCTCCTCCAAGTCTTGGATCACCTTTGACCTGAAGAACAAGGAAGTGTCTGTAA
841 ---------+---------+---------+---------+---------+---------+ 900
CCCGAAGGAGGAGGTTCAGAACCTAGTGGAAACTGGACTTCTTGTTCCTTCACAGACATT

     A  S  S  S  K  S  W  I  T  F  D  L  K  N  K  E  V  S  V  K -

       B      BS    PS
       SM     SCADNPAD    A                A  H
       TA     TRVRLUUD    L                L  P
       EE     NFAAAM9E    U                U  H
       23     11224161    1                1  1
        /      / / //
AACGGGTTACCCAGGACCCTAAGCTCCAGATGGGCAAGAAGCTCCCGCTCCACCTCACCC
901 ---------+---------+---------+---------+---------+---------+ 960
TTGCCCAATGGGTCCTGGGATTCGAGGTCTACCCGTTCTTCGAGGGCGAGGTGGAGTGGG

     R  V  T  Q  D  P  K  L  Q  M  G  K  K  L  P  L  H  L  T  L -
```

15

```
         BS                                      BSS
    M    SC HS       D           M    H          SCAHM
    N    TR AT       D           N    P          TRUAN
    L    NF EU       E           L    H          NF9EL
    1    11 31       1           1    1          11631
    /    /                                       /   /
    TGCCCCAGGCCTTGCCTCAGTATGCTGGCTCTGGAAACCTCACCCTGGCCCCTTGAAGCGA
961 -------+---------+---------+---------+---------+---------+ 1020
    ACGGGGTCCGGAACGGAGTCATACGACCGAGACCTTTGGAGTGGGACCGGGGAACTTCGCT

     P   Q   A   L   P   Q   Y   A   G   S   G   N   L   T   L   A   L   E   A   K  -

                         S           BS
                         F           SC                      H   D       A
                         A           TR                      P   D       L
                         N           NF                      H   E       U
                         1           11                      1   1       1
                                     /
     AAACAGGAAAGTTGCATCAGGAAGTGAACCTGGTGGTGATGAGAGCCACTCAGCTCCAGA
1021 -------+---------+---------+---------+---------+---------+ 1080
     TTTGTCCTTTCAACGTAGTCCTTCACTTGGACCACCACTACTCTCGGTGAGTCGAGGTCT

     T   G   K   L   H   Q   E   V   N   L   V   V   M   R   A   T   Q   L   Q   K  -

                             PS          S
             M               ADNNPA      DF  AM      DE  A
             N               VRLLUU      DA  LN      DS  L
             L               AAAAM9      EN  UL      EP  U
             1               224416      11  11      11  1
                             /////       /   /       /
     AAAATTTGACCTGTGAGGTGTGGGGACCCACCTCCCCTAAGCTGATGCTGAGCTTGAAAC
1081 -------+---------+---------+---------+---------+---------+ 1140
     TTTTAAACTGGACACTCCACACCCCTGGGTGGAGGGGATTCGACTACGACTCGAACTTTG

     N   L   T   C   E   V   W   G   P   T   S   P   K   L   M   L   S   L   K   L  -

         M                   T           H           M           DM
         N                   A           P           N           DS
         L                   Q           A           L           ET
         1                   1           2           1           12
                                                                 /
     TGGAGAACAAGGAGGCAAAGGTCTCGAAGCGGGAGAAGCCGGTGTGGGTGCTGAACCCTG
1141 -------+---------+---------+---------+---------+---------+ 1200
     ACCTCTTGTTCCTCCGTTTCCAGAGCTTCGCCCTCTTCGGCCACACCCACGACTTGGGAC

     E   N   K   E   A   K   V   S   K   R   E   K   P   V   W   V   L   N   P   E  -
```

16

```
                                 H               PS         H
             F   D   M   I   A   ADPA       I
             D   D   A   N   V   VRUU       N
             K   E   E   F   A   AAM9       F
             1   1   3   1   1   2216       1
                         ///
AGGCGGGGATGTGGCAGTGTCTGCTGAGTGACTCGGGACAGGTCCTGCTGGAATCCAACA
1201 ---------+---------+---------+---------+---------+---------+ 1260
TCCGCCCCTACACCGTCACAGACGACTCACTGAGCCCTGTCCAGGACGACCTTAGGTTGT

     A   G   M   W   Q   C   L   L   S   D   S   G   Q   V   L   L   E   S   N   I-

                     S           SA    BHF BS                    H
                     ANA         HNCP  SGNMAANXA              RSD I A
                     VLU         PCRA  PIUNMULHV              SCD N L
                     AA9         AIFL  1ADLH3ADA              AAE D U
                     236         2111  21211A421              111 3 1
                      //          //    / / / /                /
TCAAGGTTCTGCCCACATGGTCCACCCCGGTGCACGCGGATCCCGAGGGTGAGTACTAAG
1261 ---------+---------+---------+---------+---------+---------+ 1320
AGTTCCAAGACGGGTGTACCAGGTGGGGCCACGTGCGCCTAGGGCTCCCACTCATGATTC

         K   V   L   P   T   W   S   T   P   V   H   A   D   P   E

                         BS                                        B
         H               H SC HS    S         M         M   D      S
         P               A TR AT    T         N         N   D      P
         H               E NF EU    Y         L         L   E      M
         1               3 11 31    1         1         1   1      1
         /                 /  /
CTTTCTGGGGCAGGCCAGGCCTGACCTTGGCTTTGGGGCAGGGAGGGGGCTAAGGTGAGG
1321 ---------+---------+---------+---------+---------+---------+ 1380
GAAAGACCCCGTCCGGTCCGGACTGGAACCGAAACCCCGTCCCTCCCCCGATTCCACTCC

             B           A   BH                    B       P
             BASHBHHHN   P   SG                    N   BS  F            H
             AHPHBAPAL   A   PI                    L   AP  L            G
             NAMAEEHRA   L   1A                    A   N1  M            A
             121112114   1   21                    3   22  1            1
              /   /////       /                        /
CAGGTGGCGCCAGCAGGTGCACACCCAATGCCCATGAGCCCAGACACTGGACGCTGAACC
1381 ---------+---------+---------+---------+---------+---------+ 1440
GTCCACCGCGGTCGTCCACGTGTGGGTTACGGGTACTCGGGTCTGTGACCTGCGACTTGG

             F               BS    S           B SS      B S          FN
             N       M       SC DNHA       H SMAAHNABSAC          NS
             U       N       TR RLAU       H TNUUALPAPLR          UP
             D       L       NF AAE9       A NL99EAAN1UF          DB
             2       1       11 2436       1 11663412211          22
                                /            /  /// /              /
TCGCGGACAGTTAAGAACCCAGGGGCCTCTGCGCCTGGGCCCAGCTCTGTCCCACACCGC
1441 ---------+---------+---------+---------+---------+---------+ 1500
AGCGCCTGTCAATTCTTGGGTCCCCGGAGACGCGGACCCGGGTCGAGACAGGGTGTGGCG
```

```
                                  F                      BSS              BS
        MS        BNN             NM      S BMDMHNABSAA            SCB
        AA        ALL             UN      T BBRNALPAPUU            TRA
        EC        NAA             4L      Y VOALEAAN199            NFN
        32        134             H1      1 12213412266            111
         /         /              /         / /////                /
        GGTCACATGGCACCACCTCTCTTGCAGCCTCCACCAAGGGCCCATCGGTCTTCCCCCTGG
1501    ---------+---------+---------+---------+---------+---------+  1560
        CCAGTGTACCGTGGTGGAGAGAACGTCGGAGGTGGTTCCCGGGTAGCCAGAAGGGGGACC
                          A  S  T  K  G  P  S  V  F  P  L  A -


                  BH           B  NFS   BS     F   BS
        N     M   MSG         MSB SNAH  SC     N   SC
        L     N   NPI         NPB PUUA  TR     U   TR
        A     L   L1A         L1V B49E  NF     4   NF
        4     1   121         121 2H63  11     H   11
                   /                     /          /
        CACCCTCCTCCAAGAGCACCTCTGGGGGCACAGCGGCCCTGGGCTGCCTGGTCAAGGACT
1561    ---------+---------+---------+---------+---------+---------+  1620
        GTGGGAGGAGGTTCTCGTGGAGACCCCCGTGTCGCCGGGACCCGACGGACCAGTTCCTGA
         P  S  S  K  S  T  S  G  G  T  A  A  L  G  C  L  V  K  D  Y -


                                               NF   A   BH
              H M   T    H    D   BANHBHN       SN   P   SG
              P A   T    P    D   AHAHBAL       PU   A   PI
              A E   H    H    E   NARAEEA       B4   L   1A
              2 3   1    1    1   1211124       2H   1   21
                                    /    //                /
        ACTTCCCCGAACCGGTGACGGTGTCGTGGAACTCAGGCGCCCTGACCAGCGGCGTGCACA
1621    ---------+---------+---------+---------+---------+---------+  1680
        TGAAGGGGCTTGGCCACTGCCACAGCACCTTGAGTCCGCGGGACTGGTCGCCGCACGTGT
         F  P  E  P  V  T  V  S  W  N  S  G  A  L  T  S  G  V  H  T -


                    S               H              F   B
              HNC             DM    I   M      D    N   M  SM    B
              PCR             DS    N   N      D    U   N  TA    B
              AIF             ET    F   L      E    4   L  EE    V
              211             12    1   1      1    H   1  23    1
               //                   /
        CCTTCCCGGCTGTCCTACAGTCCTCAGGACTCTACTCCCTCAGCAGCGTGGTGACCGTGC
1681    ---------+---------+---------+---------+---------+---------+  1740
        GGAAGGGCCGACAGGATGTCAGGAGTCCTGAGATGAGGGAGTCGTCGCACCACTGGCACG
         F  P  A  V  L  Q  S  S  G  L  Y  S  L  S  S  V  V  T  V  P -


        B     F  B       B
        SH    N ASM  B  NSB                   H
        PP    U LTN  A  LPB          M        I
        1H    4 UXL  N  A1V          A        N
        21    H 111  1  421          E        F
               /                     2        1
        CCTCCAGCAGCTTGGGCACCCAGACCTACATCTGCAACGTGAATCACAAGCCCAGCAACA
1741    ---------+---------+---------+---------+---------+---------+  1800
        GGAGGTCGTCGAACCCGTGGGTCTGGATGTAGACGTTGCACTTAGTGTTCGGGTCGTTGT
         S  S  S  L  G  T  Q  T  Y  I  C  N  V  N  H  K  P  S  N  T -
```

```
        S                 M              HM   HM
        T                 N              AN   PN
        Y                 L              EL   HL
        1                 1              31   11
        CCAAGGTGGACAAGAAAGTTGGTGAGAGGCCAGCACAGGGAGGGAGGGTGTCTGCTGGAA
1801    --------+---------+---------+---------+---------+---------+  1860
        GGTTCCACCTGTTCTTTCAACCACTCTCCGGTCGTGTCCCTCCCTCCCACAGACGACCTT


        K   V. D   K   K   V

                  E           BS              SS      F           BS   F
            DE    CHH   F     SC          HHNCF       N           BSC  N
            DS    OHA   O     TR          PGCRA       U           BTR  U
            EP    4AE   K     NF          AAIFN       4           VNF  4
            11    712   1     11          21111       H           111  H
             /           /                 //                      //
        GCAGGCTCAGCGCTCCTGCCTGGACGCATCCCGGCTATGCAGCCCCAGTCCAGGGCAGCA
1861    --------+---------+---------+---------+---------+---------+  1920
        CGTCCGAGTCGCGAGGACGGACCTGCGTAGGGCCGATACGTCGGGGTCAGGTCCCGTCGT


                  S                   S
            DBHMHNA             HMNCN           M           MNDM
            RBABPLU             PNCRL           N           NLDB
            AVEOHA9             ALIFA           L           LAEO
            2132146             21114           1           1312
             // //               //
        AGGCAGGCCCCGTCTGCCTCTTCACCCGGAGCCTCTGCCCGCCCCACTCATGCTCAGGGA
1921    --------+---------+---------+---------+---------+---------+  1980
        TCCGTCCGGGGCAGACGGAGAAGTGGGCCTCGGAGACGGGCGGGGTGAGTACGAGTCCCT


                      BS    P                       B         BS
                      SC    F               M  B  N  S        SC
                      TR    L               A  A  L  P        TR
                      NF    M               E  N  A  1        NF
                      11    1               1  1  4  2        11
                       /                                       /
        GAGGGTCTTCTGGCTTTTTCCCAGGCTCTGGGCAGGCACAGGCTAGGTGCCCCTAACCCA
1981    --------+---------+---------+---------+---------+---------+  2040
        CTCCCAGAAGACCGAAAAAGGGTCCGAGACCCGTCCGTGTCCGATCCACGGGGATTGGGT


            S         B                   B           B           S
            DHA       S                   DBS         S   M     HNC      A
            RAU       P                   DAP         P   N     PCR      V
            AE9       M                   EN1         M   L     AIF      A
            236       1                   122         1   1     211      2
             /                             /                     /
        GGCCCTGCACACAAAGGGGCAGGTGCTGGGCTCAGACCTGCCAAGAGCCATATCCGGGAG
2041    --------+---------+---------+---------+---------+---------+  2100
        CCGGGACGTGTGTTTCCCCGTCCACGACCCGAGTCTGGACGGTTCTCGGTATAGGCCCTC
```

```
        PS
        DNPA              D                   H                    D    A    M
        RLUU              D                   A                    D    L    N
        AAM9              E                   E                    E    U    L
        2416              1                   3                    1    1    1
        / //
        GACCCTGCCCCTGACCTAAGCCCACCCCAAAGGCCAAACTCTCCACTCCCTCAGCTCGGA
2101    ----------+----------+----------+----------+----------+----------+  2160
        CTGGGACGGGGACTGGATTCGGGTGGGGTTTCCGGTTTGAGAGGTGAGGGAGTCGAGCCT


                          H
                          I     M    MM                            P    BS
                          N     N    AB                            S    AP
                          F     L    E0                            T    N1
                          1     1    32                            1    22
                                      /                                 /
        CACCTTCTCTCCTCCCAGATTCCAGTAACTCCCAATCTTCTCTCTGCAGAGCCCAAATCT
2161    ----------+----------+----------+----------+----------+----------+  2220
        GTGGAAGAGAGGAGGGTCTAAGGTCATTGAGGGTTAGAAGAGAGACGTCTCGGGTTTAGA

                                                           E    P    K    S    -

                          N          BBS              BS
        M                 NS         SSC              SC   HS              M
        A                 LP         PTR              TR   AT              N
        E                 AH         1NF              NF   EU              L
        3                 31         211              11   31              1
                           /          /               /    /
        TGTGACAAAACTCACACATGCCCACCGTGCCCAGGTAAGCCAGCCCAGGCCTCGCCCTCC
2221    ----------+----------+----------+----------+----------+----------+  2280
        ACACTGTTTTGAGTGTGTACGGGTGGCACGGGTCCATTCGGTCGGGTCCGGAGCGGGAGG
        C    D    K    T    H    T    C    P    P    C    P


                               B                    BS   S         S         S
                     A    M    B N  SM  F           SC   F    DHNA       HNC
                     L    N    A L  PA  O           TR   A    RALU       PCR
                     U    L    N A  1E  K           NF   N    AEA9       AIF
                     1    1    1 4  21  1           11   1    2346       211
                                                     /              /          /
        AGCTCAAGGCGGGACAGGTGCCCTAGAGTAGCCTGCATCCAGGGACAGGCCCCAGCCGGG
2281    ----------+----------+----------+----------+----------+----------+  2340
        TCGAGTTCCGCCCTGTCCACGGGATCTCATCGGACGTAGGTCCCTGTCCGGGGTCGGCCC


                                                   BS        S
                     A  M  M              M    D        M    SC   M  ANA M
                     F  A  B              N    D        N    TR   B  VLU B
                     L  E  0              L    E        L    NF   0  AA9 0
                     3  2  2              1    1        1    11   2  246 2
                                                              /         /
        TGCTGACACGTCCACCTCCATCTCTTCCTCAGCACCTGAACTCCTGGGGGGACCGTCAGT
2341    ----------+----------+----------+----------+----------+----------+  2400
        ACGACTGTGCAGGTGGAGGTAGAGAAGGAGTCGTGGACTTGAGGACCCCCCTGGCAGTCA
                               A    P    E    L    L    G    G    P    S    V    -
```

```
                                S              SS
              M         S       AN    M  HMANNAC  DM   M
              N         T       UL    N  PNVCLUR  DS    A
              L         Y       3A    L  ALAIA9F  ET    E
              1         1       A3    1  2121461  12    3
                                 /    / ///       /
      CTTCCTCTTCCCCCCAAAACCCAAGGACACCCTCATGATCTCCCGGACCCCTGAGGTCAC
2401  ---------+---------+---------+---------+---------+---------+  2460
      GAAGGAGAAGGGGGGTTTTGGGTTCCTGTGGGAGTACTAGAGGGCCTGGGGACTCCAGTG

       F  L  F  P  P  K  P  K  D  T  L  M  I  S  R  T  P  E  V  T  -


         N
        NS         M         M         DM   M                RM    M
        LP         A         N         DS   B                SA    N
        AH         E         L         ET   0                AE    L
        31         2         1         12   2                12    1
        /                              /
      ATGCGTGGTGGTGGACGTGAGCCACGAAGACCCTGAGGTCAAGTTCAACTGGTACGTGGA
2461  ---------+---------+---------+---------+---------+---------+  2520
      TACGCACCACCACCTGCACTCGGTGCTTCTGGGACTCCAGTTCAAGTTGACCATGCACCT

       C  V  V  V  D  V  S  H  E  D  P  E  V  K  F  N  W  Y  V  D  -


                            F  FN
                    M       N  NSS              R              M  R
                    N       U  UPA              S              A  S
                    L       4  DBC              A              E  A
                    1       H  222              1              2  1
                               //
      CGGCGTGGAGGTGCATAATGCCAAGACAAAGCCGCGGGAGGAGCAGTACAACAGCACGTA
2521  ---------+---------+---------+---------+---------+---------+  2580
      GCCGCACCTCCACGTATTACGGTTCTGTTTCGGCGCCCTCCTCGTCATGTTGTCGTGCAT

       G  V  E  V  H  N  A  K  T  K  P  R  E  E  Q  Y  N  S  T  Y  -


         S                              BS
       HNC HH                   M       SC                     R
       PCR GP                   N       TR                     S
       AIF AH                   L       NF                     A
       211 11                   1       11                     1
         /                              /
      CCGGGTGGTCAGCGTCCTCACCGTCCTGCACCAGGACTGGCTGAATGGCAAGGAGTACAA
2581  ---------+---------+---------+---------+---------+---------+  2640
      GGCCCACCAGTCGCAGGAGTGGCAGGACGTGGTCCTGACCGACTTACCGTTCCTCATGTT

       R  V  V  S  V  L  T  V  L  H  Q  D  W  L  N  G  K  E  Y  K  -


                                       M    T
                                       N    A
                                       L    Q
                                       1    1
      GTGCAAGGTCTCCAACAAAGCCCTCCCAGCCCCCATCGAGAAAACCATCTCCAAAGCCAA
2641  ---------+---------+---------+---------+---------+---------+  2700
      CACGTTCCAGAGGTTGTTTCGGGAGGGTCGGGGGTAGCTCTTTTGGTAGAGGTTTCGGTT
       C  K  V  S  N  K  A  L  P  A  P  I  E  K  T  I  S  K  A  K  -
```

21

```
              P S                 S                              S
          ADNNPMA             A H M       N        HHN       BSAH
          VRLLUNU             U A N       L        APA       GFUA
          AAAAML9             9 E L       A        EAE       LI9E
          2244116             6 3 1       3        321       1163
            //// /                                            /
          AGGTGGGACCCGTGGGGTGCGAGGGCCACATGGACAGAGGCCGGCTCGGCCCACCCTCTG
     2701 ---------+----------+----------+----------+----------+---------+ 2760
          TCCACCCTGGGCACCCCACGCTCCCGGTGTACCTGTCTCCGGCCGAGCCGGGTGGGAGAC


                    N
          D M M     S     R                    M   N       A           B
          D N A     P     S                    N   U       V           B
          E L E     B     A                    L   4       A           V
          1 1 3     2     1                    1   H       1           1
          CCCTGAGAGTGACCGCTGTACCAACCTCTGTCCTACAGGGCAGCCCCGAGAACCACAGGT
     2761 ---------+----------+----------+----------+----------+---------+ 2820
          GGGACTCTCACTGGCGACATGGTTGGAGACAGGATGTCCCGTCGGGGCTCTTGGTGTCCA


                                             G  Q  P  R  E  P  Q  V  -

                    SS                       BS                      BS
          R F     AHNNCCS    A      F        SC                      SC
          S 0     VPCCRRM    L      0        TR                      TR
          A K     AAIIFFA    U      K        NF                      NF
          1 1   .  1211111   1      1        11                      11
                  /////                       /                       /
          GTACACCCTGCCCCCATCCCGGGATGAGCTGACCAAGAACCAGGTCAGCCTGACCTGCCT
     2821 ---------+----------+----------+----------+----------+---------+ 2880
          CATGTGGGACGGGGGTAGGGCCCTACTCGACTGGTTCTTGGTCCAGTCGGACTGGACGGA


           Y  T  L  P  P  S  R  D  E  L  T  K  N  Q  V  S  L  T  C  L  -

           B                                               F
           S                                               N   H
           P                                               U   P
           M                                               4   A
           1                                               H   2
          GGTCAAAGGCTTCTATCCCAGCGACATCGCCGTGGAGTGGGAGAGCAATGGGCAGCCGGA
     2881 ---------+----------+----------+----------+----------+---------+ 2940
          CCAGTTTCCGAAGATAGGGTCGCTGTAGCGGCACCTCACCCTCTCGTTACCCGTCGGCCT


           V  K  G  F  Y  P  S  D  I  A  V  E  W  E  S  N  G  Q  P  E  -

                                        H
           B                          M I   M         N           H
           B                          N N   B         L           P
           V                          L F   0         A           H
           1                          1 1   2         4           1
          GAACAACTACAAGACCACGCCTCCCGTGCTGGACTCCGACGGCTCCTTCTTCCTCTACAG
     2941 ---------+----------+----------+----------+----------+---------+ 3000
          CTTGTTGATGTTCTGGTGCGGAGGGCACGACCTGAGGCTGCCGAGGAAGAAGGAGATGTC


           N  N  Y  K  T  T  P  P  V  L  D  S  D  G  S  F  F  L  Y  S  -
```

22

```
                B               F                       S
        M A     S               NM      MBX             NF  M
        N L     S               UB      ABM             LA  N
        L U     P               4D      EVN             AN  L
        1 1     M               H2      211             31  1
                1                                        /
        CAAGCTCACCGTGGACAAGAGCAGGTGGCAGCAGGGGAACGTCTTCTCATGCTCCGTGAT
3001    ----------+----------+----------+----------+----------+----------+  3060
        GTTCGAGTGGCACCTGTTCTCGTCCACCGTCGTCCCCTTGCAGAAGAGTACGAGGCACTA

        K   L   T   V   D   K   S   R   W   Q   Q   G   N   V   F   S   C   S   V   M   -

                                                                S
        N N                                     M   M   HNC
        S L                                     B   N   PCR
        I A                                     0   L   AIF
        1 3                                     2   1   211
                                                         /
        GCATGAGGCTCTGCACAACCACTACACGCAGAAGAGCCTCTCCCTGTCTCCGGGTAAATG
3061    ----------+----------+----------+----------+----------+----------+  3120
        CGTACTCCGAGACGTGTTGGTGATGTGCGTCTTCTCGGAGAGGGACAGAGGCCCATTTAC

        H   E   A   L   H   N   H   Y   T   Q   K   S   L   S   L   S   P   G   K   *

        CXHHN
        FMAPA
        RAEAE
        13321
             /
        AGTGCGACGGCCG
3121    ----------+---  3133
        TCACGCTGCCGGC
```

Table 2

```
 F N                                       S              B
 N S                    B        M       H  DHA           S
 U P                    B        N       G  RAU           T
 4 B                    V        L       A  AE9           X
 H 2                    1        1       1  236           1
                                            /
  GCCTGTTTGAGAAGCAGCGGGCAAGAAAGACGCAAGCCCAGAGGCCCTGCCATTTCTGTG
1 ---------+---------+---------+---------+---------+---------+ 60
  CGGACAAACTCTTCGTCGCCCGTTCTTTCTGCGTTCGGGTCTCCGGGACGGTAAAGACAC


   B    PS                                                  S
 DBS  ADNPA          D    DHNA                  M   HM     HNC
 DAP  VRLUU          D    RALU                  N   AN     PCR
 EN1  AAAM9          E    AEA9                  L   EL     AIF
 122  22416          1    2346                  1   31     211
  /   / //                 /                        /       /
   GGCTCAGGTCCCTACTGGCTCAGGCCCCTGCCTCCCTCGGCAAGGCCACAATGAACCGGG
61 ---------+---------+---------+---------+---------+---------+ 120
   CCGAGTCCAGGGATGACCGAGTCCGGGGACGGAGGGAGCCGTTCCGGTGTTACTTGGCCC


                                               M   N   R   G  -
   H                                           F
   I                    B             F        N M   D
   N                    B             N        U N   D
   F                    V             U        4 L   E
   1                    1             4        H 1   1
                                      H       12
   GAGTCCCTTTTAGGCACTTGCTTCTGGTGCTGCAACTGGCGGCTCCTCCCAGCAGCCACTC
121 ---------+---------+---------+---------+---------+---------+ 180
    CTCAGGGAAAATCCGTGAACGAAGACCACGACGTTGACCGCGAGGAGGGTCGTCGGTGAG


    V   P   F   R   H   L   L   L   V   L   Q   L   A   L   L   P   A   A   T   Q -

    B    E  E                                          R   A
    B    C  C                                          S   L
    V    0  0                                          A   U
    1    K  K                                          1   1
    AGGGAAAGAAAGTGGTGCTGGGCAAAAAAGGGGATACAGTGGAACTGACCTGTACAGCTT
181 ---------+---------+---------+---------+---------+---------+ 240
    TCCCTTTCTTTCACCACGACCCGTTTTTTCCCCTATGTCACCTTGACTGGACATGTCGAA


    G   K   K   V   V   L   G   K   K   G   D   T   V   E   L   T   C   T   A   S -


                        M  M                            H
                        B  B                            I
                        0  0                            N
                        2  2                            F
                                                        1
    CCCAGAAGAAGAGCATACAATTCCACTGGAAAAACTCCAACCAGATAAAGATTCTGGGAA
241 ---------+---------+---------+---------+---------+---------+ 300
    GGGTCTTCTTCTCGTATGTTAAGGTGACCTTTTTGAGGTTGGTCTATTTCTAAGACCCTT


    Q   K   K   S   I   Q   F   H   W   K   N   S   N   Q   I   K   I   L   G   N -
```

```
                B                    S                    S    F    H
                NBS        F         AA         A         A    N H  I
                LAP        0         VU         L         U    U H  N
                AN1        K         A9         U         3    D A  F
                422        1         26         1         A    2 1  1
                 /                    /
          ATCAGGGCTCCTTCTTAACTAAAGGTCCATCCAAGCTGAATGATCGCGCTGACTCAAGAA
      301 ---------+---------+---------+---------+---------+---------+ 360
          TAGTCCCGAGGAAGAATTGATTTCCAGGTAGGTTCGACTTACTAGCGCGACTGAGTTCTT
            Q  G  S  F  L  T  K  G  P  S  K  L  N  D  R  A  D  S  R  R -


                           S                    S         H    A         H
                           MANAS                BA        I    A         I    D
                           BVLUT                CU        N    F         N    D
                           0AA9Y                L3        F    L         F    E
                           22461                1A        1    2         1    1
                             /                    /
          GAAGCCTTTGGGACCAAGGAAACTTCCCCCTGATCATCAAGAATCTTAAGATAGAAGACT
      361 ---------+---------+---------+---------+---------+---------+ 420
          CTTCGGAAACCCTGGTTCCTTTGAAGGGGGACTAGTAGTTCTTAGAATTCTATCTTCTGA
            S  L  W  D  Q  G  N  F  P  L  I  I  K  N  L  K  I  E  D  S -


                                          S
                M         M               AMAM                    M
                B         N               VNUN                    A
                0         L               AL9L                    E
                2         1               2161                    1
                                           //
          CAGATACTTACATCTGTGAAGTGGAGGACCAGAAGGAGGAGGTGCAATTGCTAGTGTTCG
      421 ---------+---------+---------+---------+---------+---------+ 480
          GTCTATGAATGTAGACACTTCACCTCCTGGTCTTCCTCCTCCACGTTAACGATCACAAGC
            D  T  Y  I  C  E  V  E  D  Q  K  E  E  V  Q  L  L  V  F  G -


                                          B
                                          S                       S
                                          P                       T
                                          M                       Y
                                          1                       1
          GATTGACTGCCAACTCTGACACCCACCTGCTTCAGGGGCAGAGCCTGACCCTGACCTTGG
      481 ---------+---------+---------+---------+---------+---------+ 540
          CTAACTGACGGTTGAGACTGTGGGTGGACGAAGTCCCCGTCTCGGACTGGGACTGGAACC
             L  T  A  N  S  D  T  H  L  L  Q  G  Q  S  L  T  L  T  L  E -


                B    BS
                BS   SC              D         M         H
                AP   TR              D         N         I    S
                N1   NF              E         L         N    T
                22   11              1         1         F    Y
                 /    /                                  1    1
          AGAGCCCCCCTGGTAGTAGCCCCTCAGTGCAATGTAGGAGTCCAAGGGGTAAAAACATAC
      541 ---------+---------+---------+---------+---------+---------+ 600
          TCTCGGGGGGACCATCATCGGGGAGTCACGTTACATCCTCAGGTTCCCCATTTTTGTATG

             S  P  P  G  S  S  P  S  V  Q  C  R  S  P  R  G  K  N  I  Q -
```

```
                        N           BBH S   B              BS
            M    MD    ASP   A  BSSGSC   S   B  N       SC
            B    ND    LPV   L  APTIAR   T   A  L       TR
            0    LE    UBU   1  N1NACF   X   N  A       NF
            2    11    122   1  221111   1   1  4       11
                  //          /  / ///                    /
    AGGGGGGGGAAGACCCTCTCCGTGTCTCAGCTGGAGCTCCAGGATAGTGGCACCTGGACAT
601 ---------+---------+---------+---------+---------+---------+ 660
    TCCCCCCCTTCTGGGAGAGGCACAGAGTCGACCTCGAGGTCCTATCACCGTGGACCTGTA
      G  G  K  T  L  S  V  S  Q  L  E  L  Q  D  S  G  T  W  T  C -


        N
        NS                          M                    NM   A
        LP                          B                    HA   L
        AH                          0                    EE   U
        31                          2                    11   1
         /
    GCACTGTCTTGCAGAACCAGAAGAAGGTGGAGTTCAAAATAGACATCGTGGTGCTAGCTT
661 ---------+---------+---------+---------+---------+---------+ 720
    CGTGACAGAACGTCTTGGTCTTCTTCCACCTCAAGTTTTATCTGTAGCACCACGATCGAA
      T  V  L  Q  N  Q  K  K  V  E  F  K  I  D  I  V  V  L  A  F -


        HS              M  M
        AT              N  N
        EU              L  L
        31              1  1
         /
    TCCAGAAGGCCTCCAGCATAGTCTATAAGAAAGAGGGGGAACAGGTGGAGTTCTCCTTCC
721 ---------+---------+---------+---------+---------+---------+ 780
    AGGTCTTCCGGAGGTCGTATCAGATATTCTTTCTCCCCCTTGTCCACCTCAAGAGGAAGG
      Q  K  A  S  S  I  V  Y  K  K  E  G  E  Q  V  E  F  S  F  P -


                        A                   A       M
                        L                   L       N
                        U                   U       L
                        1                   1       1
    CACTCGCCTTTACAGTTGAAAAGCTGACGGGCAGTGGCGAGCTGTGGTGGCAGGCGGAGA
781 ---------+---------+---------+---------+---------+---------+ 840
    GTGAGCGGAAATGTCAACTTTTCGACTGCCCGTCACCGCTCGACACCACCGTCCGCCTCT
      L  A  F  T  V  E  K  L  T  G  S  G  E  L  W  W  Q  A  E  R -


                        P  S
            H   M FM  A                           M
            P   N LN  U                           B
            H   L ML  3                           0
            1   1 11  A                           2
    GGGCTTCCTCCTCCAAGTCTTGGATCACCTTTGACCTGAAGAACAAGGAAGTGTCTGTAA
841 ---------+---------+---------+---------+---------+---------+ 900
    CCCGAAGGAGGAGGTTCAGAACCTAGTGGAAACTGGACTTCTTGTTCCTTCACAGACATT
      A  S  S  S  K  S  W  I  T  F  D  L  K  N  K  E  V  S  V  K -
```

```
          B          BS    PS
          SM         SCADNPAD    A              A H
          TA         TRVRLUUD    L              L P
          EE         NFAAAM9E    U              U H
          23         11224161    1              1 1
          /          / / //
          AACGGGTTACCCAGGACCCTAAGCTCCAGATGGGCAAGAAGCTCCCGCTCCACCTCACCC
  901     ---------+---------+---------+---------+---------+---------+  960
          TTGCCCAATGGGTCCTGGGATTCGAGGTCTACCCGTTCTTCGAGGGCGAGGTGGAGTGGG
            R   V   T   Q   D   P   K   L   Q   M   G   K   K   L   P   L   H   L   T   L -


              BS
          M   SC HS         D              M   H              BSS
          N   TR AT         D              N   P              SCAHM
          L   NF EU         E              L   H              TRUAN
          1   11 31         1              1   1              NF9EL
               /  /                                           11631
                                                              / /
          TGCCCCAGGCCTTGCCTCAGTATGCTGGCTCTGGAAACCTCACCCTGGCCCCTTGAAGCGA
  961     ---------+---------+---------+---------+---------+---------+  1020
          ACGGGGTCCGGAACGGAGTCATACGACCGAGACCTTTGGAGTGGGACCGGGAACTTCGCT
            P   Q   A   L   P   Q   Y   A   G   S   G   N   L   T   L   A   L   E   A   K -


                             S            BS
                             F            SC             H D     A
                             A            TR             P D     L
                             N            NF             H E     U
                             1            11             1 1     1
                                          /
          AAACAGGAAAGTTGCATCAGGAAGTGAACCTGGTGGTGATGAGAGCCACTCAGCTCCAGA
  1021    ---------+---------+---------+---------+---------+---------+  1080
          TTTGTCCTTTCAACGTAGTCCTTCACTTGGACCACCACTACTCTCGGTGAGTCGAGGTCT
            T   G   K   L   H   Q   E   V   N   L   V   V   M   R   A   T   Q   L   Q   K -


                             PS            S
              M              ADNNPA        DF   AM        DE    A
              N              VRLLUU        DA   LN        DS    L
              L              AAAAM9        EN   UL        EP    U
              1              224416        11   11        11    1
                             /////         /    /         /
          AAAATTTGACCTGTGAGGTGTGGGGACCCACCTCCCCTAAGCTGATGCTGAGCTTGAAAC
  1081    ---------+---------+---------+---------+---------+---------+  1140
          TTTTAAACTGGACACTCCACACCCCTGGGTGGAGGGGATTCGACTACGACTCGAACTTTG
            N   L   T   C   E   V   W   G   P   T   S   P   K   L   M   L   S   L   K   L -


              M                    T             H              M      DM
              N                    A             P              N      DS
              L                    Q             A              L      ET
              1                    1             2              1      12
                                                                       /
          TGGAGAACAAGGAGGCAAAGGTCTCGAAGCGGGAGAAGCCGGTGTGGGTGCTGAACCCTG
  1141    ---------+---------+---------+---------+---------+---------+  1200
          ACCTCTTGTTCCTCCGTTTCCAGAGCTTCGCCCTCTTCGGCCACACCCACGACTTGGGAC
            E   N   K   E   A   K   V   S   K   R   E   K   P   V   W   V   L   N   P   E -
```

27

```
                                  H             PS        H
                         F  D  M  I  A          ADPA      I
                         0  D  A  N  V          VRUU      N
                         K  E  E  F  A          AAM9      F
                         1  1  3  1  1          2216      1
                                                 ///
      AGGCGGGGATGTGGCAGTGTCTGCTGAGTGACTCGGGACAGGTCCTGCTGGAATCCAACA
1201  ---------+---------+---------+---------+---------+---------+ 1260
      TCCGCCCCTACACCGTCACAGACGACTCACTGAGCCCTGTCCAGGACGACCTTAGGTTGT

       A  G  M  W  Q  C  L  L  S  D  S  G  Q  V  L  L  E  S  N  I -

                         S          SA    BHF BS                H
                        ANA        HNCP   SGNMAANXA          RSD I  A
                        VLU        PCRA   PIUNMULHV          SCD N  L
                        AA9        AIFL   1ADLH3A0A          AAE D  U
                        236        2111   21211A421          111 3  1
                         //         //     / / / /            /
      TCAAGGTTCTGCCCACATGGTCCACCCCGGTGCACGCGGATCCCGAGGGTGAGTACTAAG
1261  ---------+---------+---------+---------+---------+---------+ 1320
      AGTTCCAAGACGGGTGTACCAGGTGGGGCCACGTGCGCCTAGGGCTCCCACTCATGATTC

          K  V  L  P  T  W  S  T  P  V  H  A  D  P  E

                E           BS          SS    F          BS   F
            H   CHH    F    SC          HHNCF ·N          BSC  N
            P   0HA    0    TR          PGCRA  U          BTR  U
            H   4AE    K    NF          AAIFN  4          VNF  4
            1   712    1    11          21111  H          111  H
            /           /   /            //               //
      CTTCAGCGCTCCTGCCTGGACGCATCCCGGCTATGCAGCCCCAGTCCAGGGCAGCAAGGC
1321  ---------+---------+---------+---------+---------+---------+ 1380
      GAAGTCGCGAGGACGGACCTGCGTAGGGCCGATACGTCGGGGTCAGGTCCCGTCGTTCCG

              S                   S
          DBHVHNA             HVNCN        M          MNDM
          RB4BPLU             PNCRL        N          NLDB
          AVE0HA9             ALIFA        L          LAE0
          2132146             21114        1          1312
           // //               //
      AGGCCCCGTCTGCCTCTTCACCCGGAGCCTCTGCCCGCCCCACTCATGCTCAGGGAGAGG
1381  ---------+---------+---------+---------+---------+---------+ 1440
      TCCGGGGCAGACGGAGAAGTGGGCCTCGGAGACGGGCGGGGTGAGTACGAGTCCCTCTCC

               BS   P                          B      BS  S
               SC   F             M  B  N      S      SCDHA
               TR   L             A  A  L      P      TRRAU
               NF   M             E  N  A      1      NFAE9
               11   1             1  1  4      2      11236
                /                                      /  /
      GTCTTCTGGCTTTTTCCCAGGCTCTGGGCAGGCACAGGCTAGGTGCCCCTAACCCAGGCC
1441  ---------+---------+---------+---------+---------+---------+ 1500
      CAGAAGACCGAAAAAGGGTCCGAGACCCGTCCGTGTCCGATCCACGGGGATTGGGTCCGG
```

```
          B                 B             B         S        PS
          S                 DBS           S   M     HNC      ADNPA
          P                 DAP           P   N     PCR      VRLUU
          M                 EN1           M   L     AIF      AAAV9
          1                 122           1   1     211      22416
                              /                      /       ///
          CTGCACACAAAGGGGCAGGTGCTGGGCTCAGACCTGCCAAGAGCCATATCCGGGGAGGACC
1501      --------+---------+---------+---------+---------+---------+  1560
          GACGTGTGTTTCCCCGTCCACGACCCGAGTCTGGACGGTTCTCGGTATAGGCCCTCCTGG


          D                 H             D   A     M
          D                 A             D   L     N
          E                 E             E   U     L
          1                 3             1   1     1
          CTGCCCCTGACCTAAGCCCACCCCAAAGGCCAAACTCTCCACTCCCTCAGCTCGGACACC
1561      --------+---------+---------+---------+---------+---------+  1620
          GACGGGGACTGGATTCGGGTGGGGTTTCCGGTTTGAGAGGTGAGGGAGTCGAGCCTGTGG


          H
          I   M   MM                              P      BS          M
          N   N   AB                              S      AP          A
          F   L   E0                              T      N1          E
          1   1   32                              1      22          3
                   /                                     /
          TTCTCTCCTCCCAGATTCCAGTAACTCCCAATCTTCTCTCTGCAGAGCCCAAATCTTGTG
1621      --------+---------+---------+---------+---------+---------+  1680
          AAGAGAGGAGGGTCTAAGGTCATTGAGGGTTAGAAGAGAGACGTCTCGGGTTTAGAACAC

                                                  E   P   K   S   C   D  -

          N             BBS           BS
          NS            SSC           SC  HS            M A
          LP            PTR           TR  AT            N L
          AH            1NF           NF  EU            L U
          3I            2I1           11  31            1 1
           /             /             /   /
          ACAAAATCACACATGCCCACIGTGCCCAGGTAAGCCAGCCCAGGCCTCGCCCTCCAGCT
1681      --------+---------+---------+---------+---------+---------+  1740
          TGTTTTGAGTGTGTACGGGTGGCACGGGTCCATTCGGTCGGGTCCGGAGCGGGAGGTCGA

          K   T   H   T   C   P   P   C   P

                          B                BS  S        S        S
          M         B N   SM  F            SC  F        DHNA     HNC
          N         A L   PA  D            TR  A        RALU     PCR
          L         N A   1E  K            NF  N        AEA9     AIF
          1         1 4   21  1            11  1        2346     211
                                            /           /        /
          CAAGGCGGGACAGGTGCCCTAGAGTAGCCTGCATCCAGGGACAGGCCCCAGCCGGGTGCT
1741      --------+---------+---------+---------+---------+---------+  1800
          GTTCCGCCCTGTCCACGGGATCTCATCGGACGTAGGTCCCTGTCCGGGGTCGGCCCACGA
```

29

```
                                          BS        S
  A M  M              M   D          M    SC  M  ANA M
  F A  B              N   D          N    TR  B  VLU B
  L E  0              L   E          L    NF  0  AA9 0
  3 2  2              1   1          1    11  2  246 2
                                           /       /
     GACACGTCCACCTCCATCTCTTCCTCAGCACCTGAACTCCTGGGGGGACCGTCAGTCTTC
1801 --------+---------+---------+---------+---------+---------+ 1860
     CTGTGCAGGTGGAGGTAGAGAAGGAGTCGTGGACTTGAGGACCCCCCTGGCAGTCAGAAG

                         A  P  E  L  L  G  G  P  S  V  F  -

                                  S                SS          N
            M         S           AN    M  HMANNAC DM  M       NS
            N         T           UL    N  PNVCLUR DS  A       LP
            L         Y           3A    L  ALAIA9F ET  E       AH
            1         1           A3    1  2121461 12  3       31
                                          /  / //     /           /
     CTCTTCCCCCCAAAACCCAAGGACACCCTCATGATCTCCCGGACCCCTGAGGTCACATGC
1861 --------+---------+---------+---------+---------+---------+ 1920
     GAGAAGGGGGGTTTTGGGTTCCTGTGGGAGTACTAGAGGGCCTGGGGACTCCAGTGTACG

  L  F  P  P  K  P  K  D  T  L  M  I  S  R  T  P  E  V  T  C  -

            M           M        DM    M              RM    M
            A           N        DS    B              SA    N
            E           L        ET    0              AE    L
            2           1        12    2              12    1
                                  /
     GTGGTGGTGGACGTGAGCCACGAAGACCCTGAGGTCAAGTTCAACTGGTACGTGGACGGC
1921 --------+---------+---------+---------+---------+---------+ 1980
     CACCACCACCTGCACTCGGTGCTTCTGGGACTCCAGTTCAAGTTGACCATGCACCTGCCG

  V  V  V  D  V  S  H  E  D  P  E  V  K  F  N  W  Y  V  D  G  -

                             F  FN                           S
                       N     N  NSS             R      M  R HNC
                       N     U  UPA             S      A  S PCR
                       L     4  DBC             A      E  A AIF
                       1     H  222             1      2  1 211
                                  //                         /
     GTGGAGGTGCATAATGCCAAGACAAAGCCGCGGGAGGAGCAGTACAACAGCACGTACCGG
1981 --------+---------+---------+---------+---------+---------+ 2040
     CACCTCCACGTATTACGGTTCTGTTTCGGCGCCCTCCTCGTCATGTTGTCGTGCATGGCC

  V  E  V  H  N  A  K  T  K  P  R  E  E  Q  Y  N  S  T  Y  R  -
```

30

```
                                BS
HH                M             SC                                R
GP                N             TR                                S
AH                L             NF                                A
11                1             11                                1
                                 /
     GTGGTCAGCGTCCTCACCGTCCTGCACCAGGACTGGCTGAATGGCAAGGAGTACAAGTGC
2041 ----------+----------+----------+----------+----------+----------+ 2100
     CACCAGTCGCAGGAGTGGCAGGACGTGGTCCTGACCGACTTACCGTTCCTCATGTTCACG

     V   V   S   V   L   T   V   L   H   Q   D   W   L   N   G   K   E   Y   K   C   -


                                M   T
                                N   A
                                L   Q
                                1   1
     AAGGTCTCCAACAAAGCCCTCCCAGCCCCCATCGAGAAAACCATCTCCAAAGCCAAAGGT
2101 ----------+----------+----------+----------+----------+----------+ 2160
     TTCCAGAGGTTGTTTCGGGAGGGTCGGGGGTAGCTCTTTTGGTAGAGGTTTCGGTTTCCA

     K   V   S   N   K   A   L   P   A   P   I   E   K   T   I   S   K   A   K


          P S                   S                           S
      ADNNPMA               A H M       N           HHN   BSAH           D
      VRLLUNU               U A N       L           APA   GFUA           D
      AAAAML9               9 E L       A           EAE   LI9E           E
      2244116               6 3 1       3           321   1163           1
      //// /                                              /
     GGGACCCGTGGGGTGCGAGGGCCACATGGACAGAGGCCGGCTCGGCCCACCCTCTGCCCT
2161 ----------+----------+----------+----------+----------+----------+ 2220
     CCCTGGGCACCCCACGCTCCCGGTGTACCTGTCTCCGGCCGAGCCGGGTGGGAGACGGGA


                N
M   M           S   R               F
N   A           S   S           M   N   A       B           R   F
L   E           P   A           N   U   V       B           S   0
1   3           B   1           L   4   A       V           A   K
                                1   H   1       1           1   1
     GAGAGTGACCGCTGTACCAACCTCTGTCCTACAGGGCAGCCCCGAGAACCACAGGTGTAC
2221 ----------+----------+----------+----------+----------+----------+ 2280
     CTCTCACTGGCGACATGGTTGGAGACAGGATGTCCCGTCGGGGCTCTTGGTGTCCACATG

                              G   Q   P   R   E   P   Q   V   Y   -
```

31

```
          SS                      BS              BS B
        AHNNCCS    A      F       SC              SC S
        VPCCRRW    L      O       TR              TR P
        AAIIFFA    U      K       NF              NF M
        1211111    1      1       11              11 1
         /////                     /               /
     ACCCTGCCCCCATCCCGGGATGAGCTGACCAAGAACCAGGTCAGCCTGACCTGCCTGGTC
2281 ------------+---------+---------+---------+---------+---------+ 2340
     TGGGACGGGGGTAGGGCCCTACTCGACTGGTTCTTGGTCCAGTCGGACTGGACGGACCAG

      T  L  P  P  S  R  D  E  L  T  K  N  Q  V  S  L  T  C  L  V   -


                                            F
                                            N     H           B
                                            U     P           B
                                            4     A           V
                                            H     2           1
     AAAGGCTTCTATCCCAGCGACATCGCCGTGGAGTGGGAGAGCAATGGGCAGCCGGAGAAC
2341 ------------+---------+---------+---------+---------+---------+ 2400
     TTTCCGAAGATAGGGTCGCTGTAGCGGCACCTCACCCTCTCGTTACCCGTCGGCCTCTTG

      K  G  F  Y  P  S  D  I  A  V  E  W  E  S  N  G  Q  P  E  N   -


                               H
                      M  I   M          N              H       M A
                      N  N   B          L              P       N L
                      L  F   O          A              H       L U
                      1  1   2          4              1       1 1
     AACTACAAGACCACGCCTCCCGTGCTGGACTCCGACGGCTCCTTCTTCCTCTACAGCAAG
2401 ------------+---------+---------+---------+---------+---------+ 2460
     TTGATGTTCTGGTGCGGAGGGCACGACCTGAGGCTGCCGAGGAAGAAGGAGATGTCGTTC

      N  Y  K  T  T  P  P  V  L  D  S  D  G  S  F  F  L  Y  S  K   -


                      B          F                 S
                      S          NM       MBX       NF    M       N
                      P          UB       ABM       LA    N       S
                      M          40       EVN       AN    L       I
                      1          H2       211       31    1       1
                                                     /
     CTCACCGTGGACAAGAGCAGGTGGCAGCAGGGGAACGTCTTCTCATGCTCCGTGATGCAT
2461 ------------+---------+---------+---------+---------+---------+ 2520
     GAGTGGCACCTGTTCTCGTCCACCGTCGTCCCCTTGCAGAAGAGTACGAGGCACTACGTA

      L  T  V  D  K  S  R  W  Q  Q  G  N  V  F  S  C  S  V  M  H   -
```

```
                                              S
       N                             M   M   HNC
       L                             B   N   PCR
       A                             0   L   AIF
       3                             2   1   211
                                                /
       GAGGCTCTGCACAACCACTACACGCAGAAGAGCCTCTCCCTGTCTCCGGGTAAATGAGTG
2521   ----------+---------+---------+---------+---------+---------+ 2580
       CTCCGAGACGTGTTGGTGATGTGCGTCTTCTCGGAGAGGGACAGAGGCCCATTTACTCAC

       E  A  L  H  N  H  Y  T  Q  K  S  L  S  L  S  P  G  K  *

         CXH
         FMA
         RAE
         133
          /
       CGACGGCCG
2581   --------- 2589
       GCTGCCGGC
```

## Table 3

```
                 F N                            S              B
                 N S          B    M    H      DHA             S
                 U P          B    N    G      RAU             T
                 4 B          V    L    A      AE9             X
                 H 2          1    1    1      236             1
                                                /
     GCCTGTTTGAGAAGCAGCGGGCAAGAAAGACGCAAGCCCAGAGGCCCTGCCATTTCTGTG
   1 ---------+---------+---------+---------+---------+---------+ 60
     CGGACAAACTCTTCGTCGCCCGTTCTTTCTGCGTTCGGGTCTCCGGGACGGTAAAGACAC


            B     PS                S                          S
          DBS ADNPA      D    DHNA              M    HM       HNC
          DAP VRLUU      D    RALU              N    AN       PCR
          EN1 AAAM9      E    AEA9              L    EL       AIF
          122 22416      1    2346              1    31       211
            /   / //          /                     /          /
     GGCTCAGGTCCCTACTGGCTCAGGCCCCTGCCTCCCTCGGCAAGGCCACAATGAACCGGG
  61 ---------+---------+---------+---------+---------+---------+ 120
     CCGAGTCCAGGGATGACCGAGTCCGGGGACGGAGGGAGCCGTTCCGGTGTTACTTGGCCC

                                                  M  N  R  G  -

          H                      F              F
          I            B         N         HH   N M      D
          N            B         U         HA   U N      D
          F            V         4         AE   4 L      E
          1            1         H         12   H 1      1
     GAGTCCCTTTTAGGCACTTGCTTCTGGTGCTGCAACTGGCGCTCCTCCCAGCAGCCACTC
 121 ---------+---------+---------+---------+---------+---------+ 180
     CTCAGGGAAAATCCGTGAACGAAGACCACGACGTTGACCGCGAGGAGGGTCGTCGGTGAG

        V  P  F  R  H  L  L  L  V  L  Q  L  A  L  L  P  A  A  T  Q  -

          B     E  E                              R   A
          B     C  C                              S   L
          V     0  0                              A   U
          1     K  K                              1   1
     AGGGAAAGAAAGTGGTGCTGGGCAAAAAAGGGGATACAGTGGAACTGACCTGTACAGCTT
 181 ---------+---------+---------+---------+---------+---------+ 240
     TCCCTTTCTTTCACCACGACCCGTTTTTTCCCCTATGTCACCTTGACTGGACATGTCGAA

        G  K  K  V  V  L  G  K  K  G  D  T  V  E  L  T  C  T  A  S  -
```

```
                      M   M                                         H
                      B   B                                         I
                      O   O                                         N
                      2   2                                         F
                                                                    1
      CCCAGAAGAAGAGCATACAATTCCACTGGAAAAACTCCAACCAGATAAAGATTCTGGGAA
241   ------------+-----------+-----------+-----------+-----------+-----------+   300
      GGGTCTTCTTCTCGTATGTTAAGGTGACCTTTTTGAGGTTGGTCTATTTCTAAGACCCTT

        Q   K   K   S   I   Q   F   H   W   K   N   S   N   Q   I   K   I   L   G   N -

                      B                   S                   S   F   H
                    NBS         F       AA          A       A   N   H   I
                    LAP         O       VU          L       U   U   H   N
                    AN1         K       A9          U       3   D   A   F
                    422         1       26          1       A   2   1   1
                                /                   /
      ATCAGGGCTCCTTCTTAACTAAAGGTCCATCCAAGCTGAATGATCGCGCTGACTCAAGAA
301   ------------+-----------+-----------+-----------+-----------+-----------+   360
      TAGTCCCGAGGAAGAATTGATTTCCAGGTAGGTTCGACTTACTAGCGCGACTGAGTTCTT

        Q   G   S   F   L   T   K   G   P   S   K   L   N   D   R   A   D   S   R   R -

                              S                   S       H               H
                          MANAS                 BA        I       A       I   D
                          BVLUT                 CU        N       F       N   D
                          OAA9Y                 L3        F       L       F   E
                          22461                 1A        1       2       1   1
                              /                   /
      GAAGCCTTTGGGACCAAGGAAACTTCCCCCTGATCATCAAGAATCTTAAGATAGAAGACT
361   ------------+-----------+-----------+-----------+-----------+-----------+   420
      CTTCGGAAACCCTGGTTCCTTTGAAGGGGGACTAGTAGTTCTTAGAATTCTATCTTCTGA

        S   L   W   D   Q   G   N   F   P   L   I   I   K   N   L   K   I   E   D   S -

                                              S
                      M           M       AMAM                       M
                      B           N       VNUN                       A
                      O           L       AL9L                       E
                      2           1       2161                       1
                                              //
      CAGATACTTACATCTGTGAAGTGGAGGACCAGAAGGAGGAGGTGCAATTGCTAGTGTTCG
421   ------------+-----------+-----------+-----------+-----------+-----------+   480
      GTCTATGAATGTAGACACTTCACCTCCTGGTCTTCCTCCTCCACGTTAACGATCACAAGC

        D   T   Y   I   C   E   V   E   D   Q   K   E   E   V   Q   L   L   V   F   G -
```

```
                                          B                              S
                                          S                              T
                                          P                              Y
                                          M                              1
                                          1
        GATTGACTGCCAACTCTGACACCCACCTGCTTCAGGGGCAGAGCCTGACCCTGACCTTGG
    481 ---------+---------+---------+---------+---------+---------+ 540
        CTAACTGACGGTTGAGACTGTGGGTGGACGAAGTCCCCGTCTCGGACTGGGACTGGAACC

         L  T  A  N  S  D  T  H  L  L  Q  G  Q  S  L  T  L  T  L  E -

              B  BS                              H
              BS SC           D            N     I     S
              AP TR           D            N     N     T
              N1 NF           E            L     F     Y
              22 11           1            1     1     1
              /  /
        AGAGCCCCCCTGGTAGTAGCCCCTCAGTGCAATGTAGGAGTCCAAGGGGGTAAAAACATAC
    541 ---------+---------+---------+---------+---------+---------+ 600
        TCTCGGGGGGACCATCATCGGGGAGTCACGTTACATCCTCAGGTTCCCCATTTTTGTATG

         S  P  P  G  S  S  P  S  V  Q  C  R  S  P  R  G  K  N  I  Q -

                               N        BBH S  B           BS
                    M    MD     ASP   A  BSSGSC S     B  N  SC
                    B    ND     LPV   L  APTIAR T     A  L  TR
                    0    LE     UBU   U  N1NACF X     N  A  NF
                    2    11     122   1  221111 1     1  4  11
                               //        / ///              /
        AGGGGGGGGAAGACCCTCTCCGTGTCTCAGCTGGAGCTCCAGGATAGTGGCACCTGGACAT
    601 ---------+---------+---------+---------+---------+---------+ 660
        TCCCCCCCCTTCTGGGAGAGGCACAGAGTCGACCTCGAGGTCCTATCACCGTGGACCTGTA

         G  G  K  T  L  S  V  S  Q  L  E  L  Q  D  S  G  T  W  T  C -

            N
            NS                      M                 NM  A
            LP                      B                 HA  L
            AH                      0                 EE  U
            31                      2                 11  1
            /
        GCACTGTCTTGCAGAACCAGAAGAAGGTGGAGTTCAAAATAGACATCGTGGTGCTAGCTT
    661 ---------+---------+---------+---------+---------+---------+ 720
        CGTGACAGAACGTCTTGGTCTTCTTCCACCTCAAGTTTTATCTGTAGCACCACGATCGAA

         T  V  L  Q  N  Q  K  K  V  E  F  K  I  D  I  V  V  L  A  F -
```

```
            HS          M   M
            AT          N   N
            EU          L   L
            31          1   1
             /
       TCCAGAAGGCCTCCAGCATAGTCTATAAGAAAGAGGGGGAACAGGTGGAGTTCTCCTTCC
  721  ---------+---------+---------+---------+---------+---------+  780
       AGGTCTTCCGGAGGTCGTATCAGATATTCTTTCTCCCCCTTGTCCACCTCAAGAGGAAGG

         Q  K  A  S  S  I  V  Y  K  K  E  G  E  Q  V  E  F  S  F  P -

                        A                 A           M
                        L                 L           N
                        U                 U           L
                        1                 1           1
       CACTCGCCTTTACAGTTGAAAAGCTGACGGGCAGTGGCGAGCTGTGGTGGCAGGCGGAGA
  781  ---------+---------+---------+---------+---------+---------+  840
       GTGAGCGGAAATGTCAACTTTTCGACTGCCCGTCACCGCTCGACACCACCGTCCGCCTCT

         L  A  F  T  V  E  K  L  T  G  S  G  E  L  W  W  Q  A  E  R -

                         P  S
            H    M  FW  A                                M
            P    N  LN  U                                B
            H    L  ML  3                                0
            1    1  11  A                                2
       GGGCTTCCTCCTCCAAGTCTTGGATCACCTTTGACCTGAAGAACAAGGAAGTGTCTGTAA
  841  ---------+---------+---------+---------+---------+---------+  900
       CCCGAAGGAGGAGGTTCAGAACCTAGTGGAAACTGGACTTCTTGTTCCTTCACAGACATT

         A  S  S  S  K  S  W  I  T  F  D  L  K  N  K  E  V  S  V  K -

            B        BS   PS
            SM       SCADNPAD   A            A  H
            TA       TRVRLUUD   L            L  P
            EE       NFAAAM9E   U            U  H
            23       11224161   1            1  1
             /         / / //
       AACGGGTTACCCAGGACCCTAAGCTCCAGATGGGCAAGAAGCTCCCGCTCCACCTCACCC
  901  ---------+---------+---------+---------+---------+---------+  960
       TTGCCCAATGGGTCCTGGGATTCGAGGTCTACCCGTTCTTCGAGGGCGAGGTGGAGTGGG

         R  V  T  Q  D  P  K  L  Q  M  G  K  K  L  P  L  H  L  T  L -

               BS                             BSS
            M  SC HS        D        M  H     SCAHM
            N  TR AT        D        N  P     TRUAN
            L  NF EU        E        L  H     NF9EL
            1  11 31        1        1  1     11631
                / /                            / /
       TGCCCCAGGCCTTGCCTCAGTATGCTGGCTCTGGAAACCTCACCCTGGCCCTTGAAGCGA
  961  ---------+---------+---------+---------+---------+---------+  1020
       ACGGGGTCCGGAACGGAGTCATACGACCGAGACCTTTGGAGTGGGACCGGGAACTTCGCT

         P  Q  A  L  P  Q  Y  A  G  S  G  N  L  T  L  A  L  E  A  K -
```

37

```
                      S         BS
                      F         SC                   H D   A
                      A         TR                   P D   L
                      N         NF                   H E   U
                      1         11                   1 1   1
                                /
AAACAGGAAAGTTGCATCAGGAAGTGAACCTGGTGGTGATGAGAGCCACTCAGCTCCAGA
1021 --------+---------+---------+---------+---------+---------+ 1080
TTTGTCCTTTCAACGTAGTCCTTCACTTGGACCACCACTACTCTCGGTGAGTCGAGGTCT

     T  G  K  L  H  Q  E  V  N  L  V  V  M  R  A  T  Q  L  Q  K -

                                PS            S
           M                    ADNNPA        DF  AN      DE   A
           N                    VRLLUU        DA  LN      DS   L
           L                    AAAAN9        EN  UL      EP   U
           1                    224416        11  11      11   1
                                //////        /   /       /
AAAATTTGACCTGTGAGGTGTGGGGACCCACCTCCCCTAAGCTGATGCTGAGCTTGAAAC
1081 --------+---------+---------+---------+---------+---------+ 1140
TTTTAAACTGGACACTCCACACCCCTGGGTGGAGGGGATTCGACTACGACTCGAACTTTG

     N  L  T  C  E  V  W  G  P  T  S  P  K  L  M  L  S  L  K  L -

           M                    T             H           M    DM
           N                    A             P           N    DS
           L                    Q             A           L    ET
           1                    1             2           1    12
                                                                /
TGGAGAACAAGGAGGCAAAGGTCTCGAAGCGGGAGAAGCCGGTGTGGGTGCTGAACCCTG
1141 --------+---------+---------+---------+---------+---------+ 1200
ACCTCTTGTTCCTCCGTTTCCAGAGCTTCGCCCTCTTCGGCCACACCCACGACTTGGGAC

     E  N  K  E  A  K  V  S  K  R  E  K  P  V  W  V  L  N  P  E -

                             H                   PS        H
                    F   D  M  I  A                ADPA      I
                    O   D  A  N  V                VRUU      N
                    K   E  E  F  A                AAN9      F
                    1   1  3  1  1                2216      1
                                                  ///
AGGCGGGGATGTGGCAGTGTCTGCTGAGTGACTCGGGACAGGTCCTGCTGGAATCCAACA
1201 --------+---------+---------+---------+---------+---------+ 1260
TCCGCCCCTACACCGTCACAGACGACTCACTGAGCCCTGTCCAGGACGACCTTAGGTTGT

     A  G  M  W  Q  C  L  L  S  D  S  G  Q  V  L  L  E  S  N  I -
```

```
                 S              SA    BHF BS                              H
               ANA            HNCP  SGNMAANXA                         RSD I A
               VLU            PCRA  PIUNMULHV                         SCD N L
               AA9            AIFL  1ADLH3ADA                         AAE D U
               236           2111  21211A421                         111 3 1
                //             //    / / / /                           /
      TCAAGGTTCTGCCCACATGGTCCACCCCGGTGCACGCGGATCCCGAGGGTGAGTACTAAG
1261  ---------+---------+---------+---------+---------+---------+ 1320
      AGTTCCAAGACGGGTGTACCAGGTGGGGCCACGTGCGCCTAGGGCTCCCACTCATGATTC

         K  V  L  P  T  W  S  T  P  V  H  A  D  P  E

                  E           BS              SS    F              BS   F
        H       CHH  F        SC            HHNCF   N            BSC   N
        P       OHA  D        TR            PGCRA   U            BTR   U
        H       4AE  K        NF            AAIFN   4            VNF   4
        1       712  1        11            21111   H            111   H
        /             /                      //                   //
      CTTCAGCGCTCCTGCCTGGACGCATCCCGGCTATGCAGCCCCAGTCCAGGGCAGCAAGGC
1321  ---------+---------+---------+---------+---------+---------+ 1380
      GAAGTCGCGAGGACGGACCTGCGTAGGGCCGATACGTCGGGGTCAGGTCCCGTCGTTCCG

                 S                    S
        DBHMHNA              HMNCN           M         MNDM
        RBABPLU              PNCR_           N         N_DB
        AVEDHA9              ALIFA           L         LAED
        2132146              21114           1         1312
          // //               //
      AGGCCCCGTCTGCCTCTTCACCCGGAGCCTCTGCCCGCCCCACTCATGCTCAGGGAGAGG
1381  ---------+---------+---------+---------+---------+---------+ 1440
      TCCGGGGCAGACGGAGAAGTGGGCCTCGGAGACGGGCGGGGTGAGTACGAGTCCCTCTCC

                 BS   P                    M  B  N   B        BS  S
                 SC   F                    A  A  L   S        SCDHA
                 TR   L                    E  L  A   P        TRRAU
                 NF   M                    1  N  A   1        NFAE9
                 11   1                       1  4   2        11236
                  /                                            / /
      GTCTTCTGGCTTTTTCCCAGGCTCTGGGCAGGCACAGGCTAGGTGCCCCTAACCCAGGCC
1441  ---------+---------+---------+---------+---------+---------+ 1500
      CAGAAGACCGAAAAAGGGTCCGAGACCCGTCCGTGTCCGATCCACGGGGATTGGGTCCGG

                 B                    B         B        S      PS
                 S                   DBS        S  M    HNC   ADNPA
                 P                   DAP        P  N    PCR   VRLUU
                 M                   EN1        M  L    AIF   AAAM9
                 1                   122        1  1    211   22416
                                      /                  /    / //
      CTGCACACAAAGGGGCAGGTGCTGGGCTCAGACCTGCCAAGAGCCATATCCGGGAGGACC
1501  ---------+---------+---------+---------+---------+---------+ 1560
      GACGTGTGTTTCCCCGTCCACGACCCGAGTCTGGACGGTTCTCGGTATAGGCCCTCCTGG
```

```
                    D                   H                   D   A   M
                    D                   A                   D   L   N
                    E                   E                   E   U   L
                    1                   3                   1   1   1
      CTGCCCCTGACCTAAGCCCACCCCAAAGGCCAAACTCTCCACTCCCTCAGCTCGGACACC
 1561 ---------+---------+---------+---------+---------+---------+ 1620
      GACGGGGACTGGATTCGGGTGGGGGTTTCCGGTTTGAGAGGTGAGGGAGTCGAGCCTGTGG

       L  P  L  T  *  A  H  P  K  G  Q  T  L  H  S  L  S  S  D  T  -
          C  P  *  P  K  P  T  P  K  A  K  L  S  T  P  S  A  R  T  P  -
             A  P  D  L  S  P  P  Q  R  P  N  S  P  L  P  Q  L  G  H  L  -

                    H                                   S
                    I   M  MM                  DF       F
                    N   N  AB                  DO       A
                    F   L  EO                  EK       N
                    1   1  32                  11       1
                             /                  /
      TTCTCTCCTCCCAGATTCCAGTAACTCCCAATCTTCTCTCTCAGGGAGTGCATCCGCCCC
 1621 ---------+---------+---------+---------+---------+---------+ 1680
      AAGAGAGGAGGGTCTAAGGTCATTGAGGGTTAGAAGAGAGAGTCCCTCACGTAGGCGGGG

                                                        G  S  A  S  A  P  -

                              E
                          N   C
                          N   O
                          L   R
                          1   1
      AACCCTTTTTCCCCCTCGTCTCCTGTGAGAATTCC....
 1681 ---------+---------+---------+---- 1714
      TTGGGAAAAGGGGGAGCAGAGGACACTCTTAAGG....

        T  L  F  P  L  V  S  C  E  N  S  ....
```

40

## Table 4

```
            F N                                 S              B
            N S            B        M       H   DHA            S
            U P            B        N       G   RAU            T
            4 B            V        L       A   AE9            X
            H 2            1        1       1   236            1
                                                 /
     GCCTGTTTGAGAAGCAGCGGGCAAGAAAGACGCAAGCCCAGAGGCCCTGCCATTTCTGTG
   1 --------+---------+---------+---------+---------+---------+ 60
     CGGACAAACTCTTCGTCGCCCGTTCTTTCTGCGTTCGGGTCTCCGGGACGGTAAAGACAC


        B    PS            S                     M   HM           S
     DBS ADNPA          D  DHNA                  N   AN          HNC
     DAP VRLUU          D  RALU                  L   EL          PCR
     EN1 AAAM9          E  AEA9                   1   31          AIF
     122 22416          1  2346                       1          211
       /  / //             /                             /          /
     GGCTCAGGTCCCTACTGGCTCAGGCCCCTGCCTCCCTCGGCAAGGCCACAATGAACCGGG
  61 --------+---------+---------+---------+---------+---------+ 120
     CCGAGTCCAGGGATGACCGAGTCCGGGGACGGAGGGAGCCGTTCCGGTGTTACTTGGCCC

                                                         M  N  R  G -

            H                           F               F
            I               B           N       HH      N  M    D
            N               B           U       HA      U  N    D
            F               V           4       AE      4  L    E
            1               1           H       12      H  1    1
     GAGTCCCTTTTAGGCACTTGCTTCTGGTGCTGCAACTGGCGCTCCTCCCAGCAGCCACTC
 121 --------+---------+---------+---------+---------+---------+ 180
     CTCAGGGAAAATCCGTGAACGAAGACCACGACGTTGACCGCGAGGAGGGTCGTCGGTGAG

        V  P  F  R  H  L  L  L  V  L  Q  L  A  L  L  P  A  A  T  Q -

            B       E  E                                    R    A
            B       C  C                                    S    L
            V       D  D                                    A    U
            1       K  K                                    1    1
     AGGGAAAGAAAGTGGTGCTGGGCAAAAAAGGGGATACAGTGGAACTGACCTGTACAGCTT
 181 --------+---------+---------+---------+---------+---------+ 240
     TCCCTTTCTTTCACCACGACCCGTTTTTTCCCCTATGTCACCTTGACTGGACATGTCGAA

        G  K  K  V  V  L  G  K  K  G  D  T  V  E  L  T  C  T  A  S -
```

41

```
                                                               H
                 M  M                                          I
                 B  B                                          N
                 0  0                                          F
                 2  2                                          1
      CCCAGAAGAAGAGCATACAATTCCACTGGAAAAAACTCCAACCA3ATAAAGATTCTGGGAA
241   --------+---------+---------+---------+---------+---------+   300
      GGGTCTTCTTCTCGTATGTTAAGGTGACCTTTTTGAGGTTGGTCTATTTCTAAGACCCTT

       Q  K  K  S  I  Q  F  H  W  K  N  S  N  Q  I  K  I  L  G  N -

                    B                 S                 S  F  H
                 NBS         F        AA             A  A  N  H  I
                 LAP         0        VU             L  U  U  H  N
                 AN1         K        A9             U  3  D  A  F
                 422         1        26             1  A  2  1  1
                  /                    /
      ATCAGGGCTCCTTCTTAACTAAAGGTCCATCCAAGCTGAATGATCGCGCTGACTCAAGAA
301   --------+---------+---------+---------+---------+---------+   360
      TAGTCCCGAGGAAGAATTGATTTCCAGGTAGGTTCGACTTACTAGCGCGACTGAGTTCTT

       Q  G  S  F  L  T  K  G  P  S  K  L  N  D  R  A  D  S  R  R -

                    S                    S        H              H
                 MANAS                   BA       I     A        I  D
                 BVLUT                   CU       N     F        N  D
                 0AA9Y                   L3       F     L        F  E
                 22461                   1A       1     2        1  1
                   /                      /
      GAAGCCTTTGGGACCAAGGAAACTTCCCCCTGATCATCAAGAATCTTAAGATAGAAGACT
361   --------+---------+---------+---------+---------+---------+   420
      CTTCGGAAACCCTGGTTCCTTTGAAGGGGGACTAGTAGTTCTTAGAATTCTATCTTCTGA

       S  L  W  D  Q  G  N  F  P  L  I  I  K  N  L  K  I  E  D  S -

                                    S
                 M        M          AMAM                 M
                 B        N          VNUN                 A
                 0        L          AL9L                 E
                 2        1          2161                 1
                                      //
      CAGATACTTACATCTGTGAAGTGGAGGACCAGAAGGAGGAGGTGCAATTGCTAGTGTTCG
421   --------+---------+---------+---------+---------+---------+   480
      GTCTATGAATGTAGACACTTCACCTCCTGGTCTTCCTCCTCCACGTTAACGATCACAAGC

       D  T  Y  I  C  E  V  E  D  Q  K  E  E  V  Q  L  L  V  F  G -
```

```
                                              B                              S
                                              S                              T
                                              P                              Y
                                              M                              1
                                              1
      GATTGACTGCCAACTCTGACACCCACCTGCTTCAGGGGCAGAGCCTGACCCTGACCTTGG
  481 ----------+---------+---------+---------+---------+---------+ 540
      CTAACTGACGGTTGAGACTGTGGGTGGACGAAGTCCCCGTCTCGGACTGGGACTGGAACC
       L  T  A  N  S  D  T  H  L  L  Q  G  Q  S  L  T  L  T  L  E -

             B  BS
             BS SC                 D             M        H
             AP TR                 D             N        I  S
             N1 NF                 E             L        N  T
             22 11                 1             1        F  Y
                                                          1  1
             /  /
      AGAGCCCCCCTGGTAGTAGCCCCTCAGTGCAATGTAGGAGTCCAAGGGGTAAAAACATAC
  541 ----------+---------+---------+---------+---------+---------+ 600
      TCTCGGGGGGACCATCATCGGGGAGTCACGTTACATCCTCAGGTTCCCCATTTTTGTATG
          S  P  P  G  S  S  P  S  V  Q  C  R  S  P  R  G  K  N  I  Q -

                              N           BBH S  B           BS
                      M   MD  ASP     A BSSGSC  S  B  N  SC
                      B   ND  LPV     L APTIAR  T  A  L  TR
                      0   LE  UBU     U N1NACF  X  N  A  NF
                      2   11  122     1 221111  1  1  4  11
                              //        / ///                 /
      AGGGGGGGGAAGACCCTCTCCGTGTCTCAGCTGGAGCTCCAGGATAGTGGCACCTGGACAT
  601 ----------+---------+---------+---------+---------+---------+ 660
      TCCCCCCCTTCTGGGAGAGGCACAGAGTCGACCTCGAGGTCCTATCACCGTGGACCTGTA
          G  G  K  T  L  S  V  S  Q  L  E  L  Q  D  S  G  T  W  T  C -

          N
          NS                              M                    NM  A
          LP                              B                    HA  L
          AH                              0                    EE  U
          31                              2                    11  1
          /
      GCACTGTCTTGCAGAACCAGAAGAAGGTGGAGTTCAAAATAGACATCGTGGTGCTAGCTT
  661 ----------+---------+---------+---------+---------+---------+ 720
      CGTGACAGAACGTCTTGGTCTTCTTCCACCTCAAGTTTTATCTGTAGCACCACGATCGAA
          T  V  L  Q  N  Q  K  K  V  E  F  K  I  D  I  V  V  L  A  F -

             HS           M  M
             AT           N  N
             EU           L  L
             31           1  1
             /
      TCCAGAAGGCCTCCAGCATAGTCTATAAGAAAGAGGGGGAACAGGTGGAGTTCTCCTTCC
  721 ----------+---------+---------+---------+---------+---------+ 780
      AGGTCTTCCGGAGGTCGTATCAGATATTCTTTCTCCCCCTTGTCCACCTCAAGAGGAAGG

          Q  K  A  S  S  I  V  Y  K  K  E  G  E  Q  V  E  F  S  F  P -
```

43

```
                         A               A       M
                         L               L       N
                         U               U       L
                         1               1       1
   CACTCGCCTTTACAGTTGAAAAGCTGACGGGCAGTGGCGAGCTGTGGTGGCAGGCGGAGA
781 ----------+---------+---------+---------+---------+---------+ 840
   GTGAGCGGAAATGTCAACTTTTCGACTGCCCGTCACCGCTCGACACCACCGTCCGCCTCT
     L  A  F  T  V  E  K  L  T  G  S  G  E  L  W  W  Q  A  E  R  -


                       P  S
              H   M  FM A                                M
              P   N  LN U                                B
              H   L  ML 3                                0
              1   1 11  A                                2
   GGGCTTCCTCCTCCAAGTCTTGGATCACCTTTGACCTGAAGAACAAGGAAGTGTCTGTAA
841 ----------+---------+---------+---------+---------+---------+ 900
   CCCGAAGGAGGAGGTTCAGAACCTAGTGGAAACTGGACTTCTTGTTCCTTCACAGACATT
     A  S  S  S  K  S  W  I  T  F  D  L  K  N  K  E  V  S  V  K  -


              B        BS   PS
              SM       SCADNPAD   A              A  H
              TA       TRVRLUUD   L              L  P
              EE       NFAAAM9E   U              U  H
              23       11224161   1              1  1
                /        / / //
   AACGGGTTACCCAGGACCCTAAGCTCCAGATGGGCAAGAAGCTCCCGCTCCACCTCACCC
901 ----------+---------+---------+---------+---------+---------+ 960
   TTGCCCAATGGGTCCTGGGATTCGAGGTCTACCCGTTCTTCGAGGGCGAGGTGGAGTGGG
     R  V  T  Q  D  P  K  L  Q  M  G  K  K  L  P  L  H  L  T  L  -


                 BS                             BSS
              M  SC HS    D         M  H        SCAHM
              N  TR AT    D         N  P        TRUAN
              L  NF EU    E         L  H        NF9EL
              1  11 31    1         1  1        11631
                    /  /                          /   /
   TGCCCCAGGCCTTGCCTCAGTATGCTGGCTCTGGAAACCTCACCCTGGCCCTTGAAGCGA
961 ----------+---------+---------+---------+---------+---------+ 1020
   ACGGGGTCCGGAACGGAGTCATACGACCGAGACCTTTGGAGTGGGACCGGGAACTTCGCT
     P  Q  A  L  P  Q  Y  A  G  S  G  N  L  T  L  A  L  E  A  K  -


                    S          BS
                    F          SC            H  D     A
                    A          TR            P  D     L
                    N          NF            H  E     U
                    1          11            1  1     1
                                /
   AAACAGGAAAGTTGCATCAGGAAGTGAACCTGGTGGTGATGAGAGCCACTCAGCTCCAGA
1021 ----------+---------+---------+---------+---------+---------+ 1080
   TTTGTCCTTTCAACGTAGTCCTTCACTTGGACCACCACTACTCTCGGTGAGTCGAGGTCT

       T  G  K  L  H  Q  E  V  N  L  V  V  M  R  A  T  Q  L  Q  K  -
```

44

```
                              PS        S
             M                ADNNPA     DF  AM      DE  A
             N                VRLLUU     DA  LN      DS  L
             L                AAAAM9     EN  UL      EP  U
             1                224416     11  11      11  1
                              /////      /   /       /
        AAAATTTGACCTGTGAGGTGTGGGGGACCCACCTCCCCTAAGCTGATGCTGAGCTTGAAAC
   1081 ---------+---------+---------+---------+---------+---------+ 1140
        TTTTAAACTGGACACTCCACACCCCTGGGTGGAGGGGGATTCGACTACGACTCGAACTTTG
          N  L  T  C  E  V  W  G  P  T  S  P  K  L  M  L  S  L  K  L -


             M                        T              H           M        DM
             N                        A              P           N        DS
             L                        Q              A           L        ET
             1                        1              2           1        12
                                                                          /
        TGGAGAACAAGGAGGCAAAGGTCTCGAAGCGGGAGAAGCCGGTGTGGGTGCTGAACCCTG
   1141 ---------+---------+---------+---------+---------+---------+ 1200
        ACCTCTTGTTCCTCCGTTTCCAGAGCTTCGCCCTCTTCGGCCACACCCACGACTTGGGAC
          E  N  K  E  A  K  V  S  K  R  E  K  P  V  W  V  L  N  P  E -


                                   H                 PS        H
                    F  D  M  I  A   ADPA      I
                    0  D  A  N  V   VRUU      N
                    K  E  E  F  A   AAM9      F
                    1  1  3  1  1   2216      1
                                   ///
        AGGCGGGGATGTGGCAGTGTCTGCTGAGTGACTCGGGACAGGTCCTGCTGGAATCCAACA
   1201 ---------+---------+---------+---------+---------+---------+ 1260
        TCCGCCCCTACACCGTCACAGACGACTCACTGAGCCCTGTCCAGGACGACCTTAGGTTGT
          A  G  M  W  Q  C  L  L  S  D  S  G  Q  V  L  L  E  S  N  I -


                       S          SA      BHF BS                  H
                    ANA           HNCP    SGNVAANXA           RSD I A
                    VLU           PCRA    PIUNMULHV           SCD N L
                    AA9           AIFL    1ADLH3ADA           AAE D U
                    236           2111    21211A421           111 3 1
                    //            //      / / / /              /
        TCAAGGTTCTGCCCACATGGTCCACCCCCGGTGCACGCGGATCCCGAGGGTGAGTACTAAG
   1261 ---------+---------+---------+---------+---------+---------+ 1320
        AGTTCCAAGACGGGTGTACCAGGTGGGGCCACGTGCGCCTAGGGCTCCCACTCATGATTC
          K  V  L  P  T  W  S  T  P  V  H  A  D  P  E


                       E          BS              SS        F           BS  F
                    H  CHH      F  SC             HHNCF      N           BSC N
                    P  OHA      0  TR             PGCRA      U           BTR U
                    H  4AE      K  NF             AAIFN      4           VNF 4
                    1  712      1  11             21111      H           111 H
                    /                /            //                     //
        CTTCAGCGCTCCTGCCTGGACGCATCCCGGCTATGCAGCCCCAGTCCAGGGCAGCAAGGC
   1321 ---------+---------+---------+---------+---------+---------+ 1330
        GAAGTCGCGAGGACGGACCTGCGTAGGGCCGATACGTCGGGGTCAGGTCCCGTCGTTCCG
```

45

```
                S                       S
        DBHI/HNA                HMNCN           V           MNDV
        RBABPLU                 PNCRL           N           NLD5
        AVEDHA9                 ALIFA           L           LAED
        2132146                 21114           1           1312
         // //                   //
        AGGCCCCGTCTGCCTCTTCACCCGGAGCCTCTGCCCGCCCCACTCATGCTCAGGGAGAGG
1381    ----------+---------+---------+---------+---------+---------+ 1440
        TCCGGGGCAGACGGAGAAGTGGGCCTCGGAGACGGGCGGGGTGAGTACGAGTCCCTCTCC


                BS      P                       M   B   N       B       BS   S
                SC      F                       A   A   L       S       SCDHA
                TR      L                       E   N   A       P       TRRAU
                NF      M                       1   1   4       1       NFAE9
                11      1                                       2       11236
                 /                                                       /   /
        GTCTTCTGGCTTTTTCCCAGGCTCTGGGCAGGCACAGGCTAGGTGCCCCTAACCCAGGCC
1441    ----------+---------+---------+---------+---------+---------+ 1500
        CAGAAGACCGAAAAAGGGTCCGAGACCCGTCCGTGTCCGATCCACGGGGATTGGGTCCGG


                B                       B               B               S           PS
                S                       DBS             S   M           HNC         ADNPA
                P                       DAP             P   N           PCR         VRLUU
                M                       EN1             M   L           AIF         AAAV9
                1                       122             1   1           211         22416
                                         /                               /           / //
        CTGCACACAAAGGGGCAGGTGCTGGGCTCAGACCTGCCAAGAGCCATATCCGGGAGGACC
1501    ----------+---------+---------+---------+---------+---------+ 1560
        GACGTGTGTTTCCCCGTCCACGACCCGAGTCTGGACGGTTCTCGGTATAGGCCCTCCTGG


                D                       H               D       A       M
                D                       A               D       L       N
                E                       E               E       U       L
                1                       3               1       1       1
        CTGCCCCTGACCTAAGCCCACCCCAAAGGCCAAACTCTCCACTCCCTCAGCTCGGACACC
1561    ----------+---------+---------+---------+---------+---------+ 1620
        GACGGGGACTGGATTCGGGTGGGGTTTCCGGTTTGAGAGGTGAGGGAGTCGAGCCTGTGG


                H                                               F
                I   M   MM                      BP      DE  AN
                N   N   AB                      BS      DS  LU
                F   L   ED                      VT      EP  U4
                1   1   32                      11      11  1H
                         /                               /       /
        TTCTCTCCTCCCAGATTCCAGTAACTCCCAATCTTCTCTCTGCAGTGATTGCTGAGCTGC
1621    ----------+---------+---------+---------+---------+---------+ 1680
        AAGAGAGGAGGGTCTAAGGTCATTGAGGGTTAGAAGAGAGACGTCACTAACGACTCGACG


                                                V   I   A   E   L   P   -
```

46

```
                              F
  M H   M            M        N
  B G   N            B        U
  O A   L            O        D
  2 1   1            2        2
      CTCCCAAAGTGAGCGTCTTCGTCCCACCCCGCGACGGCTTCTTCGGCAACCCCCGCAAGT
1681  ---------+---------+---------+---------+---------+---------+  1740
      GAGGGTTTCACTCGCAGAAGCAGGGTGGGGCGCTGCCGAAGAAGCCGTTGGGGGCGTTCA

       P K V S V F V P P R D G F F G N P R K S -


              BS                      S        H              B S F
      A       SC  H            HNC    I    B   SMC N
      L       TR  A            PCR    N    B   TNR U
      U       NF  E            AIF    F    V   NLF 4
      1       11  3            211    1    1   111 H
              /                //                //
      CCAAGCTCATCTGCCCAGGCCACGGGTTTCAGTCCCCGGCAGATTCAGGTGTCCTGGCTGC
1741  ---------+---------+---------+---------+---------+---------+  1800
      GGTTCGAGTAGACGGTCCGGTGCCCAAAGTCAGGGGCCGTCTAAGTCCACAGGACCGACG

       K L I C Q A T G F S P R Q I Q V S W L R -


      F   B                            S   BS                   H
      NH  S           H H       AM     AA  SCM        D     H   I
      UH  P           P G       HA     VU  TRN        D     A   N
      DA  M           H A       AE     A9  NFL        E     E   F
      21  1           1 1       23     26  111        1     3   1
      /                                /   /
      GCGAGGGGAAGCAGGTGGGGGTCTGGCGTCACCACGGACCAGGTGCAGGCTGAGGCCAAAG
1801  ---------+---------+---------+---------+---------+---------+  1860
      CGCTCCCCTTCGTCCACCCCAGACCGCAGTGGTGCCTGGTCCACGTCCGACTCCGGTTTC

       E G K Q V G S G V T T D Q V Q A E A K E -


      SS      B              B
      AAHNABS                SM          H
      UUALPAP                TA          P
      99EAAN1                EE          H
      6634122                23          1
      / //                   /
      AGTCTGGGCCCACGACCTACAAGGTGACCAGCACACTGACCATCAAAGAG....
1861  ---------+---------+---------+---------+---------+        1910
      TCAGACCCGGGTGCTGGATGTTCCACTGGTCGTGTGACTGGTAGTTTCTC....

       S G P T T Y K V T S T L T I K E ....
```

47

Table 5

```
          F N                                      S             B
          N S               B       M       H      DHA           S
          U P               B       N       G      RAU           T
          4 B               V       L       A      AE9           X
          H 2               1       1       1      236           1
                                                    /
     GCCTGTTTGAGAAGCAGCGGGCAAGAAAGACGCAAGCCCAGAGGCCCTGCCATTTCTGTG
   1 ---------+---------+---------+---------+---------+---------+ 60
     CGGACAAACTCTTCGTCGCCCGTTCTTTCTGCGTTCGGGTCTCCGGGACGGTAAAGACAC

        B        PS                S                    M    HM          S
     DBS ADNPA        D   DHNA                          N    AN        HNC
     DAP VRLUU        D   RALU                          L    EL        PCR
     EN1 AAAM9        E   AEA9                          1    31        AIF
     122 22416        1   2346                               31        211
       /  / ///        /      /                         /      /         /
     GGCTCAGGTCCCTACTGGCTCAGGCCCCTGCCTCCCTCGGCAAGGCCACAATGAACCGGG
  61 ---------+---------+---------+---------+---------+---------+ 120
     CCGAGTCCAGGGATGACCGAGTCCGGGGACGGAGGGAGCCGTTCCGGTGTTACTTGGCCC

                                                      M   N   R   G -

        H                     F              F
        I           B         N      HH      N  M   D
        N           B         U      HA      U  N   D
        F           V         4      AE      4  L   E
        1           1         H      12      H  1   1
     GAGTCCCTTTTAGGCACTTGCTTCTGGTGCTGCAACTGGCGCTCCTCCCAGCAGCCACTC
 121 ---------+---------+---------+---------+---------+---------+ 180
     CTCAGGGAAAATCCGTGAACGAAGACCACGACGTTGACCGCGAGGAGGGTCGTCGGTGAG

        V  P  F  R  H  L  L  L  V  L  Q  L  A  L  L  P  A  A  T  Q -

           B     E  E                                R     A
           B     C  C                                S     L
           V     O  O                                A     U
           1     K  K                                1     1
     AGGGAAAGAAAGTGGTGCTGGGCAAAAAAGGGGATACAGTGGAACTGACCTGTACAGCTT
 181 ---------+---------+---------+---------+---------+---------+ 240
     TCCCTTTCTTTCACCACGACCCGTTTTTTCCCCTATGTCACCTTGACTGGACATGTCGAA

        G  K  K  V  V  L  G  K  K  G  D  T  V  E  L  T  C  T  A  S -
                                                       H
                      M  M                             I
                      B  B                             N
                      O  O                             F
                      2  2                             1
     CCCAGAAGAAGAGCATACAATTCCACTGGAAAAACTCCAACCAGATAAAGATTCTGGGAA
 241 ---------+---------+---------+---------+---------+---------+ 300
     GGGTCTTCTTCTCGTATGTTAAGGTGACCTTTTTGAGGTTGGTCTATTTCTAAGACCCTT

        Q  K  K  S  I  Q  F  H  W  K  N  S  N  Q  I  K  I  L  G  N -
```

```
             B                S                S     F    H
            NBS       F      AA         A      A     N  H I
            LAP       O      VU         L      U     H  H N
            AN1       K      A9         U      3     D  A F
            422       1      26         1      A     2  1 1
             /               /
    ATCAGGGCTCCTTCTTAACTAAAGGTCCATCCAAGCTGAATGATCGCGCTGACTCAAGAA
301 --------+---------+---------+---------+---------+---------+ 360
    TAGTCCCGAGGAAGAATTGATTTCCAGGTAGGTTCGACTTACTAGCGCGACTGAGTTCTT

     Q   G   S   F   L   T   K   G   P   S   K   L   N   D   R   A   D   S   R   R  -

                 S                S          H        H
            MANAS            BA        I    A       I D
            BVLUT            CU        N    F       N D
            OAA9Y           L3        F    L       F E
            22461           1A        1    2       1 1
               /                /
    GAAGCCTTTGGGACCAAGGAAACTTCCCCCTGATCATCAAGAATCTTAAGATAGAAGACT
361 --------+---------+---------+---------+---------+---------+ 420
    CTTCGGAAACCCTGGTTCCTTTGAAGGGGGACTAGTAGTTCTTAGAATTCTATCTTCTGA

     S   L   W   D   Q   G   N   F   P   L   I   I   K   N   L   K   I   E   D   S  -

                               S
            M       M      AMAM               M
            B       N      VNUN               A
            O       L      AL9L               E
            2       1      2161               1
                               //
    CAGATACTTACATCTGTGAAGTGGAGGACCAGAAGGAGGAGGTGCAATTGCTAGTGTTCG
421 --------+---------+---------+---------+---------+---------+ 480
    GTCTATGAATGTAGACACTTCACCTCCTGGTCTTCCTCCTCCACGTTAACGATCACAAGC

     D   T   Y   I   C   E   V   E   D   Q   K   E   E   V   Q   L   L   V   F   G  -

                               B
                               S                          S
                               P                          T
                               M                          Y
                               1                          1
    GATTGACTGCCAACTCTGACACCCACCTGCTTCAGGGGCAGAGCCTGACCCTGACCTTGG
481 --------+---------+---------+---------+---------+---------+ 540
    CTAACTGACGGTTGAGACTGTGGGTGGACGAAGTCCCCGTCTCGGACTGGGACTGGAACC

     L   T   A   N   S   D   T   H   L   L   Q   G   Q   S   L   T   L   T   L   E  -
```

```
                  B  BS                                      H
                  BS SC              D              M        I        S
                  AP TR              D              N        N        T
                  N1 NF              E              L        F        Y
                  22 11              1              1        1        1
                  /  /
      AGAGCCCCCCTGGTAGTAGCCCCTCAGTGCAATGTAGGAGTCCAAGGGGGTAAAAACATAC
541   ---------+---------+---------+---------+---------+---------+ 600
      TCTCGGGGGGACCATCATCGGGGAGTCACGTTACATCCTCAGGTTCCCCATTTTTTGTATG
        S  P  P  G  S  S  P  S  V  Q  C  R  S  P  R  G  K  N  I  Q -


                                  N            BBH S    B            BS
                  M    MD         ASP    A     BSSGSC   S    B   N    SC
                  B    ND         LPV    L     APTIAR   T    A   L    TR
                  0    LE         UBU    U     N1NACF   X    N   A    NF
                  2    11         122    1     221111   1    1   4    11
                                  //           / ///                /
      AGGGGGGGGAAGACCCTCTCCGTGTCTCAGCTGGAGCTCCAGGATAGTGGCACCTGGACAT
601   ---------+---------+---------+---------+---------+---------+ 660
      TCCCCCCCCTTCTGGGAGAGGCACAGAGTCGACCTCGAGGTCCTATCACCGTGGACCTGTA
        G  G  K  T  L  S  V  S  Q  L  E  L  Q  D  S  G  T  W  T  C -


      N
      NS                                M                    NV   A
      LP                                B                    HA   L
      AH                                0                    EE   U
      31                                2                    11   1
      /
      GCACTGTCTTGCAGAACCAGAAGAAGGTGGAGTTCAAAATAGACATCGTGGTGCTAGCTT
661   ---------+---------+---------+---------+---------+---------+ 720
      CGTGACAGAACGTCTTGGTCTTCTTCCACCTCAAGTTTTATCTGTAGCACCACGATCGAA
        T  V  L  Q  N  Q  K  K  V  E  F  K  I  D  I  V  V  L  A  F -


                  HS              M  M
                  AT              N  N
                  EU              L  L
                  31              1  1
                  /
      TCCAGAAGGCCTCCAGCATAGTCTATAAGAAAGAGGGGGGAACAGGTGGAGTTCTCCTTCC
721   ---------+---------+---------+---------+---------+---------+ 780
      AGGTCTTCCGGAGGTCGTATCAGATATTCTTTCTCCCCCTTGTCCACCTCAAGAGGAAGG
        Q  K  A  S  S  I  V  Y  K  K  E  G  E  Q  V  E  F  S  F  P -


                        A                    A        M
                        L                    L        N
                        U                    U        L
                        1                    1        1
      CACTCGCCTTTACAGTTGAAAAGCTGACGGGCAGTGGCGAGCTGTGGTGGCAGGCGGAGA
781   ---------+---------+---------+---------+---------+---------+ 840
      GTGAGCGGAAATGTCAACTTTTCGACTGCCCGTCACCGCTCGACACCACCGTCCGCCTCT

        L  A  F  T  V  E  K  L  T  G  S  G  E  L  W  W  Q  A  E  R -
```

EP 0 325 262 B1

```
                      P  S
         H     M  FM  A                              M
         P     N  LN  U                              B
         H     L  ML  3                              O
         1     1  11  A                              2
    GGGCTTCCTCCTCCAAGTCTTGGATCACCTTTGACCTGAAGAACAAGGAAGTGTCTGTAA
841 --------- +--------- +--------- +--------- +--------- +--------- + 900
    CCCGAAGGAGGAGGTTCAGAACCTAGTGGAAACTGGACTTCTTGTTCCTTCACAGACATT

      A   S   S   S   K   S   W   I   T   F   D   L   K   N   K   E   V   S   V   K  -
```

```
         B           BS    PS
         SM          SCADNPAD    A                    A H
         TA          TRVRLUUD    L                    L P
         EE          NFAAAM9E    U                    U H
         23          11224161    1                    1 1
          ↓           / / //
    AACGGGTTACCCAGGACCCTAAGCTCCAGATGGGCAAGAAGCTCCCGCTCCACCTCACCC
901 --------- +--------- +--------- +--------- +--------- +--------- + 960
    TTGCCCAATGGGTCCTGGGATTCGAGGTCTACCCGTTCTTCGAGGGCGAGGTGGAGTGGG

      R   V   T   Q   D   P   K   L   Q   M   G   K   K   L   P   L   H   L   T   L  -
```

```
            BS                                        BSS
         M  SC HS       D          M  H                SCAHM
         N  TR AT       D          N  P                TRUAN
         L  NF EU       E          L  H                NF9EL
         1  11 31       1          1  1                11631
             /  /                                       / /
    TGCCCCAGGCCTTGCCTCAGTATGCTGGCTCTGGAAACCTCACCCTGGCCCTTGAAGCGA
961 --------- +--------- +--------- +--------- +--------- +--------- + 1020
    ACGGGGTCCGGAACGGAGTCATACGACCGAGACCTTTGGAGTGGGACCGGGAACTTCGCT

      P   Q   A   L   P   Q   Y   A   G   S   G   N   L   T   L   A   L   E   A   K  -
```

```
                        S          BS
                        F          SC                 H D    A
                        A          TR                 P D    L
                        N          NF                 H E    U
                        1          11                 1 1    1
                                    /
    AAACAGGAAAGTTGCATCAGGAAGTGAACCTGGTGGTGATGAGAGCCACTCAGCTCCAGA
1021 --------- +--------- +--------- +--------- +--------- +--------- + 1080
    TTTGTCCTTTCAACGTAGTCCTTCACTTGGACCACCACTACTCTCGGTGAGTCGAGGTCT

      T   G   K   L   H   Q   E   V   N   L   V   V   M   R   A   T   Q   L   Q   K  -
```

51

```
                            PS          S
            M               ADNNPA      DF   AM        DE   A
            N               VRLLUU      DA   LN        DS   L
            L               AAAAM9      EN   UL        EP   U
            1               224416      11   11        11   1
                            /////       /    /         /
       AAAATTTGACCTGTGAGGTGTGGGGACCCACCTCCCCTAAGCTGATGCTGAGCTTGAAAC
1081   --------+---------+---------+---------+---------+---------+  1140
       TTTTAAACTGGACACTCCACACCCCTGGGTGGAGGGGATTCGACTACGACTCGAACTTTG

        N   L   T   C   E   V   W   G   P   T   S   P   K   L   M   L   S   L   K   L  -

            M                           T                   H               M       DM
            N                           A                   P               N       DS
            L                           Q                   A               L       ET
            1                           1                   2               1       12
                                                                                    /
       TGGAGAACAAGGAGGCAAAGGTCTCGAAGCGGGAGAAGCCGGTGTGGGTGCTGAACCCTG
1141   --------+---------+---------+---------+---------+---------+  1200
       ACCTCTTGTTCCTCCGTTTCCAGAGCTTCGCCCTCTTCGGCCACACCCACGACTTGGGAC

        E   N   K   E   A   K   V   S   K   R   E   K   P   V   W   V   L   N   P   E  -

                                    H                       PS          H
                        F   D   M   I   A                   ADPA        I
                        0   D   A   N   V                   VRUU        N
                        K   E   E   F   A                   AAM9        F
                        1   1   3   1   1                   2216        1
                                                            ///
       AGGCGGGGATGTGGCAGTGTCTGCTGAGTGACTCGGCACAGGTCCTGCTGGAATCCAACA
1201   --------+---------+---------+---------+---------+---------+  1260
       TCCGCCCCTACACCGTCACAGACGACTCACTGAGCCCTGTCCAGGACGACCTTAGGTTGT

        A   G   M   W   Q   C   L   L   S   D   S   G   Q   V   L   L   E   S   N   I  -

                        S           SA      BHF BS                          B
                        ANA         HNCP    SGNMAANXA                       SH
                        VLU         PCRA    PIUNMULHV                       PP
                        AA9         AIFL    1ADLH3A0A                       1H
                        236         2111    21211A421                       21
                        //          //      / / / /
       TCAAGGTTCTGCCCACATGGTCCACCCCGGTGCACGCGGATCCCGAGGGTGAGTGTGCCC
1261   --------+---------+---------+---------+---------+---------+  1320
       AGTTCCAAGACGGGTGTACCAGGTGGGGCCACGTGCGCCTAGGGCTCCCACTCACACGGG

        K   V   L   P   T   W   S   T   P   V   H   A   D   P   E
```

52

```
        BS S
  MF    SC F    DHNA    HNC       A M  M
  AD    TR A    RALU    PCR       F A  B
  EK    NF N    AEA9    AIF       L E  0
  11    11 1    2346    211       3 2  2
  /     /       /       /
       TAGAGTAGCCTGCATCCAGGGACAGGCCCCAGCCGGGTGCTGACACGTCCACCTCCATCT
  1321 ----------+---------+---------+---------+---------+---------+ 1380
       ATCTCATCGGACGTAGGTCCCTGTCCGGGGTCGGCCCACGACTGTGCAGGTGGAGGTAGA
```

```
              BS      S
  M  D    M   SC  M ANA M              M       S
  N  D    N   TR  B VLU B              N       T
  L  E    L   NF  0 AA9 0              L       Y
  1  1    1   11  2 246 2              1       1
                  /     /
       CTTCCTCAGCACCTGAACTCCTGGGGGGGACCGTCAGTCTTCCTCTTCCCCCCAAAACCCA
  1381 ----------+---------+---------+---------+---------+---------+ 1440
       GAAGGAGTCGTGGACTTGAGGACCCCCCTGGCAGTCAGAAGGAGAAGGGGGGTTTTGGGT
```

```
          A   P   E   L   L   G   G   P   S   V   F   L   F   P   P   K   P   K   -
```

```
          S
          AN    M HMANNAC DM. M       N           M
          UL    N PNVCLUR DS  A       NS          A
          3A    L ALAIA9F ET  E       LP          E
          A3    1 2121461 12  3       AH          2
                  / / //  /           31
                                      /
       AGGACACCCTCATGATCTCCCGGACCCCTGAGGTCACATGCGTGGTGGTGGACGTGAGCC
  1441 ----------+---------+---------+---------+---------+---------+ 1500
       TCCTGTGGGAGTACTAGAGGGCCTGGGGACTCCAGTGTACGCACCACCACCTGCACTCGG
```

```
          D   T   L   M   I   S   R   T   P   E   V   T   C   V   V   V   D   V   S   H   -
```

```
          M       DM  M               RM  M
          N       DS  B               SA  N
          L       ET  0               AE  L
          1       12  2               12  1
                  /
       ACGAAGACCCTGAGGTCAAGTTCAACTGGTACGTGGACGGCGTGGAGGTGCATAATGCCA
  1501 ----------+---------+---------+---------+---------+---------+ 1560
       TGCTTCTGGGACTCCAGTTCAAGTTGACCATGCACCTGCCGCACCTCCACGTATTACGGT
```

```
          E   D   P   E   V   K   F   N   W   Y   V   D   G   V   E   V   H   N   A   K   -
```

```
              F FN                        S
          M   N NSS         R         M  R HNC HH
          N   U UPA         S         A  S PCR GP
          L   4 DBC         A         E  A AIF AH
          1   H 222         1         2  1 211 11
                //                          /
       AGACAAAGCCGCGGGAGGAGCAGTACAACAGCACGTACCGGGTGGTCAGCGTCCTCACCG
  1561 ----------+---------+---------+---------+---------+---------+ 1620
       TCTGTTTCGGCGCCCTCCTCGTCATGTTGTCGTGCATGGCCCACCAGTCGCAGGAGTGGC
```

```
          T   K   P   R   E   E   Q   Y   N   S   T   Y   R   V   V   S   V   L   T   V   -
```

```
       BS
  M    SC                    R
  N    TR                    S
  L    NF                    A
  1    11                    1
        /
      TCCTGCACCAGGACTGGCTGAATGGCAAGGAGTACAAGTGCAAGGTCTCCAACAAAGCCC
1621  ---------+---------+---------+---------+---------+---------+ 1680
      AGGACGTGGTCCTGACCGACTTACCGTTCCTCATGTTCACGTTCCAGAGGTTGTTTCGGG

       L   H   Q   D   W   L   N   G   K   E   Y   K   C   K   V   S   N   K   A   L  -

                                            P S              S
            M   T                         ADNNPMA            A
            N   A                         VRLLUNU            U
            L   Q                         AAAAML9            9
            1   1                         2244116            6
                                           //// /
      TCCCAGCCCCCATCGAGAAAACCATCTCCAAAGCCAAAGGTGGGACCCGTGGGGTGCGAG
1681  ---------+---------+---------+---------+---------+---------+ 1740
      AGGGTCGGGGGTAGCTCTTTTGGTAGAGGTTTCGGTTTCCACCCTGGGCACCCCACGCTC

       P   A   P   I   E   K   T   I   S   K   A   K

                              S
                                                      N
      H M     N        HHN   BSAH         D M   M     S    R
      A N     L        APA   GFUA         D N   A     P    S
      E L     A        EAE   LI9E         E L   E     B    A
      3 1     3        321   1163         1 1   3     2    1

      GGCCACATGGACAGAGGCCGGCTCGGCCCACCCTCTGCCCTGAGAGTGACCGCTGTACCA
1741  ---------+---------+---------+---------+---------+---------+ 1800
      CCGGTGTACCTGTCTCCGGCCGAGCCGGGTGGGAGACGGGACTCTCACTGGCGACATGGT

                     F                                      SS
            M        N    A        B           R F          AHNNCC
            N        U    V        B           S D          VPCCRR
            L        4    A        V           A K          AAIIFF
            1        H    1        1           1 1          121111
                                                            ////
      ACCTCTGTCCTACAGGGCAGCCCCGAGAACCACAGGTGTACACCCTGCCCCCATCCCGGG
1801  ---------+---------+---------+---------+---------+---------+ 1860
      TGGAGACAGGATGTCCCGTCGGGGCTCTTGGTGTCCACATGTGGGACGGGGGTAGGGCCC

              G   Q   P   R   E   P   Q   V   Y   T   L   P   P   S   R   D  -
```

```
                          BS              BS  B
        S    A     F     SC             SC  S
        M    L     0     TR             TR  P
        A    U     K     NF             NF  M
        1    1     1     11             11  1
        /          /     /              /
        ATGAGCTGACCAAGAACCAGGTCAGCCTGACCTGCCTGGTCAAAGGCTTCTATCCCAGCG
   1861 ---------+---------+---------+---------+---------+---------+ 1920
        TACTCGACTGGTTCTTGGTCCAGTCGGACTGGACGGACCAGTTTCCGAAGATAGGGTCGC

        E  L  T  K  N  Q  V  S  L  T  C  L  V  K  G  F  Y  P  S  D  -
                                        F
                                    N   H       B
                                    U   P       B
                                    4   A       V
                                    H   2       1
        ACATCGCCGTGGAGTGGGAGAGCAATGGGCAGCCGGAGAACAACTACAAGACCACGCCTC
   1921 ---------+---------+---------+---------+---------+---------+ 1980
        TGTAGCGGCACCTCACCCTCTCGTTACCCGTCGGCCTCTTGTTGATGTTCTGGTGCGGAG

        I  A  V  E  W  E  S  N  G  Q  P  E  N  N  Y  K  T  T  P  P  -
                H
            M I     M       N           H       M A             B
            N N     B       L           P       N L             S
            L F     0       A           H       L U             P
            1 1     2       4           1       1 1             M
                                                                1
        CCGTGCTGGACTCCGACGGCTCCTTCTTCCTCTACAGCAAGCTCACCGTGGACAAGAGCA
   1981 ---------+---------+---------+---------+---------+---------+ 2040
        GGCACGACCTGAGGCTGCCGAGGAAGAAGGAGATGTCGTTCGAGTGGCACCTGTTCTCGT

        V  L  D  S  D  G  S  F  F  L  Y  S  K  L  T  V  D  K  S  R  -
            F
            NM          MBX         NF  M       N N
            UB          ABM         LA  N       S L
            40          EVN         AN  L       I A
            H2          211         31  1       1 3
                                    /
        GGTGGCAGCAGGGGGAACGTCTTCTCATGCTCCGTGATGCATGAGGCTCTGCACAACCACT
   2041 ---------+---------+---------+---------+---------+---------+ 2100
        CCACCGTCGTCCCCTTGCAGAAGAGTACGAGGCACTACGTACTCCGAGACGTGTTGGTGA

        W  Q  Q  G  N  V  F  S  C  S  V  M  H  E  A  L  H  N  H  Y  -
                                S
                    M   M   HNC         CXH
                    B   N   PCR         FMA
                    0   L   AIF         RAE
                    2   1   211         133
                            /           /
        ACACGCAGAAGAGCCTCTCCCTGTCTCCGGGTAAATGAGTGCGACGGCCG
   2101 ---------+---------+---------+---------+---------+ 2150
        TGTGCGTCTTCTCGGAGAGGGACAGAGGCCCATTTACTCACGCTGCCGGC

        T  Q  K  S  L  S  L  S  P  G  K  *
```

Example 2: Preparation of the Fusion Proteins from Supernatants of COS Cells

COS cells grown in DME medium supplemented with 10% Calf Serum and gentamicin sulfate at 15 µg/ml were split into DME medium containing 10% NuSerum (Collaborative Research) and gentamicin to give 50% confluence the day before transfection. The next day, CsCl purified plasmid DNA was added to a final concentration of 0.1 to 2.0 $\mu$g/ml followed by DEAE Dextran to 400 $\mu$g/ml and chloroquine to 100 $\mu$M. After 4 hours at 37°C, the medium was aspirated and a 10% solution of dimethyl sulfoxide in phosphate buffered saline was added for 2 minutes, aspirated, and replaced with DME/10% Calf Serum. 8 to 24 hours later, the cells were trypsinized and split 1:2.

For radiolabeling, the medium was aspirated 40 to 48 hours after transfection, the cells washed once with phosphate buffered saline, and DME medium lacking cysteine or methionine was added. 30 minutes later, $^{35}$S-labeled cysteine and methionine were added to final concentrations of 30-60 $\mu$ci and 100-200 $\mu$ci respectively, and the cells allowed to incorporate label for 8 to 24 more hours. The supernatants were recovered and examined by electrophoresis on 7.5% polyacrylamide gels following denaturation and reduction, or on 5% polyacrylamide following denaturation without reduction. The CD4B$\gamma$1 protein gave the same molecular mass with or without reduction, while the CD4E$\gamma$1 and CD4H$\gamma$1 fusion proteins showed molecular masses without reduction of twice the mass observed with reduction, indicating that they formed dimer structures. The CD4 IgM fusion proteins formed large multimers beyond the resolution of the gel system without reduction, and monomers of the expected molecular mass with reduction.

Unlabeled proteins were prepared by allowing the cells to grow for 5 to 10 days post transfection in DME medium containing 5% NuSerum and gentamicin as above. The supernatants were harvested, centrifuged, and purified by batch adsorption to either protein A trisacryl, protein A agarose, goat anti-human IgG antibody agarose, rabbit anti-human IgM antibody agarose, or monoclonal anti-CD4 antibody agarose. Antibody agarose conjugates were prepared by coupling purified antibodies to cyanogen bromide activated agarose according to the manufacturer's recommendations, and using an antibody concentration of 1 mg/ml. Following batch adsorption by shaking overnight on a rotary table, the beads were harvested by pouring into a sintered glass funnel and washed a few times on the funnel with phosphate buffered saline containing 1% Nonidet P40 detergent. The beads were removed from the funnel and poured into a small disposable plastic column (Quik-Sep QS-Q column, Isolab), washed with at least 20 column volumes of phosphate buffered saline containing 1% Nonidet P40, with 5 volumes of 0.15 M NaCl, 1 mM EDTA (pH 8.0), and eluted by the addition of either 0.1 M acetic acid, 0.1 M acetic acid containing 0.1 M NaCl, or 0.25 M glycine-HCl buffer, pH 2.5.

Example 3: Blockage of Syncytium Formation by the Fusion Proteins

Purified or partially purified fusion proteins were added to HPB-ALL cells infected 12 hours previously with a vaccinia virus recombinant encoding HIV envelope protein. After incubation for 6-8 more hours, the cells were washed with phosphate buffered saline, fixed with formaldehyde, and photographed. All of the full-length CD4 immunoglobulin fusion proteins showed inhibition of syncytium formation at a concentration of 20 $\mu$g/ml with the exception of the 4H$\gamma$1 protein, which was tested only at 5 $\mu$g/ml and showed partial inhibition of syncytium formation under the same conditions.

Example 4: Chromium Release Cytolysis Assay

The purified fusion proteins were examined for ability to fix complement in a chromium release assay using vaccinia virus infected cells as a model system. Namalwa (B cell) or HPB-ALL (T cell) lines were infected with vaccinia virus encoding HIV envelope protein, and 18 hours later were radiolabeled by incubation in 1 mci/ml sodium $^{51}$chromate in phosphate buffered saline for 1 hour at 37°. The labeled cells were centrifuged to remove the unincorporated chromate, and incubated in microtiter wells with serial dilutions of the CD4 immunoglobulin fusion proteins and rabbit complement at a final concentration of 40%. After 1 hour at 37°, the cells were mixed well, centrifuged, and the supernatants counted in a gamma-ray counter. No specific release could be convincingly documented.

Example 5: Binding of the CD4E$\gamma$1 Protein to Fc Receptors

Purified CD4E$\gamma$1 fusion protein was tested for its ability to displace radiolabeled human IgG1 from human Fc receptors expressed on COS cells in culture. The IgG1 was radiolabeled with sodium $^{125}$iodide using 1 mci of iodide, 100 $\mu$g of IgG1, and two idobeads (Pierce). The labeled protein was separated from unincorporated counts by passage over a Sephadex G25 column equilibrated with phosphate buffered saline containing 0.5 mM EDTA and 5% nonfat milk. Serial dilutions of the CO4E$\gamma$1 fusion protein or unlabeled IgG1 were prepared and mixed with a constant amount of radiolabeled IgG1 tracer. After incubation with COS cells bearing the FcRI and RcRII receptors at 4°C for at least 45 minutes in a volume of 20 $\mu$l, 200 $\mu$l of a 3:2 mixture of dibutyl to dioctyl phthalates were added, and the cells separated from the unbound label by centrifugation in a microcentrifuge for 15 to 30 seconds. The tubes were cut with scissors, and the cell pellets counted in a gamma-ray counter. The affinity of the CD4E$\gamma$1 protein for receptors was measured in parallel with the affinity of the authentic IgG1 protein, and was found to be the same, within experimental error.

Example 6: Stable Expression of the Fusion Construct pCD4E$\gamma$1 in Baby Hamster Kidney Cells

Twenty-four hours before transfection, 0.5 x 10$^6$ baby hamster kidney cells (BHK; ATCC CCL10) were seeded in a 25 cm$^2$ culture flask in Dulbecco's modified Eagle's medium (DMEM) containing 10% of fetal calf serum (FCS). The cells were cotransfected with a mixture of the plasmids pCD4E$\gamma$1 (20 $\mu$g), pSV2dhfr (5 $\mu$g; Lee et al., Nature 294:228-232 (1981)) and pRMH140 (5 $\mu$g, Hudziak et al., Cell 31:137-146 (1982)) according to a modified calcium phosphate transfection technique as described in Zettlmeissl et al. (Behring Inst. Res. Comm. 82:26-34 (1988)). 72 h post-transfection, cells were split 1:3 to 1:4 (60 mm culture dishes) and resistant colonies were selected in DMEM medium containing 10% FCS, 400 $\mu$g/ml G418 (Geneticin, Gibco) and 1 $\mu$M methotrexate (selection medium). The medium was changed twice a week. The resistant colonies (40-100/transfection) appeared 10-15 day post-transfection and were further propagated either as a mixture of clones (i.e., BHK-MK1) or as individually isolated clones. For the determination of the relative expression levels, clone mixtures or individual clones were grown to confluency in T25 culture flasks, washed twice with protein-free DMEM medium, and incubated for 24 h with 5 ml protein-free DMEM medium. These media were collected and subjected to a human IgG specific ELISA in order to determine the relative expression levels of the CD4-IgG1 fusion protein CD4E$\gamma$1. For further analysis an individual clone (BHK-UC3) was chosen due to its high relative expression levels.

Example 7: Detection of the CD4E$\gamma$1 Protein in Culture Supernatants

For $^{35}$S methionine labeling of cells, the clone BHK-UC3 and untransfected BHK cells (control) were grown to confluency in T25 culture flasks and subsequently incubated for two hours in HamF12 medium without methionine. Labeling was achieved by incubating 24 h in 2.5 ml of the same medium containing 100 $\mu$Ci $^{35}$S methionine (1070 Ci/mmole, Amersham). For the preparation of cell lysates, the labeled cells were harvested in 1 ml of phosphate buffered saline, pH 7.2 (PBS) and lysed by repetitive freezing and thawing. Cleared lysates (after centrifugation 20000 rpm, 20 min) and culture supernatants were incubated with Protein A-Sepharose (Pharmacia) and the bound material was analyzed on a 10% SDS-Protein A-Sepharose (Pharmacia) and the bound material was analyzed on a 10% SDS-gel according to Laemmli (Nature 227:680-685 (1970)), which was subsequently autoradiographed. A specific band of about 80 KDa can be detected only in the supernatant of clone BHK-UC3, which is absent in the lysate of clone BHK-UC3 and in the respective controls.

Example 8: Purification of the Protein CD4E$\gamma$1 from Culture Supernatants

In order to demonstrate that the fusion protein coded by the plasmid pCD4E$\gamma$1 can be obtained in high quantities, the clone BHK-UC3 was grown in 1750 cm$^2$ roller bottles in selection medium (500 ml). Confluent monolayers were washed twice with protein-free DMEM medium (200 ml) and further incubated for 48 h with protein-free DMEM medium (500 ml). The conditioned culture supernatants (1-2 l) and respective supernatants from untransfected BHK cells were cleared by centrifugation (9000 rpm, 30 min) and microfiltered through a 0.45 $\mu$m membrane (Nalgene). After addition of 1% (v/v) of 1.9 M Tris-HCl buffer, pH 8.6, the conditioned medium was absorbed to a Protein A-Sepharose column equilibrated with 50 mM Tris-HCl pH 8.6 buffer containing 150 mM NaCl (4°C). The loaded column was washed with 10 column volumes of equilibration buffer. Elution of the CD4-IgG1 fusion protein CD4E$\gamma$1 was achieved with 0.1 M sodium citrate buffer, pH 3, followed by immediate neutralization of the column efflux to pH 8 by Tris-base. The peak fractions were pooled, and the pool was analyzed on a Coomassie blue stained SDS-gel resulting in a band of the expected size (80 KDa), and which reacted with a polyclonal anti-human IgG heavy chain antibody and a mouse monoclonal anti-CD4 antibody (BMA040, Behringwerke) in Western Blots. The yields of purified fusion proteins obtained by the given procedure is 5-18 mg/24 h/l culture supernatant. The respective value for a BHK clone mixture (about 80 resistant clones; BHK-MK1) as described above was 2-3 mg/24 h/l.

Example 9: Physical and Biological Characterization of the CD4E$\gamma$1 Fusion Protein

As proven by SDS-electrophoresis on 10-15% gradient gels (Phast-System, Pharmacia) under non-reductive conditions, the CD4E$\gamma$1 fusion protein migrates at the position of a homodimer (about 160 KDa) like a non-reduced mouse monoclonal antibody. This result is supported by analytical equilibrium ultracentrifugation, where the fusion protein behaves as a homogeneous dimeric molecule of about 150 KDa. The absorbance coefficient of the protein was determined as $A_{280}$ = 18 cm$^2$/mg using the quantitative protein determination according to Bradford (Anal. Biochem. 72:248-254 (1976)).

The CD4E$\gamma$1-fusion protein shows specific complex formation with a solubilized $\beta$gal-gp120 fusion protein (pMB1790; Broker et al., Behring Inst. Res. Commun. 82:338-348 (1988)) expressed in E. coli. In this protein (110 KDa), a major part of the HIV gp120 protein (Val$_{49}$-Trp$_{646}$) is fused to $\beta$-galactosidase (amino acids 1-375). In a control experiment a 67-KDa $\beta$gal-HIV 3'orf fusion protein ($\beta$gal1-375; 3'orf Pro14-Asp123) showed no complex formation. In these experiments, the CD4E$\gamma$1-protein was incubated with the respective fusion protein in molar rations of about 5:1. The complex was isolated by binding to Protein A-Sepharose and the Protein A-Sepharose bound proteins--together with relevant controls--were analyzed on 10-15% gradient SDS-gels (Phast-System, Pharmacia).

The CD4E$\gamma$1 fusion protein binds to the surface of HIV (HIV1/HTLV-IIIB) infected cultured T4-lymphocytes as determined by direct immunofluorescence with fluorescein-isothiocyanate (FITC) labeled CD4E$\gamma$1 protein. It blocks syncytia formation in cultured T4-lymphocytes upon HIV infection (0.25 TCID/cell) at a concentration of 10 $\mu$g/ml. Furthermore, HIV-infected cultured T4-lymphocytes (subclone of cell line H9) are selectively killed upon incubation with CD4E$\gamma$1 in the presence or absence of complement: To a highly (>50%) HIV infected culture of T4-lymphocytes (10$^6$ cells/ml) 50, 10 or 1 $\mu$g/ml CD4E$\gamma$1 fusion protein was added in the presence or absence of guinea pig complement. Cells were observed for specific killing by the fusion protein, which is defined by the percentage of killed cells after 3 days in relation to viable cells in the culture at the beginning of the experiment corrected by the values for unspecific killing observed in control cultures, lacking the CD4E$\gamma$1 fusion protein (Table 5, Experiment I). Surprisingly, addition of CD4E$\gamma$1 protein to the infected T4 cells in the absence of complement resulted in similar specific killing rates as in the presence of complement (Table 5, Experiment II). This result demonstrates a complement independent cytolytic effect of CD4Ey1 on HIV infected T-lymphocytes in culture.

Table 5

| No. Experiment | Assay System | Specific Killing (%) |
|---|---|---|
| I | non-infected T4-cells + 50 $\mu$g/ml CD4E$\gamma$1 + Compl. | 0.7 |
|  | infected T4-cells + 50 $\mu$g/ml CD4E$\gamma$1 + Compl. | 35.1 |
|  | infected T4-cells + 10 $\mu$g/ml CD4E$\gamma$1 + Compl. | 25.1 |
|  | infected T4-cells + 1 $\mu$g/ml CD4E$\gamma$1 + Compl. | 25 |
| II | infected T4-cells + 10 $\mu$g/ml CD4E$\gamma$1 + Compl. | 49.9 |
|  | infected T4-cells + 10 $\mu$g/ml CD4E$\gamma$1 + Compl. | 69.4 |

Having now fully described this invention, it will be appreciated by those skilled in the art that the same can be performed with any wide range of equivalent parameters of composition, conditions, and methods of preparing such fusion proteins without departing from the spirit or scope of the invention or any embodiment thereof.

**Claims**

**Claims for the following Contracting States : AT, BE, CH, DE, FR, GB, IT, LI, LU, NL, SE**

**1.** A gene encoding a fusion protein which is capable of being secreted comprising 1) the DNA sequence of extracellular CD4, or fragment thereof which binds to HIV gp 120 when fused to an immunoglobulin chain, and 2) the DNA sequence of an immunoglobulin heavy chain, characterized in that the DNA sequence which encodes at least the variable region of said immunoglobulin has been replaced with the DNA sequence which encodes extracellular CD4, or said gp120 binding fragment thereof.

2. The fusion protein gene claim 1, characterized in the said immunoglobulin chain is of the class IgM, IgG1 or IgG3.

3. A vector comprising the fusion protein gene of claim 1, or having the identifying characteristics of pCD4H$\gamma$1, which has been deposited under Accession No. 67611, or pCD4M$\mu$, which has been deposited under Accession No. 67608, or of pCH4P$\mu$, which has been deposited under Accession No. 67609, or of pCD4E$\gamma$1, which has been deposited under Accession No. 67610, all in E. coli at the ATCC under the terms of the Budapest Treaty.

4. A host transformed with the vector of claim 3.

5. The host of claim 4 which expresses an immunoglobulin light chain together with the expression product of said fusion protein gene to give an immunoglobulin-like molecule which binds to gp120.

6. The host of claim 5, wherein said immunoglobulin heavy chain is of the immunoglobulin class IgM, IgG1 or IgG3.

7. A method of producing a fusion protein encoded by a gene of claim 1, characterized by cultivating in a nutrient medium under protein-producing conditions, a host strain transformed with the vector comprising the gene of claim 1, said vector further comprising expression signals which are recognized by said host strain and direct expression of said fusion protein, and recovering the fusion protein so produced.

8. The method of claim 7, wherein said host strain is a myeloma cell line which produces immunoglobulin light chains and said fusion protein comprises an immunoglobulin heavy chain of the class IgM, IgG1 or IgG3, wherein an immunoglobulin-like molecule comprising said fusion protein is produced.

9. A fusion protein encoded by a gene according to claim 1.

10. A fusion protein of claim 9, which further comprises a therapeutic agent, a detectable label or NMR imaging agent linked to said fusion protein.

11. The fusion proteins CD4H$\gamma$1, CD4M$\mu$, CD4P$\mu$, CD4E$\gamma$1 producable by the vectors ATCC 67611, 67608, 67609 and 67610, respectively or CD3B$\gamma$1 according to the following amino acid sequence

```
               F N                          S              B
               N S           B      M      H   DHA         S
               U P           B      N      G   RAU         T
               4 B           V      L      A   AE9         X
               H 2           1      1      1   236         1
                                                /
        GCCTGTTTGAGAAGCAGCGGGCAAGAAAGACGCAAGCCCAGAGGCCCTGCCATTTCTGTG
    1   -----------+---------+---------+---------+---------+---------+  60
        CGGACAAACTCTTCGTCGCCCGTTCTTTCTGCGTTCGGGTCTCCGGGACGGTAAAGACAC

               B   PS                S                          S
            DBS ADNPA       D     DHNA            M    HM        HNC
            DAP VRLUU       D     RALU            N    AN        PCR
            EN1 AAAV9       E     AEA9            L    EL        AIF
            122 22416       1     2346            1    31        211
             /   / //              /                   /          /
        GGCTCAGGTCCCTACTGGCTCAGGCCCCTGCCTCCCTCGGCAAGGCCACAATGAACCGGG
   61   -----------+---------+---------+---------+---------+---------+  120
        CCGAGTCCAGGGATGACCGAGTCCGGGGACGGAGGGAGCCGTTCCGGTGTTACTTGGCCC

                                                     M   N   R   G  -

        H                     F                   F
        I           B         N        HH         N M        D
        N           B         U        HA         U N        D
        F           V         4        AE         4 L        E
        1           1         H        12         H 1        1
        GAGTCCCTTTTAGGCACTTGCTTCTGGTGCTGCAACTGGCGCTCCTCCCAGCAGCCACTC
   121  -----------+---------+---------+---------+---------+---------+  180
        CTCAGGGAAAATCCGTGAACGAAGACCACGACGTTGACCGCGAGGAGGGTCGTCGGTGAG

            V   P   F   R   H   L   L   L   V   L   Q   L   A   L   L   P   A   A   T   Q  -

            B       E   E                                        R       A
            B       C   C                                        S       L
            V       0   0                                        A       U
            1       K   K                                        1       1
        AGGGAAAGAAAGTGGTGCTGGGCAAAAAAGGGGATACAGTGGAACTGACCTGTACAGCTT
   181  -----------+---------+---------+---------+---------+---------+  240
        TCCCTTTCTTTCACCACGACCCGTTTTTTCCCCTATGTCACCTTGACTGGACATGTCGAA

            G   K   K   V   V   L   G   K   K   G   D   T   V   E   L   T   C   T   A   S  -

                            M   M                                  H
                            B   B                                  I
                            0   0                                  N
                            2   2                                  F
                                                                   1
        CCCAGAAGAAGAGCATACAATTCCACTGGAAAAACTCCAACCAGATAAAGATTCTGGGAA
   241  -----------+---------+---------+---------+---------+---------+  300
        GGGTCTTCTTCTCGTATGTTAAGGTGACCTTTTTTGAGGTTGGTCTATTTCTAAGACCCTT

            Q   K   K   S   I   Q   F   H   W   K   N   S   N   Q   I   K   I   L   G   N  -
```

60

```
            E                S                    S    F    H
       NES            F      AA            A       A    N    H    I
       LAP            O      VU            L       U    U    H    N
       AN1            K      A9            U       3    D    A    F
       422            1      26            1       A    2    1    1
              /                /
    ATCAGGGCTCCTTCTTAACTAAAGGTCCATCCAAGCTGAATGATCGCGCTGACTCAAGAA
301 ---------+---------+---------+---------+---------+--------- 360
    TAGTCCCGAGGAAGAATTGATTTCCAGGTAGGTTCGACTTACTAGCGCGACTGAGTTCTT

    Q   G   S   F   L   T   K   G   P   S   K   L   N   D   R   A   D   S   R   R -

                 S                    S         H         A              H
            MANAS               BA         I         A              I    D
            BVLUT               CU         N         F              N    D
            OAA9Y               L3         F         L              F    E
            22461               1A         1         2              1    1
                 /                    /
    GAAGCCTTTGGGACCAAGGAAACTTCCCCCTGATCATCAAGAATCTTAAGATAGAAGACT
361 ---------+---------+---------+---------+---------+--------- 420
    CTTCGGAAACCCTGGTTCCTTTGAAGGGGGACTAGTAGTTCTTAGAATTCTATCTTCTGA

    S   L   W   D   Q   G   N   F   P   L   I   I   K   N   L   K   I   E   D   S -

                             S
            M       M        AVAV                         M
            B       N        VNUN                         A
            O       L        AL9L                         E
            2       1        2161                         1
                             //
    CAGATACTTACATCTGTGAAGTGGAGGACCAGAAGGAGGAGGTGCAATTGCTAGTGTTCG
421 ---------+---------+---------+---------+---------+--------- 480
    GTCTATGAATGTAGACACTTCACCTCCTGGTCTTCCTCCTCCACGTTAACGATCACAAGC

    D   T   Y   I   C   E   V   E   D   Q   K   E   E   V   Q   L   L   V   F   G -

                             B
                             S                              S
                             P                              T
                             M                              Y
                             1                              1
    GATTGACTGCCAACTCTGACACCCACCTGCTTCAGGGGCAGAGCCTGACCCTGACCTTGG
481 ---------+---------+---------+---------+---------+--------- 540
    CTAACTGACGGTTGAGACTGTGGGTGGACGAAGTCCCCGTCTCGGACTGGGACTGGAACC

    L   T   A   N   S   D   T   H   L   L   Q   G   Q   S   L   T   L   T   L   E -
```

```
                B   BS
                BS  SC          D           M           H
                AP  TR          D           N           I       S
                N1  NF          E           L           N       T
                22  11          1           1           F       Y
                                                        1       1
                 /   /
      AGAGCCCCCCTGGTAGTAGCCCCTCAGTGCAATGTAGGAGTCCAAGGGGTAAAAACATAC
541   --------------------------+---------+---------+---------+--------+ 600
      TCTCGGGGGGACCATCATCGGGGAGTCACGTTACATCCTCAGGTTCCCCATTTTTGTATG
       S  P  P  G  S  S  P  S  V  Q  C  R  S  P  R  G  K  N  I  Q -


                                    N         BBH S   B           BS
                M   MD          ASP       A BSSGSC   S   B   N     SC
                B   ND          LPV       L APTIAR   T   A   L     TR
                0   LE          UBU       U N1NACF   X   N   A     NF
                2   11          122       1 221111   1   1   4     11
                                 //          / ///               /
      AGGGGGGGGAAGACCCTCTCCGTGTCTCAGCTGGAGCTCCAGGATAGTGGCACCTGGACAT
601   --------------------------+---------+---------+---------+--------+ 660
      TCCCCCCCCTTCTGGGAGAGGCACAGAGTCGACCTCGAGGTCCTATCACCGTGGACCTGTA
       G  G  K  T  L  S  V  S  Q  L  E  L  Q  D  S  G  T  W  T  C -


       N
       NS                           M                       NV    A
       LP                           B                       HA    L
       AH                           0                       EE    U
       31                           2                       11    1
        /
      GCACTGTCTTGCAGAACCAGAAGAAGGTGGAGTTCAAAAATAGACATCGTGGTGCTAGCTT
661   --------------------------+---------+---------+---------+--------+ 720
      CGTGACAGAACGTCTTGGTCTTCTTCCACCTCAAGTTTTATCTGTAGCACCACGATCGAA
       T  V  L  Q  N  Q  K  K  V  E  F  K  I  D  I  V  V  L  A  F -


            HS          M   M
            AT          N   N
            EU          L   L
            31          1   1
             /
      TCCAGAAGGCCTCCAGCATAGTCTATAAGAAAGAGGGGGGAACAGGTGGAGTTCTCCTTCC
721   --------------------------+---------+---------+---------+--------+ 780
      AGGTCTTCCGGAGGTCGTATCAGATATTCTTTCTCCCCCTTGTCCACCTCAAGAGGAAGG
       Q  K  A  S  S  I  V  Y  K  K  E  G  E  Q  V  E  F  S  F  P -


                        A               A           M
                        L               L           N
                        U               U           L
                        1               1           1
      CACTCGCCTTTACAGTTGAAAAGCTGACGGGCAGTGGCGAGCTGTGGTGGCAGGCGGAGA
781   --------------------------+---------+---------+---------+--------+ 840
      GTGAGCGGAAATGTCAACTTTTCGACTGCCCGTCACCGCTCGACACCACCGTCCGCCTCT

       L  A  F  T  V  E  K  L  T  G  S  G  E  L  W  W  Q  A  E  R -
```

```
              P  S
     H    N FN  A                          M
     P    N LN  U                          B
     H    L ML  3                          0
     1    1 11  A                          2
GGGCTTCCTCCTCCAAGTCTTGGATCACCTTTGACCTGAAGAACAAGGAAGTGTCTGTAA
841 --------+---------+---------+---------+---------+---------+ 900
CCCGAAGGAGGAGGTTCAGAACCTAGTGGAAACTGGACTTCTTGTTCCTTCACAGACATT

    A  S  S  S  K  S  W  I  T  F  D  L  K  N  K  E  V  S  V  K -


     B       BS   PS
     SN     SCADNPAD    A              A H
     TA     TRVRLUUD    L              L P
     EE     NFAAAM9E    U              U H
     23     11224161    1              1 1
      ↓      / / //
AACGGGTTACCCAGGACCCTAAGCTCCAGATGGGCAAGAAGCTCCCGCTCCACCTCACCC
901 --------+---------+---------+---------+---------+---------+ 960
TTGCCCAATGGGTCCTGGGATTCGAGGTCTACCCGTTCTTCGAGGGCGAGGTGGAGTGGG

    R  V  T  Q  D  P  K  L  Q  M  G  K  K  L  P  L  H  L  T  L -


            BS                              BSS
     M     SC HS      D        M  H         SCAHM
     N     TR AT      D        N  P         TRUAN
     L     NF EU      E        L  H         NF9EL
     1     11 31      1        1  1         11631
             /  /                             /  /
TGCCCCAGGCCTTGCCTCAGTATGCTGGCTCTGGAAACCTCACCCTGGCCCTTGAAGCGA
961 --------+---------+---------+---------+---------+---------+ 1020
ACGGGGTCCGGAACGGAGTCATACGACCGAGACCTTTGGAGTGGGACCGGGAACTTCGCT

    P  Q  A  L  P  Q  Y  A  G  S  G  N  L  T  L  A  L  E  A  K -


                    S       BS
                    F       SC            H D    A
                    A       TR            P D    L
                    N       NF            H E    U
                    1       11            1 1    1
                             /
AAACAGGAAAGTTGCATCAGGAAGTGAACCTGGTGGTGATGAGAGCCACTCAGCTCCAGA
1021 --------+---------+---------+---------+---------+---------+ 1080
TTTGTCCTTTCAACGTAGTCCTTCACTTGGACCACCACTACTCTCGGTGAGTCGAGGTCT

    T  G  K  L  H  Q  E  V  N  L  V  V  M  R  A  T  Q  L  Q  K -
```

```
                    PS          S
  M                 ADNNPA      DF  AM      DE  A
  N                 VRLLUU      DA  LN      DS  L
  L                 AAAAM9      EN  UL      EP  U
  1                 224416      11  11      11  1
                    /////       /   /       /
AAAATTTGACCTGTGAGGTGTGGGGACCCACCTCCCCTAAGCTGATGCTGAGCTTGAAAC
1081 --------------------------+---------+---------+----------+ 1140
TTTTAAACTGGACACTCCACACCCCTGGGTGGAGGGGATTCGACTACGACTCGAACTTTG

  N  L  T  C  E  V  W  G  P  T  S  P  K  L  M  L  S  L  K  L  -

  M                       T           H           M           DM
  N                       A           P           N           DS
  L                       Q           A           L           ET
  1                       1           2           1           12
                                                              /
TGGAGAACAAGGAGGCAAAGGTCTCGAAGCGGGAGAAGCCGGTGTGGGTGCTGAACCCTG
1141 --------------+---------+---------+---------+---------+--- 1200
ACCTCTTGTTCCTCCGTTTCCAGAGCTTCGCCCTCTTCGGCCACACCCACGACTTGGGAC

  E  N  K  E  A  K  V  S  K  R  E  K  P  V  W  V  L  N  P  E  -

                          H               PS          H
              F     D     M     I  A       ADPA        I
              O     D     A     N  V       VRUU        N
              K     E     E     F  A       AAM9        F
              1     1     3     1  1       2216        1
                                          ///
AGGCGGGGATGTGGCAGTGTCTGCTGAGTGACTCGGCACAGGTCCTGCTGGAATCCAACA
1201 --------------+---------+---------+---------+---------+--- 1260
TCCGCCCCTACACCGTCACAGACGACTCACTGAGCCCTGTCCAGGACGACCTTAGGTTGT

  A  G  M  W  Q  C  L  L  S  D  S  G  Q  V  L  L  E  S  N  I  -

              S           SA    BHF BS                    B
              ANA         HNCP  SGNVAANXA                 SH
              VLU         PCRA  PIUNVULHV                 PP
              AA9         AIFL  1ADLH3A0A                 1H
              236         2111  21211A421                 21
              //          //    / / / /
TCAAGGTTCTGCCCACATGGTCCACCCCGGTGCACGCGGATCCCGAGGGTGAGTGTGCCC
1261 --------------+---------+---------+---------+---------+--- 1320
AGTTCCAAGACGGGTGTACCAGGTGGGGCCACGTGCGCCTAGGGCTCCCACTCACACGGG

  K  V  L  P  T  W  S  T  P  V  H  A  D  P  E
```

```
           BS  S         S          S
 MF        SC  F   DHNA   HNC       A M  M
 AD        TR  A   RALU   PCR       F A  B
 EK        NF  N   AEA9   AIF       L E  D
 11        11  1   2346   211       3 2  2
 /             /      /      /
      TAGAGTAGCCTGCATCCAGGGACAGGCCCCAGCCGGGTGCTGACACGTCCACCTCCATCT
 1321 ----------+---------+---------+---------+---------+---------+ 1380
      ATCTCATCGGACGTAGGTCCCTGTCCGGGGGTCGGCCCACGACTGTGCAGGTGGAGGTAGA


                     BS          S
 M  D         M      SC  M ANA M                    M         S
 N  D         N      TR  B VLU B                    N         T
 L  E         L      NF  D AA9 D                    L         Y
 1  1         1      11  2 246 2                    1         1
                         /     /
      CTTCCTCAGCACCTGAACTCCTGGGGGGGACCGTCAGTCTTCCTCTTCCCCCCCAAAACCCA
 1391 ----------+---------+---------+---------+---------+---------+ 1440
      GAAGGAGTCGTGGACTTGAGGACCCCCCTGGCAGTCAGAAGGAGAAGGGGGGGTTTTGGGT

          A  P  E  L  L  G  G  P  S  V  F  L  F  P  P  K  P  K -


            S
            AN    M HMANNAC DM  M        N              M
            UL    N PNVCLUR DS  A        NS             A
            3A    L ALAIA9F ET  E        LP             E
            A3    1 2121461 12  3        AH             2
                    / / //   /           31
                                        /
      AGGACACCCTCATGATCTCCCGGACCCCTGAGGTCACATGCGTGGTGGTGGACGTGAGCC
 1441 ----------+---------+---------+---------+---------+---------+ 1500
      TCCTGTGGGAGTACTAGAGGGCCTGGGGACTCCAGTGTACGCACCACCACCTGCACTCGG

          D  T  L  M  I  S  R  T  P  E  V  T  C  V  V  V  D  V  S  H -


          M         DM  M              RM  M
          N         DS  B              SA  N
          L         ET  D              AE  L
          1         12  2              12  1
                    /
      ACGAAGACCCTGAGGTCAAGTTCAACTGGTACGTGGACGGCGTGGAGGTGCATAATGCCA
 1501 ----------+---------+---------+---------+---------+---------+ 1560
      TGCTTCTGGGACTCCAGTTCAAGTTGACCATGCACCTGCCGCACCTCCACGTATTACGGT

          E  D  P  E  V  K  F  N  W  Y  V  D  G  V  E  V  H  N  A  K -


              F  FN
          M   N  NSS           R           M  R HNC HH
          N   U  UPA           S           A  S PCR GP
          L   4  DBC           A           E  A AIF AH
          1   H  222           1           2  1 211 11
                 //                          /
      AGACAAAGCCGCGGGAGGAGCAGTACAACAGCACGTACCGGGTGGTCAGCGTCCTCACCG
 1561 ----------+---------+---------+---------+---------+---------+ 1620
      TCTGTTTCGGCGCCCTCCTCGTCATGTTGTCGTGCATGGCCCACCAGTCGCAGGAGTGGC

          T  K  P  R  E  E  Q  Y  N  S  T  Y  R  V  V  S  V  L  T  V -
```

65

```
      BS
  M   SC                              R
  N   TR                              S
  L   NF                              A
  1   11                              1
       /
TCCTGCACCAGGACTGGCTGAATGGCAAGGAGTACAAGTGCAAGGTCTCCAACAAAGCCC
1621 ---------+---------+---------+---------+---------+--------- 1680
AGGACGTGGTCCTGACCGACTTACCGTTCCTCATGTTCACGTTCCAGAGGTTGTTTCGGG

  L   H   Q   D   W   L   N   G   K   E   Y   K   C   K   V   S   N   K   A   L  -


                                            P S                 S
          M   T                          ADNNPMA                A
          N   A                          VRLLUNU                U
          L   Q                          AAAAML9                9
          1   1                          2244116                6
                                          //// /
TCCCAGCCCCCATCGAGAAAACCATCTCCAAAGCCAAAGGTGGGGACCCGTGGGGTGCGAG
1681 ---------+---------+---------+---------+---------+--------- 1740
AGGGTCGGGGGTAGCCTCTTTTGGTAGAGGTTTCGGTTTCCACCCTGGGCACCCCACGCTC

  P   A   P   I   E   K   T   I   S   K   A   K


                          S                        N
  H M   N       HHN   BSAH           D M   M       S   R
  A N   L       APA   GFUA           D N   A       P   S
  E L   A       EAE   LI9E           E L   E       B   A
  3 1   3       321   1163           1 1   3       2   1
                 /
GGCCACATGGACAGAGGCCGGCTCGGCCCACCCTCTGCCCTGAGAGTGACCGCTGTACCA
1741 ---------+---------+---------+---------+---------+--------- 1800
CCGGTGTACCTGTCTCCGGCCGAGCCGGGTGGGAGACGGGACTCTCACTGGCGACATGGT


                  F                               SS
          M       N   A       B       R F       AHNNCC
          N       U   V       B       S 0       VPCCRR
          L       4   A       B       A K       AAIIFF
          1       H   1       V       1 1       121111
                                                 ////
ACCTCTGTCCTACAGGGCAGCCCCGAGAACCACAGGTGTACACCCTGCCCCCATCCCGGG
1801 ---------+---------+---------+---------+---------+--------- 1850
TGGAGACAGGATGTCCCGTCGGGGCTCTTGGTGTCCACATGTGGGACGGGGGTAGGGCCC


      G   Q   P   R   E   P   Q   V   Y   T   L   P   P   S   R   D  -
```

66

```
                            BS                BS B
          S    A      F     SC                SC S
          M    L      D     TR                TR P
          A    U      K     NF                NF M
          1    1      1     11                11 1
           /                 /                 /
          ATGAGCTGACCAAGAACCAGGTCAGCCTGACCTGCCTGGTCAAAGGCTTCTATCCCAGCG
1861      ---------+---------+---------+---------+---------+---------+ 1920
          TACTCGACTGGTTCTTGGTCCAGTCGGACTGGACGGACCAGTTTCCGAAGATAGGGTCGC

           E   L   T   K   N   Q   V   S   L   T   C   L   V   K   G   F   Y   P   S   D   -
                                               F
                                    N   H            B
                                    U   P            B
                                    4   A            V
                                    H   2            1
          ACATCGCCGTGGAGTGGGAGAGCAATGGGCAGCCGGAGAACAACTACAAGACCACGCCTC
1921      ---------+---------+---------+---------+---------+---------+ 1980
          TGTAGCGGCACCTCACCCTCTCGTTACCCGTCGGCCTCTTGTTGATGTTCTGGTGCGGAG

            I   A   V   E   W   E   S   N   G   Q   P   E   N   N   Y   K   T   T   P   P   -
                    H
                M   I   M       N           H       M   A       B
                N   N   B       L           P       N   L       S
                L   F   D       A           H       L   U       P
                1   1   2       4           1       1   1       M
                                                                1
          CCGTGCTGGACTCCGACGGCTCCTTCTTCCTCTACAGCAAGCTCACCGTGGACAAGAGCA
1981      ---------+---------+---------+---------+---------+---------+ 2040
          GGCACGACCTGAGGCTGCCGAGGAAGAAGGAGATGTCGTTCGAGTGGCACCTGTTCTCGT

            V   L   D   S   D   G   S   F   F   L   Y   S   K   L   T   V   D   K   S   R   -
                F                           S
                NV          MBX             NF  M           N   N
                UB          ABM             LA  N           S   L
                40          EVN             AN  L           I   A
                H2          211             31  1           1   3
                                             /
          GGTGGCAGCAGGGGAACGTCTTCTCATGCTCCGTGATGCATGAGGCTCTGCACAACCACT
2041      ---------+---------+---------+---------+---------+---------+ 2100
          CCACCGTCGTCCCCTTGCAGAAGAGTACGAGGCACTACGTACTCCGAGACGTGTTGGTGA

            W   Q   Q   G   N   V   F   S   C   S   V   M   H   E   A   L   H   N   H   Y   -
                                    S
                    M   M   HNC                     CXH
                    B   N   PCR                     FMA
                    O   L   AIF                     RAE
                    2   1   211                     133
                             /                       /
          ACACGCAGAAGAGCCTCTCCCTGTCTCCGGGTAAATGAGTGCGACGGCCG
2101      ---------+---------+---------+---------+-------+ 2150
          TGTGCGTCTTCTCGGAGAGGGACAGAGGCCCATTTACTCACGCTGCCGGC

            T   Q   K   S   L   S   L   S   P   G   K   *
```

12. An immunoglobulin-like molecule, comprising the fusion protein of claim 9 and an immunoglobulin light chain.

**13.** An immunoglobulin-like molecule of claim 12, further comprising a therapeutic agent, detectable label or NMR imaging agent linked to said immunoglobulin-like molecule.

**14.** A complex comprising the fusion protein of claim 9 or 10 and HIV or SIV gp120.

**15.** The complex of claim 14, wherein said gp120 is a part of an HIV or SIV, is expressed on the surface of an HIV or SIV-infected cell or is present in solution.

**16.** A method of using a fusion protein of claim 9 or 10 for the detection of HIV or SIV gp120 in a sample, characterized by
    (a) contacting a sample suspected of containing HIV or SIV gp120 with said fusion protein and
    (b) detecting whether a complex is formed, said fusion protein being detectably labeled.

**Claims for the following Contracting State : ES**

**1.** A method of producing a fusion protein encoded by a gene encoding a fusion protein which is capable of being secreted comprising 1) the DNA sequence of extracellular CD4, or fragment thereof which binds to HIV gp 120 when fused to an immunoglobulin chain, and 2) the DNA sequence of an immunoglobulin heavy chain, characterized in that the DNA sequence which encodes at least the variable region of said immunoglobulin has been replaced with the DNA sequence which encodes extracellular CD4, or said gp 120 binding fragment thereof, characterized by cultivating in a nutrient medium under protein-producing conditions, a host strain transformed with the vector comprising the gene of claim 1, said vector further comprising expression signals which are recognized by said host strain and direct expression of said fusion protein, and recovering the fusion protein so produced.

**2.** The method of claim 1, wherein said host strain is a myeloma cell line which produces immunoglobulin light chains and said fusion protein comprises an immunoglobulin heavy chain of the class IgM, IgG1 or IgG3, wherein an immunoglobulin-like molecule comprising said fusion protein is produced.

**3.** The methods of claim 1 or 2, wherein the vector having the identifying characteristics of pCD4H$\gamma$1, which has been deposited under Accession No. 67611, or pCD4M$\mu$, which has been deposited under Accession No. 67608, or of pCH4P$\mu$, which has been deposited under Accession No. 67609, or of pCD4E$\gamma$1, which has been deposited under Accession No. 67610, all in E. coli at the ATCC under the terms of the Budapest Treaty.

**Claims for the following Contracting State : GR**

**1.** A vector comprising a gene encoding a fusion protein which is capable of being secreted comprising 1) the DNA sequence of extracellular CD4, or fragment thereof which binds to HIV gp 120 when fused to an immunoglobulin chain, and 2) the DNA sequence of an immunoglobulin heavy chain, characterized in that the DNA sequence which encodes at least the variable region of said immunoglobulin has been replaced with the DNA sequence which encodes extracellular CD4, or said gp 120 binding fragment thereof.

**2.** A vector according to claim 1, characterized in the said immunoglobulin chain is of the class IgM, IgG1 or IgG3.

**3.** A vector having the identifying characteristics of pCD4H$\gamma$1, which has been deposited under Accession No. 67611, or pCD4M$\mu$, which has been deposited under Accession No. 67608, or of pCH4P$\mu$, which has been deposited under Accession No. 67609, or of pCD4E$\gamma$1, which has been deposited under Accession No. 67610, all in E. coli at the ATCC under the terms of the Budapest Treaty.

**4.** A host transformed with the vector of claims 1 - 3.

**5.** The host of claim 4 which expresses an immunoglobulin light chain together with the expression product of said fusion protein gene to give an immunoglobulin-like molecule which binds to gp 120.

**6.** The host of claim 5, wherein said immunoglobulin heavy chain is of the immunoglobulin class IgM, IgG1 or IgG3.

**7.** A method of producing a fusion protein encoded by a gene of a vector of claim 1, characterized by cultivating in a nutrient medium under protein-producing conditions, a host strain transformed with the vector of claim 1, said vector further comprising expression signals which are recognized by said host strain and direct expression of said fusion protein, and recovering the fusion protein so produced.

**8.** The method of claim 7, wherein said host strain is a myeloma cell line which produces immunoglobulin light chains and said fusion protein comprises an immunoglobulin heavy chain of the class IgM, IgG1 or IgG3, wherein an immunoglobulin-like molecule comprising said fusion protein is produced.

**9.** A method for the detection of HIV or SIV gp 120 in a sample, characterized by
a) contacting a sample suspected of containing HIV or SIV gp 120 with a fusion protein produced by a method according to claim 7 or 8 and
b) detecting whether a complex is formed, said fusion protein being detectably labeled.

**10.** A method according to claim 9, wherein an immunoglobulin-like molecule further comprising a therapeutic agent, detectable label or NMR imaging agent linked to said immunoglobulin-like molecule.

**Patentansprüche**
**Patentansprüche für folgende Vertragsstaaten : AT, BE, CH, DE, FR, GB, IT, LI, LU, NL, SE**

**1.** Gen, welches für ein Fusionsprotein kodiert, welches sezerniert werden kann, und welches Gen enthält: 1) die DNA-Sequenz von extrazellulärem CD4, oder eines Fragmentes hievon, welches CD4 bzw. Fragment hievon sich an das HIV gp 120 bindet, wenn dieses CD4 bzw. Fragment hievon an eine Immunglobulinkette fusioniert ist, und 2) die DNA-Sequenz einer Schweren Kette eines Immunglobulins, dadurch gekennzeichnet, daß die DNA-Sequenz, welche wenigstens für die variable Region dieses Immunglobulins kodiert, durch jene DNA-Sequenz ersetzt worden ist, welche für das extrazelluläre CD4 oder für das gp-120-bindende Fragment hievon kodiert.

**2.** Fusionsprotein-Gen nach Anspruch 1, dadurch gekennzeichnet, daß die Immunglobulinkette eine solche der Klassen IgM, IgG1 oder IgG3 ist.

**3.** Vektor, welcher das Fusionsprotein-Gen nach Anspruch 1 enthält, oder welcher die identifizierenden Kennmerkmale des pCD4Hγ1 aufweist, welches unter der Hinterlegungsnummer 67611 hinterlegt worden ist, oder welcher die identifizierenden Kennmerkmale des pCD4Mμ aufweist, welches unter der Hinterlegungsnummer 67608 hinterlegt worden ist, oder welcher die identifizierenden Kennmerkmale des pCH4Pμ aufweist, welches unter der Hinterlegungsnummer 67609 hinterlegt worden ist, oder welcher die identifizierenden Kennmerkmale des pCD4Eγ1 aufweist, welches unter der Hinterlegungsnummer 67610 hinterlegt worden ist, wobei alle diese Plasmide in E.coli bei der ATCC gemäß den Bestimmungen des Budapester Vertrages hinterlegt worden sind.

**4.** Wirtszelle bzw. Wirtsorganismus, die bzw. der mit dem Vektor nach Anspruch 3 transformiert worden ist.

**5.** Wirtszelle bzw. Wirtsorganismus nach Anspruch 4, welche bzw. welcher eine Leichte Kette eines Immunglobulins zusammen mit dem Expressionsprodukt des genannten Fusionsprotein-Gens exprimiert, wobei ein immunglobulinartiges Molekül entsteht, welches sich an das gp120 bindet.

**6.** Wirtszelle bzw. -organismus nach Anspruch 5, wobei die Schwere Kette des Immunglobulins der Immunglobulinklasse IgM, IgG1 oder IgG3 angehört.

**7.** Verfahren zur Herstellung eines Fusionsproteins, für welches ein Gen nach Anspruch 1 kodiert, dadurch gekennzeichnet, daß man in einem Nährmedium, unter die Proteinbildung fördernden Bedingungen, einen Stamm von Wirtszellen bzw. -organismen kultiviert, welche mit dem Vektor transformiert worden sind, welcher letztgenannte das Gen nach Anspruch 1 enthält, wobei dieser Vektor weiterhin Expressionssignale enthält, welche von dem genannten Stamm von Wirtszellen bzw. -organismen erkannt werden, und welche die Expression des Fusionsproteins dirigieren, und daß man das so entstandene Fusionsprotein gewinnt.

8. Verfahren nach Anspruch 7, wobei der Stamm von Wirtszellen eine Myelom-Zellinie ist, welche Leichte Ketten eines Immunglobulins produziert, und wobei das Fusionsprotein eine Schwere Kette eines Immunglobulins der Klasse IgM, IgG1 oder IgG3 enthält, und wobei ein immunglobulinartiges Molekül erzeugt wird, welches das genannte Fusionsprotein umfaßt.

9. Fusionsprotein, für welches ein Gen nach Anspruch 1 kodiert.

10. Fusionsprotein, welches weiterhin ein therapeutisches Mittel, ein nachweisbares Label oder ein der bildlichen Darstellung mit NMR dienendes Mittel an das Fusionsprotein gebunden enthält.

11. Die Fusionsproteine CD4H$\gamma$1, CD4M$\mu$, CD4P$\mu$, CD4E$\gamma$1, welche mit den Vektoren ATCC 67611, 67608, 67609 bzw. 67610 oder CD3B$\gamma$1 gemäß der folgenden Aminosäurensequenz hergestellt werden können:

```
          F N                                 S            B
          N S           B      M      H      DHA           S
          U P           B      N      G      RAU           T
          4 B           V      L      A      AE9           X
          H 2           1      1      1      236           1
                                                /
GCCTGTTTGAGAAGCAGCGGGCAAGAAAGACGCAAGCCCAGAGGCCCTGCCATTTCTGTG
1 -------------+---------+---------+---------+---------+------- 60
CGGACAAACTCTTCGTCGCCCGTTCTTTCTGCGTTCGGGTCTCCGGGACGGTAAAGACAC


       B      PS                   S                         S
     DBS ADNPA          D        DHNA            M    HM    HNC
     DAP VRLUU          D        RALU            N    AN    PCR
     EN1 AAAM9          E        AEA9            L    EL    AIF
     122 22416          1        2346            1    31    211
      /   / //                    /                  /        /
GGCTCAGGTCCCTACTGGCTCAGGCCCCTGCCTCCCTCGGCAAGGCCACAATGAACCGGG
61 ------------+---------+---------+---------+---------+------ 120
CCGAGTCCAGGGATGACCGAGTCCGGGGACGGAGGGAGCCGTTCCGGTGTTACTTGGCCC

                                             M   N   R   G -

     H                         F                     F
     I                         N                     N M    D
     N            B            U          HH         U N    D
    ·F            B            4          HA         4 L    E
     1            V            H          AE         H 1    1
                  1                       12
GAGTCCCTTTTAGGCACTTGCTTCTGGTGCTGCAACTGGCGCTCCTCCCAGCAGCCACTC
121 ----------+---------+---------+---------+---------+------ 180
CTCAGGGAAAATCCGTGAACGAAGACCACGACGTTGACCGCGAGGAGGGTCGTCGGTGAG

     V   P   F   R   H   L   L   L   V   L   Q   L   A   L   L   P   A   A   T   Q -

     B         E    E                              R      A
     B         C    C                              S      L
     V         0    0                              A      U
     1         K    K                              1      1
AGGGAAAGAAAGTGGTGCTGGGCAAAAAAGGGGATACAGTGGAACTGACCTGTACAGCTT
181 ----------+---------+---------+---------+---------+------ 240
TCCCTTTCTTTCACCACGACCCGTTTTTTCCCCTATGTCACCTTGACTGGACATGTCGAA

     G   K   K   V   V   L   G   K   K   G   D   T   V   E   L   T   C   T   A   S -

                M    M                       H
                B    B                       I
                0    0                       N
                2    2                       F
                                             1
CCCAGAAGAAGAGCATACAATTCCACTGGAAAAACTCCAACCAGATAAAGATTCTGGGAA
241 ----------+---------+---------+---------+---------+------ 300
GGGTCTTCTTCTCGTATGTTAAGGTGACCTTTTTGAGGTTGGTCTATTTCTAAGACCCTT

     Q   K   K   S   I   Q   F   H   W   K   N   S   N   Q   I   K   I   L   G   N -
```

71

```
                 B
                 NES          F          S                        A          S          F      H
                 LAP          0          AA                       L          A          N  H   I
                 AN1          K          VU                       U          U          U  H   N
                 422          1          A9                       1          3          D  A   F
                  /                      26                                  A          2  1   1
                                          /
     ATCAGGGCTCCTTCTTAACTAAAGGTCCATCCAAGCTGAATGATCGCGCTGACTCAAGAA
301  ------------------+-----------------+------------+-----------------+----------- 360
     TAGTCCCGAGGAAGAATTGATTTCCAGGTAGGTTCGACTTACTAGCGCGACTGAGTTCTT

      Q   G   S   F   L   T   K   G   P   S   K   L   N   D   R   A   D   S   R   R  -


                          S
                          MANAS                    S              H                      H
                          BVLUT                    BA             I          A           I   D
                          OAA9Y                    CU             N          F           N   D
                          22461                    L3             F          L           F   E
                            /                      1A             1          2           1   1
                                                    /
     GAAGCCTTTGGGACCAACGAAACTTCCCCCTGATCATCAAGAATCTTAAGATAGAAGACT
361  ------------------+-----------------+------------+-----------------+----------- 420  .
     CTTCGGAAACCCTGGTTCCTTTGAAGGGGGACTAGTAGTTCTTAGAATTCTATCTTCTGA

      S   L   W   D   Q   G   N   F   P   L   I   I   K   N   L   K   I   E   D   S  -


                                                    S
              M            M            AMAV                                  M
              B            N            VNUN                                  A
              0            L            AL9L                                  E
              2            1            2161                                  1
                                          //
     CAGATACTTACATCTGTGAAGTGGAGGACCAGAAGGAGGAGGTGCAATTGCTAGTGTTCG
421  ------------------+-----------------+------------+-----------------+----------- 480
     GTCTATGAATGTAGACACTTCACCTCCTGGTCTTCCTCCTCCACGTTAACGATCACAAGC

      D   T   Y   I   C   E   V   E   D   Q   K   E   E   V   Q   L   L   V   F   G  -


                                                    B
                                                    S
                                                    P                                  S
                                                    M                                  T
                                                    1                                  Y
                                                                                       1
     GATTGACTGCCAACTCTGACACCCACCTGCTTCAGGGGCAGAGCCTGACCCTGACCTTGG
481  ------------------+-----------------+------------+-----------------+----------- 540
     CTAACTGACGGTTGAGACTGTGGGTGGACGAAGTCCCCGTCTCGGACTGGGACTGGAACC

      L   T   A   N   S   D   T   H   L   L   Q   G   Q   S   L   T   L   T   L   E  -
```

```
          B   BS
          BS  SC                  D              M        H
          AP  TR                  D              N        I     S
          N1  NF                  E              L        N     T
          22  11                  1              1        F     Y
                                                          1     1
           /   /
      AGAGCCCCCCTGGTAGTAGCCCCTCAGTGCAATGTAGGAGTCCAAGGGGTAAAAACATAC
541   ------------------+-----------------+------------------+------------- 600
      TCTCGGGGGGACCATCATCGGGGAGTCACGTTACATCCTCAGGTTCCCCATTTTTGTATG
       S  P  P  G  S  S  P  S  V  Q  C  R  S  P  R  G  K  N  I  Q -


                                 N              BBH S   B              BS
                      M   MD    ASP           A BSSGSC  S   B  N       SC
                      B   ND    LPV           L APTIAR  T   A  L       TR
                      0   LE    UBU           U N1NACF  X   N  A       NF
                      2   11    122           1 221111  1   1  4       11
                                 //            / ///                 /
      AGGGGGGGGAAGACCCTCTCCGTGTCTCAGCTGGAGCTCCAGGATAGTGGCACCTGGACAT
601   ------------------+-----------------+------------------+------------- 660
      TCCCCCCCCTTCTGGGAGAGGCACAGAGTCGACCTCGAGGTCCTATCACCGTGGACCTGTA
        G  G  K  T  L  S  V  S  Q  L  E  L  Q  D  S  G  T  W  T  C -


      N
      NS                                      M                      NV   A
      LP                                      B                      HA   L
      AH                                      0                      EE   U
      31                                      2                      11   1
       /
      GCACTGTCTTGCAGAACCAGAAGAAGGTGGAGTTCAAAATAGACATCGTGGTGCTAGCTT
651   ------------------+-----------------+------------------+------------- 720
      CGTGACAGAACGTCTTGGTCTTCTTCCACCTCAAGTTTTATCTGTAGCACCACGATCGAA
        T  V  L  Q  N  Q  K  K  V  E  F  K  I  D  I  V  V  L  A  F -


              HS            M   M
              AT            N   N
              EU            L   L
              31            1   1
               /
      TCCAGAAGGCCTCCAGCATAGTCTATAAGAAAGAGGGGGGAACAGGTGGAGTTCTCCTTCC
721   ------------------+-----------------+------------------+------------- 780
      AGGTCTTCCGGAGGTCGTATCAGATATTCTTTCTCCCCCCTTGTCCACCTCAAGAGGAAGG
        Q  K  A  S  S  I  V  Y  K  K  E  G  E  Q  V  E  F  S  F  P -


                            A              A        M
                            L              L        N
                            U              U        L
                            1              1        1
      CACTCGCCTTTACAGTTGAAAAGCTGACGGGCAGTGGCGAGCTGTGGTGGCAGGCGGAGA
781   ------------------+-----------------+------------------+------------- 840
      GTGAGCGGAAATGTCAACTTTTCGACTGCCCGTCACCGCTCGACACCACCGTCCGCCTCT
        L  A  F  T  V  E  K  L  T  G  S  G  E  L  W  W  Q  A  E  R -
```

EP 0 325 262 B1

```
                  P  S
          H    N  FN A                           N
          F    N  LN U                           B
          H    L  NL 3                           0
          1    1  11 A                           2
    GGGCTTCCTCCTCCAAGTCTTGGATCACCTTTGACCTGAAGAACAAGGAAGTGTCTGTAA
841 ------------------------+-------------------+-----------------+-----------------+------------- 900
    CCCGAAGGAGGAGGTTCAGAACCTAGTGGAAACTGGACTTCTTGTTCCTTCACAGACATT

     A   S   S   S   K   S   W   I   T   F   D   L   K   N   K   E   V   S   V   K -

          B         BS   PS
          SN        SCADNPAD    A                      A H
          TA        TRVRLUUD    L                      L P
          EE        NFAAAN9E    U                      U H
          23        11224161    1                      1 1
           ♦          / / //
    AACGGGTTACCCAGGACCCTAAGCTCCAGATGGGCAAGAAGCTCCCGCTCCACCTCACCC
901 ------------------------+-------------------+-----------------+-----------------+------------- 960
    TTGCCCAATGGGTCCTGGGATTCGAGGTCTACCCGTTCTTCGAGGGCGAGGTGGAGTGGG

     R   V   T   Q   D   P   K   L   Q   M   G   K   K   L   P   L   H   L   T   L -

          BS                                          BSS
     N    SC  HS      D           M   H               SCAHN
     N    TR  AT      D           N   P               TRUAN
     L    NF  EU      E           L   H               NF9EL
     1    11  31      1           1   1               11631
           /   /                                       / /
    TGCCCCAGGCCTTGCCTCAGTATGCTGGCTCTGGAAACCTCACCCTGGCCCCTTGAAGCCGA
961 ------------------------+-------------------+-----------------+-----------------+------------- 1020
    ACGGGGTCCGGAACGGAGTCATACGACCGAGACCTTTGGAGTGGGACCGGGAACTTCGCT

     P   Q   A   L   P   Q   Y   A   G   S   G   N   L   T   L   A   L   E   A   K -

                         S           BS
                         F           SC              H D     A
                         A           TR              P D     L
                         N           NF              H E     U
                         1           11              1 1     1
                          /
    AAACAGGAAAGTTGCATCAGGAAGTGAACCTGGTGGTGATGAGAGCCACTCAGCTCCAGA
1021 ------------------------+-------------------+-----------------+-----------------+------------- 1080
    TTTGTCCTTTCAACGTAGTCCTTCACTTGGACCACCACTACTCTCGGTGAGTCGAGGTCT

     T   G   K   L   H   Q   E   V   N   L   V   V   M   R   A   T   Q   L   Q   K -
```

74

```
                              PS            S
        M                     ADNNPA        DF  AM      DE  A
        N                     VRLLUU        DA  LN      DS  L
        L                     AAAAM9        EN  UL      EP  U
        1                     224416        11  11      11  1
                              /////         /   /       /
AAAATTTGACCTGTGAGGTGTGGGGACCCACCTCCCCTAAGCTGATGCTGAGCTTGAAAC
1081 ------------------------------+-----------+-----------+---------- 1140
TTTTAAACTGGACACTCCACACCCCTGGGTGGAGGGGATTCGACTACGACTCGAACTTTG

     N  L  T  C  E  V  W  G  P  T  S  P  K  L  M  L  S  L  K  L -

     M                       T           H           M        DM
     N                       A           P           N        DS
     L                       Q           A           L        ET
     1                       1           2           1        12
                                                              /
TGGAGAACAAGGAGGCAAAGGTCTCGAAGCGGGAGAAGCCGGTGTGGGTGCTGAACCCTG
1141 ------------------------------+-----------+-----------+---------- 1200
ACCTCTTGTTCCTCCGTTTCCAGAGCTTCGCCCTCTTCGGCCACACCCACGACTTGGGAC

     E  N  K  E  A  K  V  S  K  R  E  K  P  V  W  V  L  N  P  E -

                         H                   PS          H
               F     D   M   I  A            ADPA        I
               0     D   A   N  V            VRUU        N
               K     E   E   F  A            AAM9        F
               1     1   3   1  1            2216        1
                                             ///
AGGCCGGGGATGTGGCAGTGTCTGCTGAGTGACTCGGCACAGGTCCTGCTGGAATCCAACA
1201 ------------------------------+-----------+-----------+---------- 1260
TCCGCCCCTACACCGTCACAGACGACTCACTGAGCCCTGTCCAGGACGACCTTAGGTTGT

     A  G  M  W  Q  C  L  L  S  D  S  G  Q  V  L  L  E  S  N  I -

               S           SA    BHF BS                        B
           ANA             HNCP  SGNVAANXA                     SH
           VLU             PCRA  PIUNVULHV                     PP
           AA9             AIFL  1ADLH3A0A                     1H
           236             2111  21211A421                     21
           //              //    / / / /
TCAAGGTTCTGCCCACATGGTCCACCCCGGTGCACGCGGATCCCGAGGGTGAGTGTGCCC
1261 ------------------------------+-----------+-----------+---------- 1320
AGTTCCAAGACGGGTGTACCAGGTGGGGCCACGTGCGCCTAGGGCTCCCACTCACACGGG

     K  V  L  P  T  W  S  T  P  V  H  A  D  P  E
```

```
         BS  S      ·  S          S
MF       SC  F    DHNA       HNC         A  M  M
A0       TR  A    RALU       PCR         F  A  B
EK       NF  N    AEA9       AIF         L  E  0
11       11  1    2346       211         3  2  2
/        /    /    /          /
TAGAGTAGCCTGCATCCAGGGACAGGCCCCAGCCGGGTGCTGACACGTCCACCTCCATCT
1321 ----------------•------------------•------------------•------------------ 1380
ATCTCATCGGACGTAGGTCCCTGTCCGGGGTCGGCCCACGACTGTGCAGGTGGAGGTAGA


         BS     S
M  D     M    SC  M ANA M                        M        S
N  D     N    TR  B VLU B                        N        T
L  E     L    NF  0 AA9 0                        L        Y
1  1     1    11  2 246 2                        1        1
        /      /
CTTCCTCAGCACCTGAACTCCTGGGGGGACCGTCAGTCTTCCTCTTCCCCCCCAAAACCCA
1381 ----------------•------------------•------------------•------------------ 1440
GAAGGAGTCGTGGACTTGAGGACCCCCCTGGCAGTCAGAAGGAGAAGGGGGGGTTTTGGGT

        A  P  E  L  L  G  G  P  S  V  F  L  F  P  P  K  P  K -

         S            SS              N
AN       M  HMANNAC DM  M           NS                M
UL       N  PNVCLUR DS  A           LP                A
3A       L  ALAIA9F ET  E           AH                E
A3       1  2121461 12  3           31                2
            / / // /                 /
AGGACACCCTCATGATCTCCCGGACCCCTGAGGTCACATGCGTGGTGGTGGACGTGAGCC
1441 ----------------•------------------•------------------•------------------ 1500
TCCTGTGGGAGTACTAGAGGGCCTGGGGACTCCAGTGTACGCACCACCACCTGCACTCGG

        D  T  L  M  I  S  R  T  P  E  V  T  C  V  V  V  D  V  S  H -

M             DM   M                RM   M
N             DS   B                SA   N
L             ET   0                AE   L
1             12   2                12   1
             /
ACGAAGACCCTGAGGTCAAGTTCAACTGGTACGTGGACGGCGTGGAGGTGCATAATGCCA
1501 ----------------•------------------•------------------•------------------ 1560
TGCTTCTGGGACTCCAGTTCAAGTTGACCATGCACCTGCCGCACCTCCACGTATTACGGT

        E  D  P  E  V  K  F  N  W  Y  V  D  G  V  E  V  H  N  A  K -

            F  FN
         M  N  NSS          R          M  R  HNC HH
         N  U  UPA          S          A  S  PCR GP
         L  4  DBC          A          E  A  AIF AH
         1  H  222          1          2  1  211 11
               //                                /
AGACAAAGCCGCGGGAGGAGCAGTACAACAGCACGTACCGGGTGGTCAGCGTCCTCACCG
1561 ----------------•------------------•------------------•------------------ 1620
TCTGTTTCGGCGCCCTCCTCGTCATGTTGTCGTGCATGGCCCACCAGTCGCAGGAGTGGC

        T  K  P  R  E  E  Q  Y  N  S  T  Y  R  V  V  S  V  L  T  V -
```

76

```
              BS
      M       SC                        R
      N       TR                        S
      L       NF                        A
      1       11                        1
              /
     TCCTGCACCAGGACTGGCTGAATGGCAAGGAGTACAAGTGCAAGGTCTCCAACAAAGCCC
1621 -------------------------------------------------------------- 1680
     AGGACGTGGTCCTGACCGACTTACCGTTCCTCATGTTCACGTTCCAGAGGTTGTTTCGGG

      L   H   Q   D   W   L   N   G   K   E   Y   K   C   K   V   S   N   K   A   L   -

                                                    P S                S
                  M   T                           ADNNPMA              A
                  N   A                           VRLLUNU              U
                  L   Q                           AAAAML9              G
                  1   1                           2244116              6
                                                  //// /
     TCCCAGCCCCCATCGAGAAAACCATCTCCAAAGCCAAAGGTGGGACCCGTGGGGTGCGAG
1681 -------------------------------------------------------------- 1740
     AGGGTCGGGGGTAGCTCTTTTGGTAGAGGTTTCGGTTTCCACCCTGGGCACCCCACGCTC

      P   A   P   I   E   K   T   I   S   K   A   K

                              S
                                                           N
     H M   N       HHN  BSAH        D M   M          S   R
     A N   L       APA  GFUA        D N   A          P   S
     E L   A       EAE  LI9E        E L   E          B   A
     3 1   3       321  1163        1 1   3          2   1
                        /
     GGCCACATGGACAGAGGCCGGCTCGGCCCACCCTCTGCCCTGAGAGTGACCGCTGTACCA
1741 -------------------------------------------------------------- 1800
     CCGGTGTACCTGTCTCCGGCCGAGCCGGGTGGGAGACGGGACTCTCACTGGCGACATGGT

                  F                                            SS
     M       N   A       B           R F          AHNNCC
     N       U   V       B           S 0          VPCCRR
     L       4   A       V           A K          AAIIFF
     1       H   1       1           1 1          121111
                                                  ////
     ACCTCTGTCCTACAGGGCAGCCCCCAGAACCACAGGTGTACACCCTGCCCCCATCCCGGG
1801 -------------------------------------------------------------- 1850
     TGGAGACAGGATGTCCCGTCGGGGCTCTTGGTGTCCACATGTGGGACGGGGGTAGGGCCC

              G   Q   P   R   E   P   Q   V   Y   T   L   P   P   S   R   D   -
```

```
                        BS              -     BS B       .
        S   A     F     SC                    SC S
        M   L     O     TR                    TR P
        A   U     K     NF                    NF M
        1   1     1     11                    11 1
                 /            /                      /
        ATGAGCTGACCAAGAACCAGGTCAGCCTGACCTGCCTGGTCAAAGGCTTCTATCCCAGCG
   1861 -----------+---------+---------+---------+---------+---------- 1920
        TACTCGACTGGTTCTTGGTCCAGTCGGACTGGACGGACCAGTTTCCGAAGATAGGGTCGC

        E   L   T   K   N   Q   V   S   L   T   C   L   V   K   G   F   Y   P   S   D   -
                                        F
                            N   H       B
                            U   P       B
                            4   A       V
                            H   2       1
        ACATCGCCGTGGAGTGGGAGAGCAATGGGCAGCCGGAGAACAACTACAAGACCACGCCTC
   1921 -----------+---------+---------+---------+---------+---------- 1980
        TGTAGCGGCACCTCACCCTCTCGTTACCCGTCGGCCTCTTGTTGATGTTCTGGTGCGGAG

        I   A   V   E   W   E   S   N   G   Q   P   E   N   N   Y   K   T   T   P   P   -
                    H
            M   I   M           N           H           M   A           B
            N   N   B           L           P           N   L           S
            L   F   O           A           H           L   U           P
            1   1   2           4           1           1   1           W
                                                                        1
        CCGTGCTGGACTCCGACGGCTCCTTCTTCCTCTACAGCAAGCTCACCGTGGACAAGAGCA
   1981 -----------+---------+---------+---------+---------+---------- 2040
        GGCACGACCTGAGGCTGCCGAGGAAGAAGGAGATGTCGTTCGAGTGGCACCTGTTCTCGT

        V   L   D   S   D   G   S   F   F   L   Y   S   K   L   T   V   D   K   S   R   -
            F
            N   V               V   E   X           S
            U   B               A   B   V           NF  M       N   N
            4   0               E   V   N           LA  N.      S   L
            H   2               2   1   1           AN  L       I   A
                                                    31  1       1   3
                                     /
        GGTGGCAGCAGGGGAACGTCTTCTCATGCTCCGTGATGCATGAGGCTCTGCACAACCACT
   2041 -----------+---------+---------+---------+---------+---------- 2100
        CCACCGTCGTCCCCTTGCAGAAGAGTACGAGGCACTACGTACTCCGAGACGTGTTGGTGA

        W   Q   Q   G   N   V   F   S   C   S   V   M   H   E   A   L   H   N   H   Y   -
                                    S
                            M   M   HNC                     CXH
                            B   N   PCR                     FMA
                            O   L   AIF                     RAE
                            2   1   211                     133
                                     /                       /
        ACACGCAGAAGAGCCTCTCCCTGTCTCCGGGTAAATGAGTGCGACGGCCG
   2101 -----------+---------+---------+---------+-------------- 2150
        TGTGCGTCTTCTCGGAGAGGGACAGAGGCCCATTTACTCACGCTGCCGGC

        T   Q   K   S   L   S   L   S   P   G   K   *
```

**12.** Immunglobulinartiges Molekül, welches das Fusionsprotein nach Anspruch 9 und eine Leichte Kette eines Immunglobulins enthält.

**13.** Immunglobulinartiges Molekül nach Anspruch 12, welches weiterhin ein therapeutisches Mittel, ein nachweisbares Label oder ein der bildlichen Darstellung mit NMR dienendes Mittel an das immunglobulinartige Molekül gebunden enthält.

**14.** Komplex, welcher das Fusionsprotein nach Anspruch 9 oder 10 und das HIV- oder SIV-gp120 enthält.

**15.** Komplex nach Anspruch 14, wobei das genannte gp120 einen Teil eines HIV oder SIV bildet, auf der Oberfläche einer mit HIV oder SIV infizierten Zelle exprimiert wird oder in Lösung vorliegt.

**16.** Verfahren zur Verwendung eines Fusionsproteins nach Anspruch 9 oder 10 für den Nachweis von HIV- oder SIV-gp120 in einer Probe, dadurch gekennzeichnet, daß man:

(a) eine Probe, welche unter Verdacht steht, daß sie das HIV- oder SIV-gp120 enthält, mit dem Fusionsprotein in Berührung bringt; und

(b) feststellt, ob sich ein Komplex bildet, wobei das genannte Fusionsprotein nachweisbar markiert ist.

**Patentansprüche für folgenden Vertragsstaat : ES**

**1.** Verfahren zur Herstellung eines Fusionsproteins, für welches ein Gen kodiert, welches Gen für ein Fusionsprotein kodiert, welches sezerniert werden kann, und welches Gen enthält: 1) die DNA-Sequenz von extrazellulärem CD4, oder eines Fragmentes hievon, welches CD4 bzw. Fragment hievon sich an das HIV gp 120 bindet, wenn dieses CD4 bzw. Fragment hievon an eine Immunglobulinkette fusioniert ist, und 2) die DNA-Sequenz einer Schweren Kette eines Immunglobulins, dadurch gekennzeichnet, daß die DNA-Sequenz, welche wenigstens für die variable Region dieses Immunglobulins kodiert, durch jene DNA-Sequenz ersetzt worden ist, welche für das extrazelluläre CD4 oder für das gp-120-bindende Fragment hievon kodiert, dadurch gekennzeichnet, daß man in einem Nährmedium, unter die Proteinbildung fördernden Bedingungen, einen Stamm von Wirtszellen bzw. -organismen kultiviert, welche mit dem Vektor transformiert worden sind, welcher letztgenannte das Gen nach Anspruch 1 enthält, wobei dieser Vektor weiterhin Expressionssignale enthält, welche von dem genannten Stamm von Wirtszellen bzw. -organismen erkannt werden, und welche die Expression des Fusionsproteins dirigieren, und daß man das so entstandene Fusionsprotein zurückgewinnt.

**2.** Verfahren nach Anspruch 1, wobei der Stamm von Wirtszellen eine Myelom-Zellinie ist, welche Leichte Ketten eines Immunglobulins produziert, und wobei das Fusionsprotein eine Schwere Kette eines Immunglobulins der Klasse IgM, IgG1 oder IgG3 enthält, und wobei ein immunglobulinartiges Molekül erzeugt wird, welches das genannte Fusionsprotein enthält.

**3.** Verfahren nach Anspruch 1 oder 2, wobei der Vektor die identifizierenden Kennmerkmale des pCD4Hγ1 aufweist, welches unter der Hinterlegungsnummer 67611 hinterlegt worden ist, oder welcher die identifizierenden Kennmerkmale des pCD4Mμ aufweist, welches unter der Hinterlegungsnummer 67608 hinterlegt worden ist, oder welcher die identifizierenden Kennmerkmale des pCH4Pμ aufweist, welches unter der Hinterlegungsnummer 67609 hinterlegt worden ist, oder welcher die identifizierenden Kennmerkmale des pCD4Eγ1 aufweist, welches unter der Hinterlegungsnummer 67610 hinterlegt worden ist, wobei alle diese Plasmide in E.coli bei der ATCC gemäß den Bestimmungen des Budapester Vertrages hinterlegt worden sind.

**Patentansprüche für folgenden Vertragsstaat : GR**

**1.** Vektor, welcher ein Gen enthält, welches für ein Fusionsprotein kodiert, welches sezerniert werden kann, und welches Gen enthält: 1) die DNA-Sequenz von extrazellulärem CD4, oder eines Fragmentes hievon, welches CD4 bzw. Fragment hievon sich an das HIV gp 120 bindet, wenn dieses CD4 bzw. Fragment hievon an eine Immunglobulinkette fusioniert ist, und 2) die DNA-Sequenz einer Schweren Kette eines Immunglobulins, dadurch gekennzeichnet, daß die DNA-Sequenz, welche wenigstens für die variable Region dieses Immunglobulins kodiert, durch jene DNA-Sequenz ersetzt worden ist, welche für das extrazelluläre CD4 oder für das gp-120-bindende Fragment hievon kodiert.

**2.** Vektor nach Anspruch 1, dadurch gekennzeichnet, daß die Immunglobulinkette eine solche der Klassen IgM, IgG1 oder IgG3 ist.

**3.** Vektor, welcher die identifizierenden Kennmerkmale des pCD4Hγ1 aufweist, welches unter der Hinterlegungsnummer 67611 hinterlegt worden ist, oder welcher die identifizierenden Kennmerkmale des pCD4Mμ aufweist, welches unter der Hinterlegungsnummer 67608 hinterlegt worden ist, oder welcher die identifizierenden Kennmerkmale des pCH4Pμ aufweist, welches unter der Hinterlegungsnummer 67609 hinterlegt worden ist, oder welcher die identifizierenden Kennmerkmale des pCD4Eγ1 aufweist, welches unter der Hinterlegungsnummer 67610 hinterlegt worden ist, wobei alle diese Plasmide in E.coli bei der ATCC gemäß den Bestimmungen des Budapester Vertrages hinterlegt worden sind.

**4.** Wirtszelle bzw. Wirtsorganismus, die bzw. der mit dem Vektor nach den Ansprüchen 1 bis 3 transformiert worden ist.

**5.** Wirtszelle bzw. Wirtsorganismus nach Anspruch 4, welche bzw. welcher eine Leichte Kette eines Immunglobulins zusammen mit dem Expressionsprodukt des genannten Fusionsprotein-Gens exprimiert, wobei ein immunglobulinartiges Molekül entsteht, welches sich an das gp120 bindet.

**6.** Wirtszelle bzw. Wirtsorganismus nach Anspruch 5, wobei die Schwere Kette des Immunglobulins der Immunglobulinklasse IgM, IgG1 oder IgG3 angehört.

**7.** Verfahren zur Herstellung eines Fusionsproteins, für welches ein Gen eines Vektors nach Anspruch 1 kodiert, dadurch gekennzeichnet, daß man in einem Nährmedium, unter die Proteinbildung fördernden Bedingungen, einen Stamm von Wirtszellen bzw. -organismen kultiviert, welche mit dem Vektor nach Anspruch 1 transformiert worden sind, wobei dieser Vektor weiterhin Expressionssignale enthält, welche von dem genannten Stamm von Wirtszellen bzw. -organismen erkannt werden, und welche die Expression des Fusionsproteins dirigieren, und daß man das so entstandene Fusionsprotein gewinnt.

**8.** Verfahren nach Anspruch 7, wobei der Stamm von Wirtszellen eine Myelom-Zellinie ist, welche Leichte Ketten eines Immunglobulins produziert, und wobei das Fusionsprotein eine Schwere Kette eines Immunglobulins der Klasse IgM, IgG1 oder IgG3 enthält, und wobei ein immunglobulinartiges Molekül erzeugt wird, welches das genannte Fusionsprotein umfaßt.

**9.** Verfahren zum Nachweisen von HIV- oder SIV-gp120 in einer Probe, dadurch gekennzeichnet, daß man:
(a) eine Probe, welche unter Verdacht steht, daß sie das HIV- oder SIV-gp120 enthält, mit einem Fusionsprotein in Berührung bringt, welches nach einem Verfahren nach Anspruch 7 oder 8 erzeugt worden ist, und daß man
(b) feststellt, ob sich ein Komplex bildet, wobei das genannte Fusionsprotein nachweisbar markiert ist.

**10.** Verfahren nach Anspruch 9, wobei ein immunglobulinartiges Molekül ferner ein therapeutisches Agens, eine nachweisbare Markierung oder ein NMR abbildendes Agens enthält, welches an das genannte immunglobulinartige Molekül gebunden ist.

**Revendications**
**Revendications pour les Etats contractants suivants : AT, BE, CH, DE, FR, GB, IT, LI, LU, NL, SE**

**1.** Gène codant pour une protéine de fusion qui est apte à être sécrétée, comprenant 1) la séquence d'ADN de CD4 extracellulaire, ou d'un fragment de CD4 qui se lie à gp120 de HIV lorsqu'elle est fusionnée à une chaîne d'immunoglobuline, et 2) la séquence d'ADN d'une chaîne lourde d'immunoglobuline, caractérisé en ce que la séquence d'ADN qui code **pour au** moins la région variable de ladite immunoglobuline a été remplacée par la séquence d'ADN qui code pour la protéine CD4 extracellulaire, ou ledit fragment de celle-ci qui se lie à gp120.

**2.** Protéine de fusion selon la revendication 1, caractérisée en ce que ladite chaîne d'immunoglobuline appartient à la classe IgM, IgG1 ou IgG3.

**3.** Vecteur comprenant le gène de protéine de fusion de la revendication 1, ou ayant les caractéristiques d'identification de pCD4Hγ1, qui a été déposé sous le n° 67611, ou de pCD4Mμ, qui a été déposé sous le n° 67608, ou de pCH4Pμ, qui a été déposé sous le n° 67609, ou de pCD4Eγ1, qui a été

déposé sous le n° 67610, tous dans *E. coli*, à l'ATCC, sous les clauses du Traité de Budapest.

**4.** Hôte transformé par le vecteur de la revendication 3.

**5.** Hôte de la revendication 4, qui exprime une chaîne légère d'immunoglobuline conjointement avec le produit d'expression dudit gène de protéine de fusion, pour donner une molécule de type immunoglobuline qui se lie à gp120.

**6.** Hôte de la revendication 5, dans lequel ladite chaîne lourde d'immunoglobuline appartient à la classe d'immunoglobulines IgM, IgG1 ou IgG3.

**7.** Procédé de production d'une protéine de fusion codée par un gène de la revendication 1, caractérisé par la culture, dans un milieu nutritif, dans des conditions productrices de protéines, d'une souche hôte transformée par le vecteur comprenant le gène de la revendication 1, ledit vecteur comprenant en outre des signaux d'expression qui sont reconnus par ladite souche hôte et dirigent l'expression de ladite protéine de fusion, et la récupération de la protéine de fusion ainsi produite.

**8.** Procédé de la revendication 7, dans lequel ladite souche hôte est une lignée cellulaire de myélome qui produit des chaînes légères d'immunoglobulines, et ladite protéine de fusion comprend une chaîne lourde d'immunoglobuline appartenant à la classe IgM, IgG1 ou IgG3, et dans lequel est produite une molécule de type immunoglobuline comprenant ladite protéine de fusion.

**9.** Protéine de fusion codée par un gène selon la revendication 1.

**10.** Protéine de fusion de la revendication 9, qui en outre comprend un agent thérapeutique, un marqueur détectable, ou un agent d'imagerie de RMN, liés à ladite protéine de fusion.

**11.** Protéines de fusion CD4H$\gamma$1, CD4M$\mu$, CD4P$\mu$, CD4E$\gamma$1, pouvant être produites par les vecteurs ATCC 67611, 67608, 67609 et 67610, respectivement, ou CD3B$\gamma$1 conformément à la séquence d'aminoacides suivante

```
                    F N                           S                    B
                    N S           B     M     H   DHA                  S
                    U P           B     N     C   RAU                  T
                    4 B           V     L     A   AE9                  X
                    H 2           1     1     1   236                  1
                                                  /
     GCCTGTTTGAGAAGCAGCGGGCAAGAAAGACGCAAGCCCAGAGGCCCTGCCATTTCTGTG
   1 -------------+---------+---------+---------+---------+---------+ 60
     CGGACAAACTCTTCGTCGCCCGTTCTTTCTGCGTTCGGGTCTCCGGGACGGTAAAGACAC

         B    PS                    S                                S
         DBS ADNPA       D    DHNA                M    HM            HNC
         DAP VRLUU       D    RALU                N    AN            PCR
         EN1 AAAV9       E    AEA9                L    EL            AIF
         122 22416       1    2346                1    31            211
          /   / //                  /                  /              /
     GGCTCAGGTCCCTACTGGCTCAGGCCCCTGCCTCCCTCGGCAAGGCCACAATGAACCGGG
  61 -------------+---------+---------+---------+---------+---------+ 120
     CCGAGTCCAGGGATGACCGAGTCCGGGGACGGAGGGAGCCGTTCCGGTGTTACTTGGCCC

                                                          M   N   R   G -
     H                           F                        F
     I              B            N            HH          N  M    D
     N              B            U            HA          U  N    D
     F              V            4            AE          4  L    E
     1              1            H            12          H  1    1
     GAGTCCCTTTTAGGCACTTGCTTCTGGTGCTGCAACTGGCGCTCCTCCCAGCAGCCACTC
 121 -------------+---------+---------+---------+---------+---------+ 180
     CTCAGGGAAAATCCGTGAACGAAGACCACGACGTTGACCGCGAGGAGGGTCGTCGGTGAG

        V   P   F   R   H   L   L   L   V   L   Q   L   A   L   L   P   A   A   T   Q -
            B        E   E                                    R       A
            B        C   C                                    S       L
            V        0   0                                    A       U
            1        K   K                                    1       1
     AGGGAAAGAAAGTGGTGCTGGGCAAAAAAGGGGATACAGTGGAACTGACCTGTACAGCTT
 181 -------------+---------+---------+---------+---------+---------+ 240
     TCCCTTTCTTTCACCACGACCCGTTTTTTCCCCTATGTCACCTTGACTGGACATGTCGAA

        G   K   K   V   V   L   G   K   K   G   D   T   V   E   L   T   C   T   A   S -
                           M   M                                H
                           B   B                                I
                           0   0                                N
                           2   2                                F
                                                               1
     CCCAGAAGAAGAGCATACAATTCCACTGGAAAAACTCCAACCAGATAAAGATTCTGGGA
 241 -------------+---------+---------+---------+---------+---------+ 300
     GGGTCTTCTTCTCGTATGTTAAGGTGACCTTTTTGAGGTTGGTCTATTTCTAAGACCCTT

        Q   K   K   S   I   Q   F   H   W   K   N   S   N   Q   I   K   I   L   G   N -
```

```
                E                      S                  S   F   H
               NES         F          AA        A        A   N   H   I
               LAP         0          VU        L        U   U   H   N
               AN1         K          A9        U        3   D   H   F
               422         1          26        1        A   2   1   1
                /                      /
     ATCAGGGCTCCTTCTTAACTAAAGGTCCATCCAAGCTGAATGATCGCGCTGACTCAAGAA
301  --------------+--------------+--------------+--------------+----------  360
     TAGTCCCGAGGAAGAATTGATTTCCAGGTAGGTTCGACTTACTAGCGCGACTGAGTTCTT

       Q  G  S  F  L  T  K  G  P  S  K  L  N  D  R  A  D  S  R  R

                      S                      S         H            H
                    MANAS                   BA         I      A      I  D
                    BVLUT                   CU         N      F      N  D
                    0AA9Y                   L3         F      L      F  E
                    22461                   1A         1      2      1  1
                      /                      /
     GAAGCCTTTGGGACCAAGGAAACTTCCCCCTGATCATCAAGAATCTTAAGATAGAAGACT
361  --------------+--------------+--------------+--------------+----------  420
     CTTCGGAAACCCTGGTTCCTTTGAAGGGGGACTAGTAGTTCTTAGAATTCTATCTTCTGA

       S  L  W  D  Q  G  N  F  P  L  I  I  K  N  L  K  I  E  D  S  -

                                              S
                 M          M               AMAV                 M
                 B          N               VNUN                 A
                 0          L               AL9L                 E
                 2          1               2161                 1
                                             //
     CAGATACTTACATCTGTGAAGTGGAGGACCAGAAGGAGGAGGTGCAATTGCTAGTGTTCG
421  --------------+--------------+--------------+--------------+----------  480
     GTCTATGAATGTAGACACTTCACCTCCTGGTCTTCCTCCTCCACGTTAACGATCACAAGC

       D  T  Y  I  C  E  V  E  D  Q  K  E  E  V  Q  L  L  V  F  G  -

                                     B
                                     S
                                     P                         S
                                     M                         T
                                     1                         Y
                                                               1
     GATTGACTGCCAACTCTGACACCCACCTGCTTCAGGGGCAGAGCCTGACCCTGACCTTGG
481  --------------+--------------+--------------+--------------+----------  540
     CTAACTGACGGTTGAGACTGTGGGTGGACGAAGTCCCCGTCTCGGACTGGGACTGGAACC

       L  T  A  N  S  D  T  H  L  L  Q  G  Q  S  L  T  L  T  L  E  -
```

83

```
            B    BS                                        H
            BS   SC                              M         I         S
            AP   TR               D              N         N         T
            N1   NF               D                        F         Y
            22   11               E              L         1         1
             /    /               1              1
        AGACCCCCCCTGGTAGTAGCCCCTCAGTGCAATGTAGGAGTCCAAGGGGTAAAAACATAC
   541  ----------------+---------------+---------------+----------------+  600
        TCTCGGGGGGGACCATCATCGGGGAGTCACGTTACATCCTCAGGTTCCCCATTTTTGTATG
          S  P  P  G  S  S  P  S  V  Q  C  R  S  P  R  G  K  N  I  Q  -

                             N              BBH S·  B              BS
             M    MD          ASP           A BSSGSC  S   B  N       SC
             B    ND          LPV           L APTIAR  T   A  L       TR
             O    LE          UBU           U N1NACF  X   A  N       NF
             2    11          122           1 221111  1   1  4       11
                              //             /  ///                   /
        AGGGGGGGAAGACCCTCTCCGTGTCTCAGCTGGAGCTCCAGGATAGTGGCACCTGGACAT
   601  ----------------+---------------+---------------+----------------+  660
        TCCCCCCCTTCTGGGAGAGGCACAGAGTCGACCTCGAGGTCCTATCACCGTGGACCTGTA
          G  G  K  T  L  S  V  S  Q  L  E  L  Q  D  S  G  T  W  T  C  -

             N
             NS
             LP                             M                    NV     A
             AH                             B                    HA     L
             31                             O                    EE     U
              /                             2                    11     1
        GCACTGTCTTGCAGAACCAGAAGAACGTGGAGTTCAAAATAGACATCGTGGTGCTAGCTT
   661  ----------------+---------------+---------------+----------------+  720
        CGTGACAGAACGTCTTGGTCTTCTTCCACCTCAAGTTTTATCTGTAGCACCACGATCGAA
          T  V  L  Q  N  Q  K  K  V  E  F  K  I  D  I  V  V  L  A  F  -

             HS             M  M
             AT             N  N
             EU             L  L
             31             1  1
              /
        TCCAGAAGGCCCTCCAGCATAGTCTATAAGAAAGAGGGGGAACAGGTGGAGTTCTCCTTCC
   721  ----------------+---------------+---------------+----------------+  780
        AGGTCTTCCGGAGGTCGTATCAGATATTCTTTCTCCCCCTTGTCCACCTCAAGAGGAAGG
          Q  K  A  S  S  I  V  Y  K  K  E  G  E  Q  V  E  F  S  F  P  -

                            A                       A           M
                            L                       L           N
                            U                       U           L
                            1                       1           1
        CACTCGCCTTTACAGTTGAAAAGCTGACGGGCAGTGGCGAGCTGTGGTGGCAGGCGGAGA
   781  ----------------+---------------+---------------+----------------+  840
        GTGAGCGGAAATGTCAACTTTTCGACTGCCCGTCACCGCTCGACACCACCGTCCGCCTCT
          L  A  F  T  V  E  K  L  T  G  S  G  E  L  W  W  Q  A  E  R  -
```

```
                      P   S
               H   M  FN  A
               F      N LN U                              M
               H      L ML 3                              B
               1      1 11 A                              D
                                                          2
     CGGCTTCCTCCTCCAAGTCTTGGATCACCTTTGACCTGAAGAACAAGGAAGTGTCTGTAA
841  ------------+------------+------------+------------+------------  900
     CCCGAAGGAGGAGGTTCAGAACCTAGTGGAAACTGGACTTCTTGTTCCTTCACAGACATT

      A  S  S  S  K  S  W  I  T  F  D  L  K  N  K  E  V  S  V  K -


               B        BS    PS
               SN       SCADNPAD   A                  A H
               TA       TRVRLUUD   L                  L P
               EE       NFAAAM9E   U                  U H
               23       11224161   1                  1 1
                ↓        / / //
     AACGGGTTACCCAGGACCCTAAGCTCCAGATGGGCAAGAAGCTCCCGCTCCACCTCACCC
901  ------------+------------+------------+------------+------------  960
     TTGCCCAATGGGTCCTGGGATTCGAGGTCTACCCGTTCTTCGAGGGCGAGGTGGAGTGGG

      R  V  T  Q  D  P  K  L  Q  M  G  K  K  L  P  L  H  L  T  L -


                 BS
               M  SC HS     D        M  H             BSS
               N  TR AT     D        N  P             SCAHM
               L  NF EU     E        L  H             TRUAN
               1  11 31     1        1  1             NF9EL
                                                      11631
                /  /                                   / /
     TGCCCCAGGCCTTGCCTCAGTATCCTGGCTCTGGAAACCTCACCCTGGCCCCTTGAAGCGA
961  ------------+------------+------------+------------+------------  1020
     ACGGGGTCCGGAACGGAGTCATACGACCGAGACCTTTGGAGTGGGACCGGGAACTTCGCT

      P  Q  A  L  P  Q  Y  A  G  S  G  N  L  T  L  A  L  E  A  K -


                      S        BS
                      F        SC             H D    A
                      A        TR             P D    L
                      N        NF             H E    U
                      1        11             1 1    1
                                /
     AAACAGGAAAGTTGCATCAGGAACTGAACCTGGTGGTGATGAGAGCCACTCAGCTCCAGA
1021 ------------+------------+------------+------------+------------  1080
     TTTGTCCTTTCAACGTAGTCCTTGACTTGGACCACCACTACTCTCGGTGAGTCGAGGTCT

      T  G  K  L  H  Q  E  V  N  L  V  V  M  R  A  T  Q  L  Q  K -
```

```
                           PS            S
        M                  ADNNPA        DF  AM      DE  A
        N                  VRLLUU        DA  LN      DS  L
        L                  AAAAM9        EN  UL      EP  U
        1                  224416        11  11      11  1
                           //////        /    /       /
        AAAATTTGACCTGTGAGGTGTGGGGACCCACCTCCCCTAAGCTGATGCTGAGCTTGAAAC
1081    ------------------------------------------------------------  1140
        TTTTAAACTGGACACTCCACACCCCTGGGTGGAGGGGATTCGACTACGACTCGAACTTTG

          N  L  T  C  E  V  W  G  P  T  S  P  K  L  M  L  S  L  K  L -

        M                     T           H           M           DM
        N                     A           P           N           DS
        L                     Q           A           L           ET
        1                     1           2           1           12
                                                                   /
        TGGAGAACAAGGAGGCAAAGGTCTCGAAGCGGGAGAAGCCGGTGTGGGTGCTGAACCCTG
1141    ------------------------------------------------------------  1200
        ACCTCTTGTTCCTCCGTTTCCAGAGCTTCGCCCTCTTCGGCCACACCCACGACTTGGGAC

          E  N  K  E  A  K  V  S  K  R  E  K  P  V  W  V  L  N  P  E -

                             H                   PS          H
                  F    D  M  I  A              ADPA          I
                  0    D  A  N  V              VRUU          N
                  K    E  E  F  A              AAM9          F
                  1    1  3  1  1              2216          1
                                               ///
        AGGCCGGGGATGTGGCCAGTGTCTGCTGAGTGACTCGGGCACAGGTCCTGCTGGAATCCAACA
1201    ------------------------------------------------------------  1250
        TCCGGCCCCTACACCGTCACAGACGACTCACTGAGCCCTGTCCAGGACGACCTTAGGTTGT

          A  G  M  W  Q  C  L  L  S  D  S  G  Q  V  L  L  E  S  N  I -

                     S          SA    BHF BS                     B
                  ANA        HNCP   SGNWAANXA                    SH
                  VLU        PCRA   PIUNWJLHV                    PP
                  AA9        AIFL   1ADLH3A0A                    1H
                  236        2111   21211A421                    21
                   //          //     / / / /
        TCAAGGTTCTGCCCACATGGTCCACCCCGGTGCACGCGGATCCCGAGGGTGAGTGTGCCC
1261    ------------------------------------------------------------  1320
        AGTTCCAAGACGGGTGTACCAGGTGGGGCCACGTGCGCCTAGGGCTCCCACTCACACGGG

          K  V  L  P  T  W  S  T  P  V  H  A  D  P  E
```

```
                    BS  S        S          S
      MF            SC  F   DHNA  HNC      A M M
      AO            TR  A   RALU  PCR      F A B
      EK            NF  N   AEA9  AIF      L E O
      11            11  1   2346  211      3 2 2
       /             /         /     /
      TAGAGTAGCCTGCATCCAGGGACAGGCCCCAGCCGGGTGCTGACACGTCCACCTCCATCT
1321  ------------------------+--------------+-------------+------ 1360
      ATCTCATCGGACGTAGGTCCCTGTCCGGGGGTCGGCCCACGACTGTGCAGGTGGAGGTAGA


      M  D         M      BS     S
      N  D         N      SC  M ANA M                     M        S
      L  E         L      TR  B VLU B                     N        T
      1  1         1      NF  0 AA9 0                     L        Y
                          11  2 246 2                     1        1
                           /       /
      CTTCCTCAGCACCTGAACTCCTGGGGGGGACCGTCAGTCTTCCTCTTCCCCCCCAAAACCCA
1361  ------------------------+--------------+-------------+------ 1440
      GAAGGAGTCGTGGACTTGAGGACCCCCCCTGGCAGTCAGAAGGAGAAGGGGGGTTTTGGGT

          A  P  E  L  L  G  G  P  S  V  F  L  F  P  P  K  P  K  -

               S           SS
              AN    M  HMANNAC DM   M        N
              UL    N  PNVCLUR DS   A        NS             M
              3A    L  ALAIA9F ET   E        LP             A
              A3    1  2121461 12   3        AH             E
                                             31             2
                      /  /  //   /             /
      ACGACACCCTCATGATCTCCCGGACCCCTGAGGTCACATGCGTGGTGGTGGACGTGAGCC
1441  ------------------------+--------------+-------------+------ 1500
      TCCTGTGGGAGTACTAGAGGGCCTGGGGACTCCAGTGTACGCACCACCACCTGCACTCGG

          D  T  L  M  I  S  R  T  P  E  V  T  C  V  V  V  D  V  S  H  -

              M          DM   M            RM   M
              N          DS   B            SA   N
              L          ET   O            AE   L
              1          12   2            12   1
                          /
      ACGAAGACCCTGAGGTCAAGTTCAACTGGTACGTGGACGGCGTGGAGGTGCATAATGCCA
1501  ------------------------+--------------+-------------+------ 1560
      TGCTTCTGGGACTCCAGTTCAAGTTGACCATGCACCTGCCGCACCTCCACGTATTACGGT

          E  D  P  E  V  K  F  N  W  Y  V  D  G  V  E  V  H  N  A  K  -

                   F FN                       S
              M    N NSS           R        M  R HNC HH
              N    U UPA           S        A  S PCR GP
              L    4 DBC           A        E  A AIF AH
              1    H 222           1        2  1 211 11
                     //                        /
      AGACAAAGCCGCGGGAGGAGCAGTACAACAGCACGTACCGGGTGGTCAGCGTCCTCACCG
1561  ------------------------+--------------+-------------+------ 162
      TCTGTTTCGGCGCCCTCCTCGTCATGTTGTCGTGCATGGCCCACCAGTCGCAGGAGTGGC

          T  K  P  R  E  E  Q  Y  N  S  T  Y  R  V  V  S  V  L  T  V  -
```

```
                        BS
             M          SC                              R
             N          TR                              S
             L          NF                              A
             1          11                              1
                         /
        TCCTGCACCAGGACTGGCTGAATGGCAACGAGTACAAGTGCAAGGTCTCCAACAAAGCCC
  1621  -------------•-------------•-------------•-------------•------------•  1680
        AGGACGTGGTCCTGACCGACTTACCGTTCCTCATGTTCACGTTCCAGAGGTTGTTTCGGG

          L   H   Q   D   W   L   N   G   K   E   Y   K   C   K   V   S   N   K   A   L  -

                         M     T                            P S                S
                         N     A                         ADNNPMA               A
                         L     Q                         VRLLUNU               U
                         1     1                         AAAAML9               9
                                                         2244116               6
                                                          //// /
        TCCCAGCCCCCATCGAGAAAACCATCTCCAAAGCCAAAGGTGGGACCCCGTGGGGTGCGAG
  1681  -------------•-------------•-------------•-------------•------------•  1740
        AGGGTCGGGGGTAGCTCTTTTTGGTAGAGGTTTCGGTTTCCACCCTGGGCACCCCACGCTC

          P   A   P   I   E   K   T   I   S   K   A   K

                                           S
            H M      N        HHN      BSAH         D M   M        N
            A N      L        APA      GFUA         D N   A        S   R
            E L      A        EAE      LI9E         E L   E        P   S
            3 1      3        321      1163         1 1   3        B   A
                                                                  2   1
                              /
        GGCCACATGGACAGAGGCCGGCTCGGCCCACCCTCTGCCCTGAGAGTGACCGCTGTACCA
  1741  -------------•-------------•-------------•-------------•------------•  1800
        CCGGTGTACCTGTCTCCGGCCGAGCCGGGTGGGAGACGGGACTCTCACTGGCGACATGGT

                         F                                            SS
            M        N        A        B        R F             AHNNCC
            N        U        V        B        S D             VPCCRR
            L        4        A        V  .      A K             AAIIFF
            1        H        1        1        1 1             121111
                                                                ////
        ACCTCTGTCCTACAGGGCAGCCCCGAGAACCACACGGTGTACACCCTGCCCCCATCCCGGG
  1801  -------------•-------------•-------------•-------------•------------•  1850
        TGGAGACAGGATGTCCCGTCGGGGCTCTTGGTGTCCACATGTGGGACGGGGGTAGGGCCC

              G   Q   P   R   E   P   Q   V   Y   T   L   P   P   S   R   D  -
```

```
              BS              BS B
  S   A    F  SC              SC S
  M   L    O  TR              TR P
  A   U    K  NF              NF M
  1   1    1  11              11 1
  /         /                 /
  ATGAGCTGACCAACAACCAGGTCAGCCTGACCTGCCTGGTCAAAGGCTTCTATCCCAGCG
1861 --------------------+--------------------+---------+---------- 1920
  TACTCGACTGGTTCTTGGTCCAGTCGGACTGGACGGACCAGTTTCCGAAGATAGGGTCGC

    E  L  T  K  N  Q  V  S  L  T  C  L  V  K  G  F  Y  P  S  D  -
                          F
                          N  H        B
                          U  P        B
                          4  A        V
                          H  2        1
  ACATCGCCGTGGAGTGGGAGAGCAATGGGCAGCCGGAGAACAACTACAAGACCACGCCTC
1921 --------------------+--------------------+---------+---------- 1980
  TGTAGCGGCACCTCACCCTCTCGTTACCCGTCGGCCTCTTGTTGATGTTCTGGTGCGGAG

    I  A  V  E  A  E  S  N  G  Q  P  E  N  N  Y  K  T  T  P  P  -
           H                                          B
     M  I   M       N           H        M  A         S
     N  N   B       L           P        N  L         P
     L  F   O       A           H        L  U         M
     1  1   2       4           1        1  1         1
  CCGTGCTGGACTCCGACGGCTCCTTCTTCCTCTACAGCAAGCTCACCGTGGACAAGAGCA
1981 --------------------+--------------------+---------+---------- 2040
  GGCACGACCTGAGGCTGCCGAGGAAGAAGGAGATGTCGTTCGAGTGGCACCTGTTCTCGT

    V  L  D  S  D  G  S  F  F  L  Y  S  K  L  T  V  D  K  S  R  -
     F                          S
     N V       M E X       N F  M       N  N
     U B       A B V       L A  N       S  L
     4 0       E V N       A N  L       I  A
     H 2       2 1 1       3 1  1       1  3
                          /-
  GGTGGCAGCAGGGGAACGTCTTCTCATGCTCCGTGATGCATGAGGCTCTGCACAACCACT
2041 --------------------+--------------------+---------+---------- 2100
  CCACCGTCGTCCCCTTGCAGAAGAGTACGAGGCACTACGTACTCCGAGACGTGTTGGTGA

    W  Q  Q  G  N  V  F  S  C  S  V  M  H  E  A  L  H  N  H  Y  -
                          S
     M      M   H N C          C X H
     B      N   P C R          F M A
     O      L   A I F          R A E
     2      1   2 1 1          1 3 3
                   /                   /
  ACACGCAGAAGAGCCTCTCCCTGTCTCCGGGTAAATGAGTGCGACGGCCG
2101 --------------------+--------------------+---------+-- 2150
  TGTGCGTCTTCTCGGAGAGGGACAGAGGCCCATTTACTCACGCTGCCGGC

    T  Q  K  S  L  S  L  S  P  G  K  *
```

12. Molécule de type immunoglobuline, comprenant la protéine de fusion de la revendication 9 et une chaîne légère d'immunoglobuline.

**13.** Molécule de type immunoglobuline de la revendication 12, comprenant en outre un agent thérapeutique, un marqueur détectable, ou un agent d'imagerie de RMN, liés à ladite molécule de type immunoglobuline.

**14.** Complexe comprenant la protéine de fusion de la revendication 9 ou 10 et gp120 de HIV ou de SIV.

**15.** Complexe de la revendication 14, dans lequel ladite protéine gp120 est une partie d'un HIV ou SIV, est exprimée sur la surface d'une cellule infectée par le HIV ou le SIV, ou est présente en solution.

**16.** Procédé d'utilisation d'une protéine de fusion de la revendication 9 ou 10 pour la détection de gp120 de HIV ou SIV dans un échantillon, caractérisé par

(a) la mise en contact d'un échantillon, soupçonné contenir gp120 de HIV ou de SIV, avec ladite protéine de fusion, et

(b) la détection de la formation ou non d'un complexe, ladite protéine de fusion étant marquée de façon détectable.

**Revendications pour l'Etat contractant suivant : ES**

**1.** Procédé de production d'une protéine de fusion codée par un gène codant pour une protéine de fusion qui est apte à être sécrétée, comprenant 1) la séquence d'ADN de CD4 extracellulaire, ou d'un fragment de CD4 qui se lie à gp120 de HIV lorsqu'elle est soudée à une chaîne d'immunoglobuline, et 2) la séquence d'ADN d'une chaîne lourde d'immunoglobuline, caractérisé en ce que la séquence d'ADN qui code au moins pour la région variable de ladite immunoglobuline a été remplacée par la séquence d'ADN qui code pour la protéine CD4 extracellulaire, ou ledit fragment de CD4 qui se lie à gp120, caractérisé par la culture dans un milieu nutritif, dans des conditions productrices de protéines, d'une souche hôte transformée par le vecteur comprenant le gène de la revendication 1, ledit vecteur comprenant en outre des signaux d'expression qui sont reconnus par ladite souche hôte et dirigent l'expression de ladite protéine de fusion, et la récupération de la protéine de fusion ainsi produite.

**2.** Procédé de la revendication 1, dans lequel ladite souche hôte est une lignée cellulaire de myélome qui produit des chaînes légères d'immunoglobulines, et ladite protéine de fusion comprend une chaîne lourde d'immunoglobuline appartenant à la classe IgM, IgG1 ou IgG3, et dans lequel est produite une molécule de type immunoglobuline comprenant ladite protéine de fusion.

**3.** Procédé de la revendication 1 ou 2, dans lequel le vecteur a les caractéristiques d'identification de pCD4Hγ1, qui a été déposé sous le n° 67611, ou de pCD4Mμ, qui a été déposé sous le n° 67608, ou de pCH4Pμ, qui a été déposé sous le n° 67609, ou de pCD4Eγ1, qui a été déposé sous le n° 67610, tous dans *E. coli*, à l'ATCC, sous les clauses du Traité de Budapest.

**Revendications pour l'Etat contractant suivant : GR**

**1.** Vecteur comprenant un gène codant pour une protéine de fusion qui est apte à être sécrétée, comprenant 1) la séquence d'ADN de CD4 extracellulaire, ou d'un fragment de CD4 qui se lie à gp120 de HIV lorsqu'elle est fusionnée à une chaîne d'immunoglobuline, et 2) la séquence d'ADN d'une chaîne lourde d'immunoglobuline, caractérisé en ce que la séquence d'ADN qui code au moins pour la région variable de ladite immunoglobuline a été remplacée par la séquence d'ADN qui code pour la protéine CD4 extracellulaire, ou ledit fragment de celle-ci qui se lie à gp120.

**2.** Vecteur selon la revendication 1, caractérisé en ce que ladite chaîne d'immunoglobuline appartient à la classe IgM, IgG1 ou IgG3.

**3.** Vecteur ayant les caractéristiques d'identification de pCD4Hγ1, qui a été déposé sous le n° 67611, ou de pCD4Mμ, qui a été déposé sous le n° 67608, ou de pCH4Pμ, qui a été déposé sous le n° 67609, ou de pCD4Eγ1, qui a été déposé sous le n° 67610, tous dans *E. coli*, à l'ATCC, sous les clauses du Traité de Budapest.

**4.** Hôte transformé par le vecteur des revendications 1 à 3.

**5.** Hôte de la revendication 4, qui exprime une chaîne légère d'immunoglobuline conjointement avec le produit d'expression dudit gène de protéine de fusion, pour donner une molécule de type immunoglobuline qui se lie à gp120.

**6.** Hôte de la revendication 5, dans lequel ladite chaîne lourde d'immunoglobuline appartient à la classe d'immunoglobulines IgM, IgG1 ou IgG3.

**7.** Procédé de production d'une protéine de fusion codée par un gène d'un vecteur de la revendication 1, caractérisé par la culture, dans un milieu nutritif, dans des conditions productrices de protéines, d'une souche hôte transformée par le vecteur de la revendication 1, ledit vecteur comprenant en outre des signaux d'expression qui sont reconnus par ladite souche hôte et dirigent l'expression de ladite protéine de fusion, et la récupération de la protéine de fusion ainsi produite.

**8.** Procédé de la revendication 7, dans lequel ladite souche hôte est une lignée cellulaire de myélome qui produit des chaînes légères d'immunoglobulines, et ladite protéine de fusion comprend une chaîne lourde d'immunoglobuline appartenant à la classe IgM, IgG1 ou IgG3, et dans lequel est produite une molécule de type immunoglobuline comprenant ladite protéine de fusion.

**9.** Procédé pour la détection de gp120 de HIV ou de SIV dans un échantillon, caractérisé par
    (a) la mise en contact d'un échantillon, soupçonné contenir gp120 de HIV ou de SIV, avec une protéine de fusion produite par un procédé selon la revendication 7 ou 8, et
    (b) la détection de la formation ou non d'un complexe, ladite protéine de fusion étant marquée de façon détectable.

**10.** Procédé selon la revendication 9, dans lequel une molécule de type immunoglobuline comprend en outre un agent thérapeutique, un marqueur détectable, ou un agent d'imagerie de RMN, liés à ladite molécule de type immunoglobuline.